# EUROPEAN PATENT APPLICATION

(11) **EP 4 616 784 A1**
(43) Date of publication of application: **17.09.2025**
(21) Application number: 25162921.8
(22) Date of filing: 11.03.2025
(51) Int. Cl.: A61B 1/00, A61B 1/045, G06F 3/01, H04N 23/50, H04N 23/63

(54) **MEDICAL VISUALIZATION SYSTEM WITH A USER INTERFACE**

(30) Priority: 11.03.2024 DE 102024106875; 11.03.2024 DE 102024106876
(71) Applicant: Ambu A/S, 2750 Ballerup (DK)
(72) Inventor: DAMYANOV, Denis Nikolaev, 2610 Rødovre (DK); UBBESEN, Line Sandahl, 2840 Holte (DK); ELMGREEN, Bo, 2750 Ballerup (DK); HOFFMANN, Veronica Julie Lodskov, 4000 Roskilde (DK); LØVGREEN, Mikkel Nøhr, 2765 Smørum (DK); TJELL, Peter, 2740 Skovlunde (DK)
(74) Representative: COPA Copenhagen Patents

(57) **Abstract**

Various aspects of a video processing apparatus operable to receive image data from an endoscope, having an image sensor configured to generate image data indicative of a view from the endoscope, are disclosed. The video processing apparatus comprises a housing, a processing unit adapted to receive the image data from the endoscope as the image data is being generated and cause a display to display a graphical user interface. The graphical user interface comprises a plurality of nonoverlapping portions including a first portion and a second portion, wherein the first portion is arranged next to the second portion along the second direction. The present disclosure provides various advantageous improvements of the disclosed video processing apparatus facilitating i.a. easier and more efficient operation of the video processing apparatus.

## Description

The present disclosure relates to a medical visualization system and elements thereof. Particularly, a medical visualization system comprising a video processing apparatus with a graphical user interface. The medical visualization system may also comprise a medical visualization device, such as an endoscope.

### BACKGROUND

An endoscope may be utilized to visually examine certain areas of the body of a person, such as inside a body cavity of the person. For example, an endoscope may be used to inspect the airways, the digestive tract, or the intestines.

An endoscope may be provided with a camera and be attached to a video processing apparatus, such as a video processing apparatus with a display screen, a video output from the camera of the endoscope may be received and displayed at the video processing apparatus or a coupled display screen, thereby allowing an operator to control the endoscope to inspect an area of interest.

An endoscope may comprise an operating handle at a proximal end and an insertion cord extending from the handle towards a distal end. The handle is configured to be held by an operator and inter alia comprises externally protruding operating members connected to internal control means allowing the operator to control the movement of a bending section near the distal end of the insertion cord, while advancing the distal end of the insertion cord to a desired location, e.g. within a body cavity of a person. In some situations, an endoscope may comprise, e.g. as an alternative or addition to the operating handle, a control interface which may be robotically controlled, e.g. for robot-assisted surgery. By means of a coupled video processing apparatus, such as a video processing apparatus with a display screen, the location to which the distal end has been advanced may be inspected using the endoscope. As an exemplary alternative to the endoscope comprising an insertion cord, the endoscope may be a laryngoscope, which is a more rigid type of endoscope, usually having an L-shape, used to inspect the larynx. In even further exemplary alternatives, the endoscope may be an endotracheal tube, laryngeal masks, or other devices configured with a distal portion to be inserted into a patient and a proximal portion to be held or manipulated by a user.

An endoscope may be a disposable endoscope. It is generally advantageous that endoscopes are disposable so as to avoid demanding cleaning processes and risks of cross contamination between patients.

The video processing apparatus may be provided with some functionality, such as an ability to save still images and/or video sequences of the view from the attached visualization device. Furthermore, the video processing apparatus may comprise some image processing capabilities and/or may be configured to output a video or image output, e.g. to an external display.

### SUMMARY

It is an object of the present disclosure to provide a solution which at least improves the solutions of the prior art. Particularly, it is an object of the present disclosure to provide a medical visualization system and elements thereof.

The present disclosure is set out by the appended claims.

Particularly, a video processing apparatus is disclosed as well as a medical visualization system comprising such video processing apparatus and an endoscope. The endoscope has an image sensor (e.g. a camera module) configured to generate image data indicative of a view from the endoscope. The endoscope may be a disposable endoscope. The video processing apparatus is operable to receive image data from the endoscope.

The video processing apparatus comprises a housing and a processing unit. The processing unit is adapted to receive the image data from the endoscope as the image data is being generated. The processing unit is further adapted to cause a display (a display of the video processing apparatus itself and/or an external display) to display a graphical user interface. The graphical user interface extends a first length along a first direction (e.g. a vertical direction) and a second length along a second direction (e.g. a horizontal direction) perpendicular to the first direction. The graphical user interface comprises a plurality of non-overlapping portions including a first portion and a second portion, wherein the first portion is arranged next to the second portion along the second direction. The plurality of non-overlapping portions may comprise a third portion and/or a fourth portion.

In a first aspect of the disclosure, the video processing apparatus displays a live representation of the image data within the first portion of the graphical user interface. The live representation is displayed at a full size covering the first length along the first direction.

The video processing apparatus is adapted to detect a first user input signal indicative of a request to activate a freeze function, and in response to detecting the first user input signal the video processing apparatus activates the freeze function. Activating the freeze function includes ceasing display of the live representation of the image data within the first portion of the graphical user interface, displaying within the first portion of the graphical user interface a first representation of a still image corresponding to the image data received when the first user input signal was detected, wherein the first representation of the still image is displayed at the full size, and displaying the live representation of the image data within the second portion of the graphical user interface, wherein the live representation is displayed at a reduced size smaller than the full size.

Advantages of the first aspect of the present disclosure include that it gives the operator an opportunity to more conveniently perform a closer inspection of a view during the procedure, and that before deciding to store an image for later retrieval, the operator may inspect the image to confirm that what he/she is attempting to capture is actually seen in the picture and/or ensure that the picture quality is as expected/necessary.

In a second aspect of the disclosure, the video processing apparatus further comprises a peripheral input device interface (e.g. comprising a USB interface, Bluetooth transceiver, and/or another interface type known in the art) adapted to couple the video processing apparatus to one or more peripheral input devices (e.g. a foot pedal, a keyboard, a mouse, a trackpad, a game controller, or similar). The processing unit may be adapted to detect one or more peripheral input signals from the one or more peripheral input devices.

In the second aspect the video processing apparatus displays a shortcut setup user interface within the first portion of the graphical user interface. While displaying the shortcut setup user interface, the video processing apparatus is adapted to detect a first peripheral input signal from a first peripheral input device coupled to the video processing apparatus (via the peripheral input device interface). The first peripheral input signal is indicative of activation of an input component of the first peripheral input device.

In response to detecting the first peripheral input signal the video processing apparatus displays a shortcut indicator indicative of the input component of the first peripheral input device associated with the first peripheral input signal, and displays a function selection interface comprising two or more representation of two or more functions assignable to the input component of the first peripheral input device.

While displaying the function selection interface, the video processing apparatus is adapted to detect a first user input signal indicative of selection of a first function of the two or more functions. In response to detecting the first user input, the video processing apparatus stores first shortcut information including assignment of the first peripheral input signal to the first function.

After storing the first shortcut information, the video processing apparatus is adapted to detect the first peripheral input signal from the first peripheral input device. In response to detecting the first peripheral input signal the video processing apparatus, in accordance with the video processing apparatus operating in a first mode where the first function is available, activates the first function.

An advantage of the second aspect of the present disclosure is that it allows a user to configure peripheral input devices (e.g. USB devices) to perform desired functions (such as storing of images and/or recording of videos), aiding the user to more efficiently perform a procedure with less need of assistance. A further advantage is that by allowing peripheral input devices to activate certain functions, remote activation of certain functions may be achieved without the need for the endoscope to comprise programmable buttons. Thus, the second aspect of the present disclosure enhances the flexibility of using the video processing apparatus in combination with a range of different endoscopes.

In a third aspect of the disclosure, the video processing apparatus displays a patient information panel within the second portion of the graphical user interface. The video processing apparatus may display a live representation of the image data within the first portion of the graphical user interface. The patient information panel comprises a heading section and a detailed section further comprising detailed patient information and a hide button. The video processing apparatus is further adapted to detect a first user input corresponding to selection of the hide button.

In response to detecting the first user input and in accordance with a hide data function being active when detecting the first user input, the video processing apparatus deactivates the hide data function including displaying first patient information in the heading section. In response to detecting the first user input and in accordance with the hide data function not being active when detecting the first user input, the video processing apparatus activates the hide data function including ceasing display of the first patient information in the heading section.

The video processing apparatus is further adapted to detect a second user input indicative of a request to toggle the detailed section of the patient information panel. In response to detecting the second user input and in accordance with the detailed section being displayed when detecting the second user input, the video processing apparatus ceases display of the detailed section of the patient information panel. In response to detecting the second user input and in accordance with the detailed section not being displayed when detecting the second user input, the video processing apparatus displays the detailed section of the patient information panel.

An advantage of the third aspect of the present disclosure is that it provides a convenient possibility for an operator to hide patient information where appropriate, e.g. if bystanders are joining the procedures. Furthermore, the third aspect provides a balanced approach where patient information may be hidden, while still providing a possibility for the operator to easily access the information if needed.

In a fourth aspect of the disclosure, the video processing apparatus retrieves worklist data from a remote server. The worklist data comprises patient information data for a plurality of scheduled procedures.

The video processing apparatus opens a procedure session and displays a live representation of the image data within the first portion of the graphical user interface. Alternatively, the video processing apparatus displays a plurality of representations corresponding to a plurality of stored data files stored during a procedure session within the first portion of the graphical user interface.

The video processing apparatus displays a patient information panel within the second portion of the graphical user interface. The patient information panel comprises a worklist section comprising a plurality of selectable patient information entries corresponding to the patient information data of the worklist data.

The video processing apparatus is adapted to detect a first user input corresponding to selection of a first patient information entry of the plurality of selectable patient information entries. In accordance with detecting the first user input the video processing apparatus assigns first patient information data corresponding to the first patient information entry to the procedure session.

Advantages of the fourth aspect of the present disclosure includes easy assigning of patient data to either an ongoing (new) procedure session or a previously performed procedure session, as well as enhancing the likelihood that patient data assigned to sessions (in effect assigned to stored images/videos) are complete, correct and/or compliant with other records of the patient.

In a fifth aspect of the disclosure, the video processing apparatus opens a procedure session and displays a live representation of the image data within the first portion of the graphical user interface. Alternatively, the video processing apparatus displays a plurality of representations corresponding to a plurality of stored data files stored during a procedure session within the first portion of the graphical user interface.

The video processing apparatus displays a patient entry panel within the second portion of the graphical user interface comprising one or more patient info entry fields and a confirm button. The video processing apparatus is adapted to receive a sequence of inputs corresponding to input of a sequence of characters in the one or more patient entry fields and a subsequent confirm user input corresponding to selection of the confirm button.

In response to receiving the confirm user input the video processing apparatus assigns first patient information data corresponding to the sequence of characters in the one or more patient entry fields to the procedure session.

An advantage of the fifth aspect of the present disclosure is that it provides for easy assigning of patient data to either an ongoing (new) procedure session or a previously performed procedure session, in cases where the necessary patient data is not automatically retrievable (as in relation to the fourth aspect of the disclosure). This may, for example, be advantageous in situations where the procedure is an unplanned procedure, where schedules are changed and/or where the operator does not have required access to retrieve the patient data electronically. The fifth aspect of the present disclosure is further advantageous in combination with the fourth aspect of the present solution, where the fifth aspect may provide a backup solution in certain circumstances. Thus, providing for increased flexibility in use of the video processing apparatus of the present disclosure.

In a sixth aspect of the disclosure, the video processing apparatus comprises a network interface.

The video processing apparatus displays a network drive setup within the first portion of the graphical user interface. The network drive setup comprises a plurality of network drive input fields and a confirm button. While displaying the network drive setup, the video processing apparatus is adapted to receive a sequence of one or more user inputs corresponding to input of a sequence of characters in the one or more network drive input fields and a confirm user input corresponding to selection of the confirm button.

In response to receiving the confirm user input the video processing apparatus stores network drive information for a first network drive corresponding to the sequence of characters in the one or more network drive input fields.

An advantage of the sixth aspect of the present disclosure is that it provides an easy and convenient way of enabling export of stored images/videos to a network drive, e.g. using a standard file sharing protocol. Furthermore, the sixth aspect of the disclosure facilitates that the video processing apparatus may be configured to work in various IT environments.

In a seventh aspect of the disclosure, which advantageously may be combined with the sixth aspect, the video processing apparatus may comprise a network interface and/or an external communication interface, such as a USB interface.

The video processing apparatus displays, within the first portion of the graphical user interface, a plurality of representations corresponding to a plurality of stored data files stored during a procedure session, and the video processing apparatus displays an export button (e.g. within the third portion of the graphical user interface). The video processing apparatus is adapted to detect an export user input corresponding to selection of the export button.

In response to detection of the export user input the video processing apparatus displays an export menu comprising an export confirm button. The video processing apparatus is adapted to receive an export confirm user input corresponding to selection of the export confirm button. The export menu further comprises a plurality of selectable export modalities including export to a first PACS server and optionally export to a first network drive and/or export to a first external storage device (e.g. a USB drive). The video processing apparatus is adapted to receive a modality selection user input corresponding to selection of a selected export modality of the plurality selectable export modalities.

The video processing apparatus, in response to detecting the export confirm user input, and in accordance with the selected export modality corresponding to the export to the first network drive, transmits stored image data corresponding to one or more of the plurality of stored data files to the first network drive.

The video processing apparatus, in response to detecting the export confirm user input, and in accordance with the selected export modality corresponding to the export to the first external storage device, transmits stored image data corresponding to one or more of the plurality of stored image data files to the first external storage device (e.g. the first USB drive).

An advantage of the seventh aspect of the present disclosure is that it provides a plurality of possible ways to export stored images/videos easily and conveniently, also the seventh aspect of the present disclosure facilitates use of standard or de facto standard devices and/or protocols, thus in turn simplifying use and increasing flexibility in using the video processing apparatus.

As will also become apparent in the following description, it should be noted that the above-mentioned aspects of the present disclosures may be combined in any convenient way, and accordingly may form parts of the same graphical user interface. Furthermore, examples of the various aspects as described in the present disclosure may be combined as appropriate, and it should be noted that all of the mentioned aspects and examples may be employed at one single video processing apparatus.

### GENERAL DESCRIPTION

A video processing apparatus is disclosed as well as a medical visualization system comprising such video processing apparatus and an endoscope. The endoscope has an image sensor (e.g. a camera module) configured to generate image data indicative of a view from the endoscope. The endoscope may be a disposable endoscope. The video processing apparatus is operable to receive image data from the endoscope. The endoscope may, for example, be an arthroscope, a bronchoscope, a cholangioscope, a colonoscope, a cystoscope, a duodenoscope, an endotracheal tube, a gastroscope, a laparoscope, a laryngeal mask, a laryngoscope, or an ureteroscope.

The endoscope, such as a handle of the endoscope, may comprise one or more buttons. The endoscope may be adapted to transmit (e.g. to the video processing apparatus) one or more respective endoscope signal indicative of a user activating a respective button on the endoscope. For example, the one or more buttons may include a first button and the endoscope may be adapted to transmit (e.g. to the video processing apparatus) a first endoscope signal indicative of a user activating the first button. The one or more buttons may include a second button and the endoscope may be adapted to transmit (e.g. to the video processing apparatus) a second endoscope signal indicative of a user activating the second button. The video processing apparatus may be adapted to detect the one or more endoscope signals.

The video processing apparatus comprises a housing and a processing unit. The video processing apparatus may comprise electronic memory, which may comprise volatile and/or non-volatile memory.

The processing unit is adapted to receive the image data from the endoscope as the image data is being generated. The processing unit is further adapted to cause a display to display a graphical user interface. The video processing apparatus may comprise the display. For example, the display may be accommodated in the housing of the video processing apparatus. Alternatively or additionally, the display may be an external display coupled to the video processing apparatus, such as to allow the video processing apparatus to cause the external display to display the graphical user interface and/or a secondary graphical user interface.

The display may be a touch sensitive display. Accordingly, when in the following referring to a user input it may generally be a touch user input detected by the touch sensitive display. However, the video processing unit may, alternatively, be controlled by other means, such as a mouse or another human interface device (HID).

The graphical user interface extends a first length along a first direction (e.g. a vertical direction of the display) and a second length along a second direction (e.g. a horizontal direction of the display) perpendicular to the first direction. The second length may be larger than the first length. For example, the ratio between the second length and the first length may be 4:3, 16:9, or 16:10.

The graphical user interface comprises a plurality of non-overlapping portions including a first portion and a second portion, wherein the first portion is arranged next to the second portion along the second direction. Each of the portions may extend substantially throughout the first length in the first direction. The plurality of non-overlapping portions may comprise a third portion and/or a fourth portion. The first portion may be arranged between the third portion and the second portion along the second direction. The second portion may be arranged between the fourth portion and the first portion along the second direction. The fourth portion may be arranged between a side of the display and the second portion along the second direction. The third portion may be arranged between another (opposite) side of the display and the first portion along the second direction. The first portion and the second portion may be arranged between the third portion and the fourth portion along the second direction.

The video processing apparatus may display a live representation of the image data within the first portion of the graphical user interface. The live representation may be displayed at a full size covering the first length along the first direction.

The term "live representation" or "live view" may be interpreted to mean that the view simultaneously being captured by the image sensor generating the image data is presented with very little or no significant delay/latency, e.g. so that the physician observing the live representation/live view can rely on the view being representative of the current position/orientation of the endoscope. Generally, insignificant latency between image generation and image presentation may be in the order of milliseconds, which may correspond to a few frames. Generally, the live representation is presented as video comprising a plurality of frames, where each frame can be stored as a still image, if desired.

The video processing apparatus may be adapted to detect a first user input signal indicative of a request to activate a freeze function. In response to detecting the first user input signal the video processing apparatus may activate the freeze function. Activating the freeze function may include ceasing display of the live representation of the image data within the first portion of the graphical user interface, displaying within the first portion of the graphical user interface a first representation of a still image corresponding to the image data received when the first user input signal was detected, wherein the first representation of the still image is displayed at the full size, and displaying the live representation of the image data within the second portion of the graphical user interface, wherein the live representation is displayed at a reduced size smaller than the full size.

Displaying the first representation of the still image within the first portion of the graphical user interface may include displaying a freeze indicator element overlaying a portion of the first representation of the still image. Thus, the video processing apparatus may, with the freeze indicator element, provide a visual indication that the representation currently being displayed is not the live view but rather a still image.

While the freeze function is active, the video processing apparatus may be adapted to detect a second user input signal indicative of a request to deactivate the freeze function. In response to detecting the second user input signal, the video processing apparatus may deactivate the freeze function. Deactivating the freeze function may include ceasing display of the first representation of the still image within the first portion of the graphical user interface, displaying the live representation of the image data within the first portion of the graphical user interface, wherein the live representation is displayed at the full size, and ceasing display of the live representation of the image data within the second portion of the graphical user interface.

The video processing apparatus may be adapted to detect a user input directed to the live representation of the image data being displayed within the second portion of the graphical user interface. The second user input signal may be instigated by detection of the user input directed to the live representation of the image data. For example, the user may provide a user input to the live representation in the second portion, and thereby deactivate the freeze function.

The video processing apparatus may be adapted to detect disconnection of the endoscope. The second user input signal may be instigated in response to detecting disconnection of the endoscope from the video processing apparatus. For example, the video processing apparatus may automatically deactivate the freeze function if the endoscope is disconnected from the video processing apparatus.

The video processing apparatus may be adapted to detect connection of an endoscope. In some examples, the video processing apparatus may be adapted to facilitate simultaneous connection of more than one endoscope. Thus, the video processing apparatus may be adapted to detect connection of an endoscope, which may be connection of a first endoscope and/or a second endoscope. The second user input signal may be instigated in response to detecting connection of a first endoscope and/or a second endoscope to the video processing apparatus. For example, the video processing apparatus may automatically deactivate the freeze function if an additional/second endoscope is connected to the video processing apparatus.

The video processing apparatus may display a freeze button, e.g. within the third portion of the graphical user interface. The video processing apparatus may be adapted to detect a user input corresponding to selection of the freeze button. The first user input signal may be instigated by detection of the user input corresponding to selection of the freeze button. Thus, the user may request activation of the freeze function by providing a user input to the freeze button. Alternatively or additionally, the second user input signal may be instigated by detection of the user input corresponding to selection of the freeze button. Thus, the user may request deactivation of the freeze function by providing a user input to the freeze button. In some examples, the freeze button may work as a toggle button, which works to activate the freeze function if the freeze function is not active and to deactivate the freeze function if the freeze function is active.

The video processing apparatus may be adapted to detect a first endoscope signal from the endoscope indicative of a user activating a first button on the endoscope. The first user input signal may be instigated by detection of the first endoscope signal. Thus, the user may request activation of the freeze function by activating the first button on the endoscope. Alternatively or additionally, the second user input signal may be instigated by detection of the first endoscope signal. Thus, the user may request deactivation of the freeze function by activating the first button on the endoscope. In some examples, the first button on the endoscope may work as a toggle button, which works to activate the freeze function if the freeze function is not active and to deactivate the freeze function if the freeze function is active.

While the freeze function is active, the video processing apparatus is adapted to detect a third user input signal indicative of a request to store the still image (i.e. the still image of which the first representation is being displayed within the first portion of the graphical user interface). In response to detecting the third user input signal the video processing apparatus may store, in the electronic memory of the video processing apparatus, an image data file corresponding to the image data received when the first user input signal was detected. The image data file (stored in response to detecting the third user input signal while the freeze function was active) may comprise information indicative of the image data file being captured while the freeze function was active. For example, the freeze indicator element may be included in the image data file, e.g. overlaying a portion of the still image.

The video processing apparatus may display one or more actionable items, e.g. within the third portion of the graphical user interface. The one or more actionable items may comprise an image capture button. The video processing apparatus may be adapted to detect a capture user input (e.g. a touch input) corresponding to selection of the image capture button. The third user input signal may be instigated by the user input corresponding to selection of the image capture button.

The video processing apparatus may be adapted to detect a second endoscope signal from the endoscope indicative of a user activating a second button on the endoscope (e.g. a different button than the previously mentioned first button of the endoscope). The third user input signal may be instigated by detection of the second endoscope signal.

In response to detecting the third user input signal, the video processing apparatus may further deactivate the freeze function. Thus, the video processing apparatus may automatically deactivate the freeze function after storing the image corresponding to the representation currently being displayed in the first portion.

In response to detecting the third user input signal, the video processing apparatus may display the first representation of the still image within the second portion of the graphical user interface, e.g. at a different position than where the live representation was displayed while the freeze function was active. After a predetermined delay after detection of the third user input signal, the video processing apparatus may cease display of the first representation of the still image within the second portion of the graphical user interface.

The video processing apparatus may comprise one or more image modifying functions. The one or more image modifying functions may include one or more of ARC (Advanced Red Contrast), color adjustment, contrast adjustment, sharpness adjustment, image brightness adjustment, zoom function, rotation of live representation, and endoscope light on/off. Each of the one or more image When being activated each of the one or more image modifying functions may modify one or more parameters of the live representation of the image data. While the freeze function is not active, the one or more image modifying functions may be activatable. While the freeze function is active, the one or more image modifying functions may be not activatable (e.g. disabled and/or unavailable).

While the freeze function is active, the video processing apparatus, in response to detecting a fourth user input signal indicative of a request to activate an image modifying function of the one or more image modifying functions, may display a notification, e.g. within the second portion of the graphical user interface, indicating that the image modifying function is disabled while the freeze function is active.

The video processing apparatus may comprise an endoscope light switch function. The endoscope light-switch function may when being activated instigate deactivation of a light emitter of the endoscope. While the freeze function is not active, the endoscope light switch function may be activatable. While the freeze function is active, the endoscope light switch function may be not activatable (e.g. disabled and/or unavailable).

The video processing apparatus may, in response to detecting the first user input signal, in accordance with the light emitter of the endoscope being active, activate the freeze function. The video processing apparatus may, in response to detecting the first user input signal, in accordance with the light emitter of the endoscope not being active, forego activation of the freeze function. Thus, the user may be allowed to activate the freeze function when the endoscope light emitter is active, and/or the user may be not allowed to activate the freeze function when the endoscope light emitter is not active.

The video processing apparatus may comprise a peripheral input device interface adapted to couple the video processing apparatus to one or more peripheral input devices. The peripheral input device interface may comprise a USB interface, a Bluetooth transceiver, and/or another interface type known in the art. The one or more peripheral input devices may include (or be) a foot pedal, a keyboard, a mouse, a trackpad, a game controller, or other devices capable of providing an input signal to the video processing apparatus. The one or more peripheral input devices may generate and transmit one or more peripheral input signals. The processing unit may be adapted to detect one or more peripheral input signals from the one or more peripheral input devices, e.g. when being coupled to the video processing apparatus, e.g. via the peripheral input device interface.

The video processing apparatus may display a shortcut setup user interface within the first portion of the graphical user interface. The shortcut setup user interface may comprise instructions to connect a first peripheral input device and/or to activate an input component of the first peripheral input device.

The video processing apparatus may store one or more user profiles each including a username and a password for activating an authorized mode of the video processing apparatus. In some examples, the shortcut setup user interface is displayed in accordance with the authorized mode being active.

While displaying the shortcut setup user interface, the video processing apparatus is adapted to detect a first peripheral input signal from the first peripheral input device coupled to the video processing apparatus (e.g. via the peripheral input device interface). The first peripheral input signal may be indicative of activation of the input component of the first peripheral input device.

In response to detecting the first peripheral input signal the video processing apparatus may display a shortcut indicator, e.g. within the shortcut setup user interface. The shortcut indicator may be indicative of the input component of the first peripheral input device associated with the first peripheral input signal.

In response to detecting the first peripheral input signal and in accordance with the video processing apparatus comprising shortcut information (e.g. in the electronic memory) including assignment of the first peripheral input signal to a previous function, the video processing apparatus may display a warning message. The warning message may include information of the previous function. Thereby, the operator may be made aware what feature is already associated with the input component of the first peripheral input device, and which may potentially be overwritten if setup is continued.

In response to detecting the first peripheral input signal, the video processing apparatus may display a cancel button selectable to exit the shortcut setup user interface.

Further in response to detecting the first peripheral input signal the video processing apparatus may display a function selection interface, e.g. within the shortcut setup user interface. The function selection interface may comprise two or more representations of two or more functions assignable to the input component of the first peripheral input device.

The two or more functions assignable to the input component of the first peripheral input device may include a capture image function causing the video processing apparatus to store an image data file corresponding to the image data received when the capture image function was activated. Alternatively or additionally, the two or more functions assignable to the input component of the first peripheral input device may include a video capture function causing the video processing apparatus to store a video sequence of image data corresponding to the image data received when the video capture function was activated.

While displaying the function selection interface, the video processing apparatus may be adapted to detect a first user input indicative of selection of a first function of the two or more functions. For example, the first function may be the capture image function, or the first function may be the video capture function. In response to detecting the first user input, the video processing apparatus may store first shortcut information including assignment of the first peripheral input signal to the first function.

In response to detecting the first user input (before, after or concurrently with storing the first shortcut information) the video processing apparatus may display a naming dialogue. The naming dialogue may include an input text-field. The naming dialogue may be adapted to receive a first user input name for identifying the assignment of the first peripheral input signal to the first function. The stored first shortcut information may include the first user input name. In accordance with the first user input name not being received or being empty or being invalid, the stored first shortcut information may include a default and/or system generated name.

The video processing apparatus may be adapted to detect a user input corresponding to selection of the cancel button. In response to detecting the user input corresponding to selection of the cancel button, the video processing apparatus may exit the shortcut setup user interface and forego storing the first shortcut information.

After storing the first shortcut information, the video processing apparatus may be adapted to detect the first peripheral input signal from the first peripheral input device.

In response to detecting the first peripheral input signal, e.g. in accordance with the video processing apparatus operating in a first mode where the first function is available, the video processing apparatus may activate the first function. When operating in the first mode, the video processing apparatus may display a live representation of the image data within the first portion of the graphical user interface.

In response to detecting the first peripheral input signal, e.g. in accordance with the video processing apparatus operating in a second mode where the first function is not available, the video processing apparatus may display an error notification in the graphical user interface and may forgo activating the first function. The error notification may be displayed in the second portion of the graphical user interface. Some situations may cause the video processing apparatus to operate in the second mode. For example, the video processing apparatus may operate in the second mode when the live representation of the image data is not displayed and/or when no endoscope is connected to the video processing apparatus.

The video processing apparatus may display a patient information panel within the second portion of the graphical user interface, e.g. while with the video processing apparatus may display a live representation of the image data within the first portion of the graphical user interface. The patient information panel may comprise a heading section and a detailed section. The detailed section may comprise detailed patient information and a hide button. The detailed patient information may include name, personal identification number, date of birth and/or gender of the patient. The video processing apparatus may be adapted to detect a hide user input corresponding to selection of the hide button.

In response to detecting the hide user input and in accordance with a hide data function being active when detecting the hide user input, the video processing apparatus may deactivate the hide data function. Deactivating the hide data function may include displaying first patient information in the heading section. The first patient information may include a name of the patient and/or a personal identification number of the patient. The detailed patient information may include the first patient information. In response to detecting the hide user input and in accordance with the hide data function not being active when detecting the hide user input, the video processing apparatus may activate the hide data function. Activating the hide data function may include ceasing display of the first patient information in the heading section.

Deactivating the hide data function may include changing a visual appearance of the hide button to a first appearance (e.g. a first color). Activating the hide data function may include changing the visual appearance of the hide button to a second appearance (e.g. a second color) different from the first appearance.

The video processing apparatus may be further adapted to detect a detail user input indicative of a request to toggle the detailed section of the patient information panel. The request to toggle the detailed section may be a user input corresponding to selection of an expand icon.

In response to detecting the detail user input and in accordance with the detailed section being displayed when detecting the detail user input, the video processing apparatus may cease display of the detailed section of the patient information panel. In response to detecting the detail user input and in accordance with the detailed section not being displayed when detecting the detail user input, the video processing apparatus may display the detailed section of the patient information panel.

The video processing apparatus may comprise a display interface, e.g. DVI, HDMI or other, adapted to connect the video processing apparatus to an external display. The video processing apparatus may be adapted to cause the external display to display a secondary graphical user interface. The secondary graphical user interface may extend a first secondary length along a first secondary direction (e.g. a vertical direction of the external display) and a second secondary length along a second secondary direction (e.g. a horizontal direction of the external display) perpendicular to the first secondary direction. The second secondary length may be larger than the first secondary length. For example, the ratio between the second secondary length and the first secondary length may be 4:3, 16:9, or 16:10.

The secondary graphical user interface may comprise a plurality of non-overlapping secondary portions including a first secondary portion and a second secondary portion, wherein the first secondary portion is arranged next to the second secondary portion along the second secondary direction. Each of the secondary portions may extend substantially throughout the first secondary length in the first secondary direction. The plurality of non-overlapping secondary portions may comprise a third secondary portion and/or a fourth secondary portion. The first secondary portion may be arranged between the third secondary portion and the second secondary portion along the second secondary direction. The second secondary portion may be arranged between the fourth secondary portion and the first secondary portion along the second secondary direction. The fourth secondary portion may be arranged between a side of the external display and the second secondary portion along the second secondary direction. The third secondary portion may be arranged between another (opposite) side of the external display and the first secondary portion along the second secondary direction. The first secondary portion and the second secondary portion may be arranged between the third secondary portion and the fourth secondary portion along the second secondary direction.

The video processing apparatus may display the live representation of the image data within the first secondary portion of the secondary graphical user interface.

In accordance with the hide data function not being active the video processing apparatus may display a secondary patient information panel within the second secondary portion of the secondary graphical user interface. The secondary patient information panel may comprise the detailed patient information. In accordance with the hide data function being active the video processing apparatus may forego display of the secondary patient information panel within the second secondary portion of the secondary graphical user interface.

In response to detecting the detail user input and in accordance with the hide data function not being active and the detailed section not being displayed when detecting the detail user input, the video processing apparatus may display the secondary patient information panel comprising the detailed patient information.

In response to detecting the detail user input and in accordance with the hide data function not being active and the detailed section being displayed when detecting the detail user input, the video processing apparatus may cease display of the secondary patient information panel or at least the detailed patient information of the secondary patient information panel.

In response to detecting the detail user input and in accordance with the hide data function being active when detecting the detail user input, the video processing apparatus may forego display of the secondary patient information panel within the second secondary portion of the secondary graphical user interface.

The video processing apparatus may retrieve worklist data from a remote server. The video processing apparatus and the remote server may be part of a local area network. The video processing apparatus may retrieve the worklist data via the local area network. The worklist data may comprise patient information data for a plurality of scheduled procedures.

The video processing apparatus may open a (new) procedure session and display a live representation of the image data within the first portion of the graphical user interface. Alternatively, the video processing apparatus may display a plurality of representations corresponding to a plurality of stored data files stored during a (previous) procedure session within the first portion of the graphical user interface.

The video processing apparatus may display a patient information panel within the second portion of the graphical user interface. Before displaying the patient information panel, the video processing apparatus may display a worklist button, e.g. in the second portion of the graphical user interface. The video processing apparatus may be adapted to detect a worklist button user input corresponding to selection of the worklist button. In response to detecting the worklist button user input the video processing apparatus may display the patient information panel.

The patient information panel may comprise a heading section. The patient information panel may comprise a worklist section. The video processing apparatus may store one or more user profiles each including a username and a password for activating an authorized mode of the video processing apparatus. In some examples, the worklist section is displayed in accordance with the authorized mode being active. The worklist section may comprise a plurality of selectable patient information entries corresponding to the patient information data of the worklist data. Each of the plurality of selectable patient information entries may comprise one or more of patient name, patient identification number, and scheduled time for procedure (e.g. including date and time of day).

The video processing apparatus may be adapted to detect a first user input corresponding to selection of a first patient information entry of the plurality of selectable patient information entries.

In accordance with detecting the first user input, and optionally one or more further user inputs (e.g. for confirming the choice), the video processing apparatus may assign first patient information data corresponding to the first patient information entry to the procedure session (i.e. to the newly opened procedure session in the case where the video processing apparatus displays a live representation of the image data, or to the previous procedure session in the use case where the video processing apparatus displays a plurality of representations corresponding to a plurality of stored data files).

In response to detecting the first user input the video processing apparatus may display a representation of the first patient information data and a confirmation button, e.g. in the second portion of the graphical user interface. The video processing apparatus may be adapted to detect a confirmation user input corresponding to selection of the confirmation button. In response to detecting the confirmation user input the video processing apparatus may assign the first patient information data to the procedure session.

The video processing apparatus may be adapted to detect a second user input indicative of a request to toggle display of the worklist section of the patient information panel. The second user input may correspond to selection of an expand icon, e.g. within the heading section of the patient information panel.

In response to detecting the second user input and in accordance with the worklist section being displayed when detecting the second user input, the video processing apparatus may cease display of the worklist section of the patient information panel. In response to detecting the second user input and in accordance with the worklist section not being displayed when detecting the second user input, the video processing apparatus may display the worklist section of the patient information panel.

In response to detecting the second user input and in accordance with the worklist section not being displayed when detecting the second user input, the video processing apparatus may retrieve the worklist data from the remote server.

The plurality of selectable patient information entries of the worklist section may be sorted in chronological order, e.g. based on scheduled time for procedure.

The patient information data of the worklist may consist of patient information data having a scheduled time for procedure between a first time, e.g. 12 hours, prior to a current system time of the video processing apparatus and a second time, e.g. 24 hours, after the current system time. The first time may be shorter than the second time.

The selectable patient information entries may comprise a primary selectable patient information entry and a secondary selectable patient information entry. The primary selectable patient information entry may have a primary scheduled time for procedure and the secondary selectable patient information entry may have a secondary scheduled time for procedure. The secondary scheduled time for procedure may be later than (i.e. after) the primary scheduled time for procedure.

The primary selectable patient information entry may be adjacent to the secondary selectable patient information entry. In other words, the selectable patient information entries may be void of an intermediate selectable patient information entry having an intermediate scheduled time for procedure between the primary scheduled time for procedure and the secondary scheduled time for procedure.

In accordance with the current system time of the video processing apparatus being between the primary scheduled time for procedure and the secondary scheduled time for procedure, the video processing apparatus, in displaying the patient information panel, may forego display of the primary selectable patient information entry in the worklist section and may display the secondary selectable patient information entry in the worklist section adjacent to a top of the worklist section.

In accordance with the current system time of the video processing apparatus being prior to the primary scheduled time for procedure, the video processing apparatus, in displaying the patient information panel, may display the primary selectable patient information entry in the worklist section adjacent to the top of the worklist section and may display the secondary selectable patient information entry in the worklist section adjacent to and below the primary selectable patient information entry.

In accordance with the current system time of the video processing apparatus being after the secondary scheduled time for procedure, the video processing apparatus, in displaying the patient information panel, may forego display of the primary selectable patient information entry in the worklist section and may forego display of the secondary selectable patient information entry in the worklist section.

The worklist section may be scrollable in a first scroll direction (e.g. parallel to or the same as the first direction) to show earlier selectable patient information entries of the plurality of selectable patient information entries. The worklist may be scrollable in a second scroll direction (e.g. opposite the first scroll direction) to show later selectable patient information entries of the plurality of selectable patient information entries.

The video processing apparatus may be adapted to detect connection of the endoscope. In response to detecting connection of the endoscope the video processing apparatus may retrieve the worklist data. In response to detecting connection of the endoscope the video processing apparatus may open the procedure session. In response to detecting connection of the endoscope the video processing apparatus may obtain device identifier information from a device identifier (e.g. an EPROM, a QR code, an RFID tag, an NFC, etc.) of the endoscope. The procedure session may be associated with the device identifier information.

The video processing apparatus may display one or more actionable items within the graphical user interface, e.g. within the third portion of the graphical user interface. The one or more actionable items may comprise an image capture button. The video processing apparatus may be adapted to detect a capture user input corresponding to selection of the image capture button. In response to detecting the capture user input the video processing apparatus may store an image data file corresponding to the image data received when the capture user input was detected. The video processing apparatus may further associate the image data file with the procedure session.

The patient information panel may comprise a search field. The video processing apparatus may be adapted to receive a sequence of inputs corresponding to inputs of a sequence of characters in the search field. In response to receiving the sequence of inputs the video processing apparatus may filter the displayed selectable patient information entries of the plurality of selectable patient information entries, e.g. based on a comparison between the sequence of characters in the search field and the patient information data corresponding to the plurality of selectable patient information entries.

After assigning the first patient information data to the procedure session, the video processing apparatus may display first patient information of the first patient information data in the heading section of the patient information panel.

After assigning the first patient information data to the procedure session the video processing apparatus may cease display of the worklist section of the patient information panel.

The patient information panel may comprise a change patient button, e.g. after assigning the first patient information data to the procedure session. The video processing apparatus may be adapted to detect a change request user input corresponding to selection of the change patient button. In response to detecting the change request user input the video processing apparatus may display a confirmation dialogue, e.g. in the first portion of the graphical user interface or in the second portion of the graphical user interface. The confirmation dialogue may include a confirm button and a cancel button. The video processing apparatus may be adapted to detect a confirm user input corresponding to selection of the confirm button and a cancel user input corresponding to selection of the cancel button.

In accordance with detecting the confirm user input the video processing apparatus may remove the assignment of the first patient information data to the procedure session. The video processing apparatus may further cease display of the confirmation dialogue and redisplay the worklist section in the patient information panel.

In accordance with detecting the cancel user input the video processing apparatus may maintain the assignment of the first patient information data to the procedure session. The video processing apparatus may further cease display of the confirmation dialogue and forego redisplay of the worklist section in the patient information panel.

The video processing apparatus may display a patient entry panel within the second portion of the graphical user interface comprising one or more patient info entry fields and a confirm button.

Before displaying the patient entry panel, the video processing apparatus may display a manual entry button, e.g. within the second portion of the graphical user interface. The patient information panel may comprise the manual entry button. The video processing apparatus may be adapted to detect a manual entry button user input corresponding to selection of the manual entry button. In accordance with detecting the manual entry button user input the video processing apparatus may display the patient entry panel within the second portion of the graphical user interface comprising the one or more patient info entry fields and a confirm button.

The video processing apparatus may be adapted to receive a sequence of inputs corresponding to input of a sequence of characters in the one or more patient entry fields. The video processing apparatus may be adapted to detect a confirm user input corresponding to selection of the confirm button (e.g. subsequently to receiving the sequence of inputs). In response to receiving the confirm user input the video processing apparatus may assign manual patient information data corresponding to the sequence of characters in the one or more patient entry fields to the procedure session.

After assigning the manual patient information data to the procedure session the video processing apparatus may cease display of the patient entry panel and may displays a detailed section comprising detailed patient information within the second portion of the graphical user interface. The video processing apparatus may display the patient information panel, e.g. comprising the detailed section, within the second portion of the graphical user interface. The patient information panel may comprise a heading section. The video processing apparatus may display first patient information of the manual patient information data in the heading section.

The patient information panel, e.g. displayed after assigning the manual patient information data to the procedure session, may comprise an edit button. The video processing apparatus may be adapted to detect an edit button user input corresponding to selection of the edit button. In response to detecting the edit button user input the video processing apparatus may (re)display the patient entry panel within the second portion of the graphical user interface comprising the one or more patient info entry fields.

The patient information panel, e.g. displayed after assigning the manual patient information data to the procedure session, may comprise a delete button. The video processing apparatus may be adapted to detect a delete button user input corresponding to selection of the delete button. In response to detecting the delete button user input the video processing apparatus may display a confirmation dialogue, e.g. including a confirm button and/or a cancel button. The video processing apparatus may be adapted to detect a confirm user input corresponding to selection of the confirm button. The video processing apparatus may be adapted to detect a cancel user input corresponding to selection of the cancel button.

In accordance with detecting the confirm user input the video processing apparatus may remove the assignment of the manual patient information data to the procedure session. The video processing apparatus may further cease display of the confirmation dialogue and may display the manual entry button within the second portion of the graphical user interface. For example, the video processing apparatus may display the patient information panel comprising the manual entry button.

In accordance with detecting the cancel user input the video processing apparatus may maintain the assignment of the manual patient information data to the procedure session. The video processing apparatus may further cease display of the confirmation dialogue and may redisplay the patient information panel, e.g. as displayed after assigning the manual patient information data to the procedure session, within the second portion of the graphical user interface.

The video processing apparatus may comprise a network interface.

The video processing apparatus may display a network drive setup within the first portion of the graphical user interface. The network drive setup may comprise a plurality of network drive input fields and a confirm button. The plurality of network drive input fields may include one or more of server name, server IP address, server port number, directory name, and user credentials.

While displaying the network drive setup, the video processing apparatus may be adapted to receive a sequence of one or more user inputs corresponding to input of a sequence of characters in the one or more network drive input fields. The video processing apparatus may be adapted to detect a confirm user input corresponding to selection of the confirm button.

In response to receiving the confirm user input the video processing apparatus may store network drive information for a first network drive corresponding to the sequence of characters in the one or more network drive input fields.

The video processing apparatus may comprise the network interface and/or an external communication interface, such as a USB interface.

The video processing apparatus may display, within the first portion of the graphical user interface, a plurality of representations corresponding to a plurality of stored data files stored during a procedure session.

Each of the plurality of representations corresponding to the plurality of stored image data files may comprise a selection indicator. The video processing apparatus may be adapted to detect a first selection user input corresponding to selection of a first selection indicator of a first representation of the plurality of representations. In response to detecting the first selection user input and in accordance with the first selection indicator not indicating current activation, the video processing apparatus may display the first selection indicator indicating activation. In response to detecting the first selection user input and in accordance with the first selection indicator indicating current activation, the video processing apparatus may display the first selection indicator not indicating activation.

The video processing apparatus, e.g. while displaying the plurality of representations corresponding to the plurality of stored data files, may further display an export button, e.g. within the third portion of the graphical user interface. The video processing apparatus may be adapted to detect an export user input corresponding to selection of the export button.

In response to detection of the export user input the video processing apparatus may display an export menu comprising an export confirm button. The video processing apparatus may be adapted to receive an export confirm user input corresponding to selection of the export confirm button. The export menu further may comprise a plurality of selectable export modalities including export to a first PACS server and optionally export to a first network drive (such as the first network drive mentioned above) and/or export to a first external storage device (e.g. a USB drive). The video processing apparatus may be adapted to receive a modality selection user input corresponding to selection of a selected export modality of the plurality selectable export modalities. In some examples the first network drive may be an SMB network drive.

In response to detecting the export confirm user input, and in accordance with the selected export modality corresponding to the export to the first PACS server, the video processing apparatus may transmit stored image data corresponding to one or more of the plurality of stored data files to the first PACS server.

In response to detecting the export confirm user input, and in accordance with the selected export modality corresponding to the export to the first network drive, the video processing apparatus may transmit stored image data corresponding to one or more of the plurality of stored data files to the first network drive.

The video processing apparatus may transmit the stored image data corresponding to the one or more of the plurality of stored image data files to a location on the first network drive based on stored network drive information for the first network drive at the video processing apparatus. For example, the stored network drive information may be the network drive information stored based on the content of the plurality of network drive input fields of the network drive setup. Alternatively or additionally, the location may be based on details of the procedure session. For example, a folder of the location may be named based on a date of the procedure and/or a patient identifier (e.g. patient name and/or patient identification number).

In response to detecting the export confirm user input, and in accordance with the selected export modality corresponding to the export to the first external storage device, the video processing apparatus may transmit stored image data corresponding to one or more of the plurality of stored image data files to the first external storage device (e.g. the first USB drive).

The video processing apparatus may transmit the stored image data corresponding to the one or more of the plurality of stored image data files to a location on the first external storage device based on details of the procedure session. For example, a folder of the location may be named based on a date of the procedure and/or a patient identifier (e.g. patient name and/or patient identification number).

The stored image data transmitted to the first PACS server, the first network drive or the first USB drive may correspond to one or more of the plurality of stored image data files each having a selection indicator indicating current activation.

The stored image data transmitted to the first network drive and/or to the first USB drive may comprise the one or more of the plurality of stored images files in a common picture file format, e.g. PNG, JPEG, or TIFF. In the case where the stored image data is transmitted to the first PACS server, the stored image data may comprise the one or more of the plurality of stored image files in DICOM format.

The present disclosure further relates to a method for operating a video processing apparatus to receive image data from an endoscope, the claimed method being performed contemporaneously with an unclaimed surgical procedure, the endoscope having an image sensor configured to generate image data indicative of a view from the endoscope, the method comprising the steps of:
- receive the image data from the endoscope as the image data is being generated and cause a display to display a graphical user interface extending a first length along a first direction and a second length along a second direction perpendicular to the first direction, the graphical user interface comprising a plurality of non-overlapping portions including a first portion and a second portion, wherein the first portion is arranged next to the second portion along the second direction;
- display a live representation of the image data within the first portion of the graphical user interface at a full size covering the first length along the first direction;
- detect a first user input signal indicative of a request to activate a freeze function;
- cease display of the live representation of the image data within the first portion of the graphical user interface;
- display within the first portion of the graphical user interface a first representation of a still image at full size, corresponding to the image data received when the first user input signal was detected; and
- display the live representation of the image data within the second portion of the graphical user interface, wherein the live representation is displayed at a reduced size smaller than the full size.

The surgical procedure is to be understood as the insertion of the endoscope or a part of the endoscope into a patient or a patient's cavity and any direct interaction of the endoscope with the patient.

In one aspect, the method may further comprise the steps of:
- detect a third user input signal indicative of a request to store the still image; and
- store an image data file corresponding to the image data received when the first user input signal was detected.

### BRIEF DESCRIPTION OF THE FIGURES

Embodiments of the disclosure will be described in more detail in the following with regard to the accompanying figures. The figures show one way of implementing the present disclosure and are not to be construed as being limiting to other possible embodiments falling within the scope of the attached claim set.
Fig. 1 schematically illustrates an exemplary medical visualization system,
Fig. 2 schematically illustrates an exemplary video processing apparatus,
Fig. 3 schematically illustrates an exemplary video processing apparatus,
Fig. 4 is a block diagram of an exemplary video processing apparatus,
Fig. 5 schematically illustrates an exemplary video processing apparatus and an exemplary external display connected to the video processing apparatus,
Figs. 6A-6H schematically illustrate parts of an exemplary graphical user interface in relation to a freeze function of the video processing apparatus,
Figs. 7A-7H schematically illustrate parts of an exemplary graphical user interface in relation to an exemplary procedure for setting up a shortcut functionality,
Figs. 8A-8D schematically illustrate parts of an exemplary graphical user interface in relation use of a shortcut functionality,
Figs. 9A-9D schematically illustrate parts of exemplary graphical user interfaces in relation to a hide functionality,
Figs. 10A-10J schematically illustrate parts of an exemplary graphical user interface in relation to a procedure for entering patient data,
Figs. 11A-11B schematically illustrate parts of an exemplary graphical user interface in relation to a procedure for entering patient data,
Figs. 12A-12D schematically illustrate parts of an exemplary graphical user interface in relation to procedures for exporting stored data files, and
Figs. 13A-13E schematically illustrate parts of an exemplary graphical user interface in relation to procedures for configuring export of stored data files.

### DETAILED DESCRIPTION

Various exemplary embodiments and details are described hereinafter with reference to the figures when relevant. It should be noted that the figures may or may not be drawn to scale and that elements of similar structures or functions are represented by like reference numerals throughout the figures. It should also be noted that the figures are only intended to facilitate the description of the embodiments. They are not intended as an exhaustive description of the invention or as a limitation on the scope of the invention. In addition, an illustrated embodiment need not have all the aspects or advantages shown. An aspect or an advantage described in conjunction with a particular embodiment is not necessarily limited to that embodiment and can be practiced in any other embodiment even if not so illustrated, or if not so explicitly described.

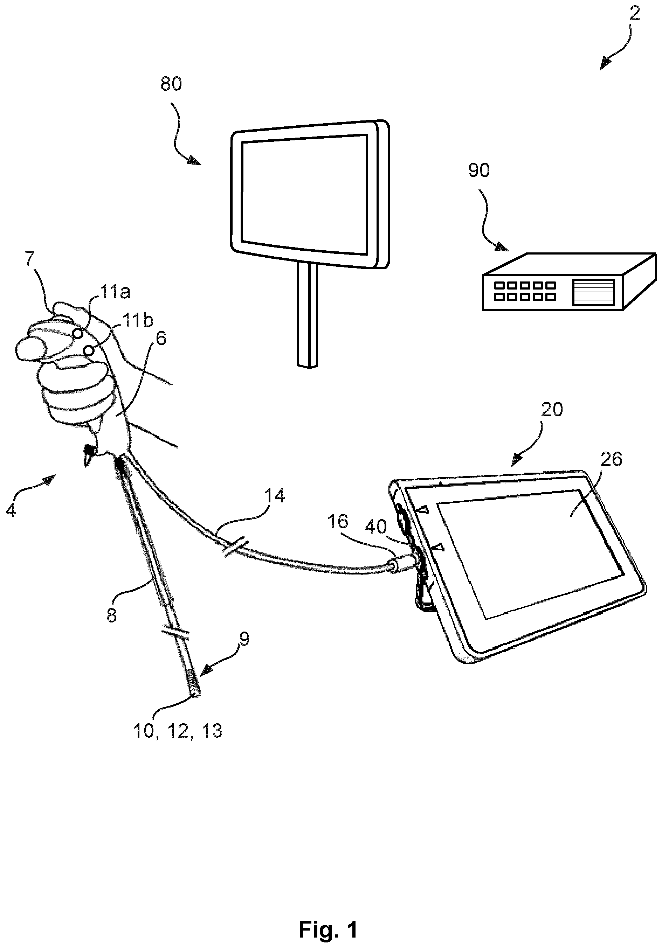

Fig. 1 schematically illustrates an exemplary medical visualization system 2 comprising an endoscope 4 and a video processing apparatus 20. The endoscope 4 has an image sensor 12, e.g. a CCD or a CMOS, configured to generate image data indicative of a view from the endoscope 4. In the illustrated example, the endoscope 4 comprises a handle 6 and an insertion cord 8, extending from the handle 6 to a distal cord portion 10. In alternative embodiments the endoscope may be a laryngoscope, an endotracheal tube, laryngeal masks, or other devices configured with a distal portion to be inserted into a patient and a proximal portion to be held or manipulated by a user. The image sensor 12 may be configured to generate image data indicative of a view from the distal cord portion 10 of the insertion cord 8. The endoscope 4 may further comprise a light emitter 13 adapted to provide illumination of the view. The light emitter 13 may be an LED, an optical fiber connectable to a light source, or similar element known to provide illumination. The image data may be preprocessed before presentation in a display. Preprocessing may comprise adjustment of image attributes, the attributes, e.g., including white balance, contrast, gamma, etc.

The endoscope 4 may be connected to the video processing apparatus 20. In the illustrated example, a device cable 14 extending from the handle 6 terminates in a device connector 16 connected to a connection port 40 of the video processing apparatus 20. The video processing apparatus 20 is operable to receive image data generated by the image sensor 12 of the endoscope 4, as the image data is being generated. For example, the video processing apparatus 20 may receive image data generated by the image sensor 12 via the device cable 14, the connector 16 and connection port 40. Alternatively, the endoscope 4 may be wirelessly connected to the video processing apparatus 20.

The video processing apparatus 20 is configured to cause display of the images received from the endoscope 4. The video processing apparatus 20 may comprise a display 26, such as a touch sensitive display, as illustrated, and/or may be connected to an external display 80, where the images received from the endoscope 4 are displayed. Thus, the video processing apparatus 20 may be configured to cause the display 26 and/or the external display 80 to display the images received from the endoscope 4. The external display 80 may form part of the medical visualization system 2. In some examples, the external display 80 and the video processing apparatus 20 may be connected by a wired connection, e.g. HDMI or DVI. Alternatively, the external display 80 and the video processing apparatus 20 may be connected by a wireless connection, e.g. wireless HDMI or another known protocol for wirelessly connecting a display.

The handle 6 comprises a control mechanism 7 adapted to receive an input in a first input direction and/or in a second input direction. The touch input in the first input direction on the control mechanism 7 causes a bending section 9 of the insertion cord 8 to bend in a first bending direction, e.g. via wires extending from the handle, through the insertion cord 8 to the bending section 9. The touch input in the second input direction on the control mechanism 7 causes the bending section 9 of the insertion cord 8 to bend in a second bending direction. The first input direction and the second input direction may be opposite. The first bending direction and the second bending direction may be opposite. Bending the bending section 9 of the insertion cord 8 may cause a movement of the distal end 10 and the image sensor 12 in a direction relative to the image sensor 12. Thereby, seeing an image generated by the image sensor 12, a direction, e.g. up or down, in the image may correspond to a respective input on the control mechanism 7.

The endoscope 4, such as the handle 6, may comprise one or more buttons 11a, 11b, which may activate various functions. For example, a button 11a or 11b on the handle may activate a function in the video processing apparatus 20, such as storing a still image or a video sequence. The one or more buttons 11a, 11b may be programmable, e.g. in the sense that the video processing apparatus may be configured to activate a user selected function when receiving a signal indicative of the user pressing a certain button 11a, 11b.

The system 2 may comprise a remote server 90, and the video processing apparatus 20 may be connected to the remote server 90, e.g. by a network (e.g. a LAN), either by wired connections or wireless connections. Alternatively, the video processing apparatus 20 may be connected directly to the remote server 90, either by a wired or wireless connection.

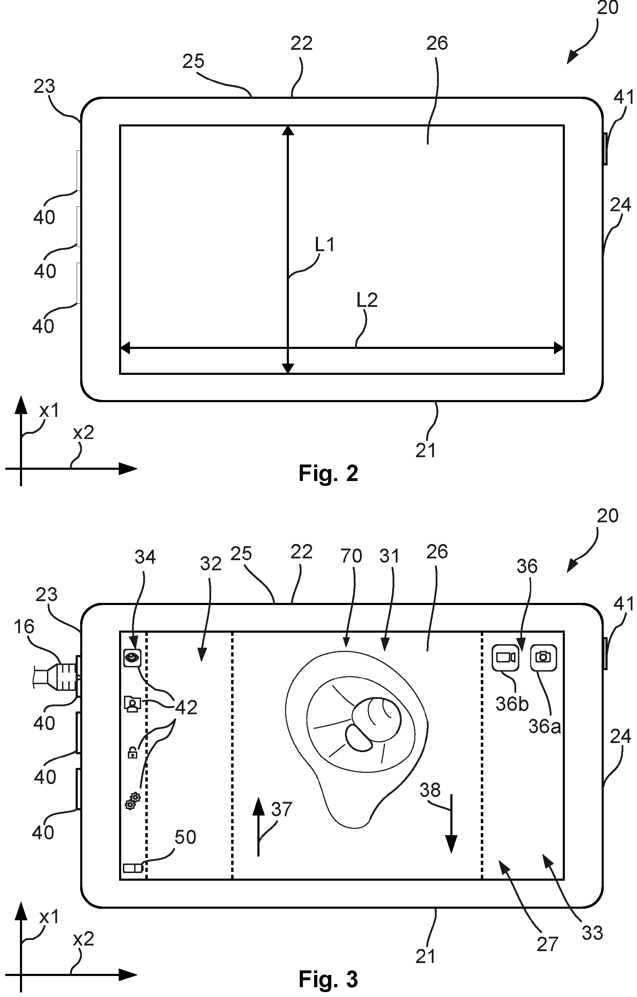

Fig. 2 schematically illustrates an exemplary video processing apparatus 20, such as the video processing apparatus 20 as illustrated in Fig. 1. The video processing apparatus 20 comprises a first housing 25. The first housing 25 extends in a first direction x1 from a first housing side 21 to a second housing side 22 and in a second direction x2 perpendicular to the first direction x1 from a third housing side 23 to a fourth housing side 24. The video processing apparatus 20 comprises a display, such as a touch sensitive display 26, accommodated in the first housing 25. The touch sensitive display 26 has a first length L1 in the first direction x1 and a second length L2 in the second direction x2. The second length L2 may be longer than the first length L1 as illustrated. In some examples, the video processing apparatus 20 may comprise a second housing (not shown), which does not comprise a display. For example, the second housing may be coupled to the first housing 25, or the second housing may be coupled to an external display. In such example, a processing unit, electronic memory and/or other components of the video processing apparatus 20 may be arranged in the second housing.

The video processing apparatus 20 may comprise one or more connection port(s) 40, such as three connection ports 40, as illustrated. The connection ports 40 may allow endoscopes to be connected. The connection port(s) 40 may be arranged at the third housing side 23, as illustrated. Alternatively or additionally, connection port(s) 40 may be arranged at the fourth housing side 24. In some examples, the video processing apparatus 20 may provide for wireless connection with the endoscopes, in which case the connection ports 40 may be omitted. However, in some examples, the video processing apparatus 20 may comprise both connection ports 40 and the ability to wireless connect to endoscopes. In some examples, where the video processing apparatus 20 comprises a second housing, as mentioned above, the second housing may comprise the connection ports 40.

The video processing apparatus may comprise an on/off button 41, which may be provided on the fourth housing side 24, as illustrated.

Fig. 3 schematically illustrates an exemplary video processing apparatus 20, such as the video processing apparatus 20 as illustrated in Figs. 1-2. As illustrated a device connector 16 may be connected to a connection port 40.

The video processing apparatus 20 may be provided with a graphical user interface 27. The graphical user interface 27 may be displayed with the touch sensitive display 26, and the user may interact with the graphical user interface 27, e.g. by means of providing touch inputs on the touch sensitive display 26. In some examples, the video processing apparatus may be provided without a touch sensitive display, and the user may interact with the graphical user interface by other means, e.g. by a keyboard, a trackpad or a mouse. In some examples, the video processing apparatus 20 may display the graphical user interface 27 on an external display (e.g. display 80 of Fig. 1). An external display may be provided as a supplement or as an alternative to a display of the video processing apparatus 20.

The graphical user interface 27 extends along the first direction x1 and along the second direction x2. The graphical user interface 27 may comprise a plurality of non-overlapping portions 31, 32, 33, 34. Each of the portions 31, 32, 33, 34 may extend substantially throughout the first length L1 in the first direction x1. In other words each of the portions 31, 32, 33, 34 may occupy the full height of the graphical user interface 27. The non-overlapping portions include a first portion 31 and a second portion 32. The first portion 31 is next to the second portion 32 along the second direction x2. In the presently illustrated example, the non-overlapping portions also include a third portion 33 and a fourth portion 34. The first portion 31 is arranged between the third portion 33 and the second portion 32 along the second direction x2. The second portion 32 is arranged between the fourth portion 34 and the first portion 31 along the second direction x2. The fourth portion 34 is arranged between a side of the first housing, e.g. the third housing side 23, and the second portion 32 along the second direction x2. The third portion 33 is arranged between another side of the first housing 25, e.g. the fourth housing side 24, and the first portion 31 along the second direction x2. The first portion 31 and the second portion 32 are arranged between the third portion 33 and the fourth portion 34 along the second direction x2.

The video processing apparatus 20 may display a live representation 70 of the image data within the first portion 31 of the graphical user interface 27. Preferably, the live representation 70 is displayed at a full size covering the first length L1 along the first direction x1, as illustrated.

The first bending direction and the second bending direction of the bending section 9 of the insertion cord 8, as described with respect to Fig. 1, may correspond to a first image direction 37 and a second image direction 38 of the live representation 70, respectively. The first image direction 37 and the second image direction 38 may be parallel to the first direction x1, as illustrated. The first image direction 37 and the second image direction 38 may be opposite, as illustrated. Thereby, a user operating the control mechanism 7 of endoscope 4 may cause movement of the bending section 9 of the insertion cord 8 to bend in a direction corresponding to the first image direction 37 or the second image direction 38 of the live representation 70.

The video processing apparatus 20 may display with the touch sensitive display 26 one or more actionable items 36, e.g. within the third portion 33 of the graphical user interface 27. The actionable items 36 may comprise an image capture button 36a, e.g. for storing an image data file corresponding to the image data received when the image capture button 36a was activated. Alternatively or additionally, the actionable items 36 may comprise a video capture button 36b, e.g. for storing a video sequence (i.e. a video data file) of image data corresponding to the image data received when the video capture button 36b was activated.

The video processing apparatus 20 may display with the touch sensitive display 26 one or more actionable menu items 42 within the fourth portion 34 of the graphical user interface 27. The actionable menu items 42 may, for example, comprise a login menu item for initiating a login procedure, a settings menu item for accessing a settings menu, an archive menu item for browsing an archive, and a default menu item for returning to a default view. Also, a battery indicator 50 may be displayed in the fourth portion 34.

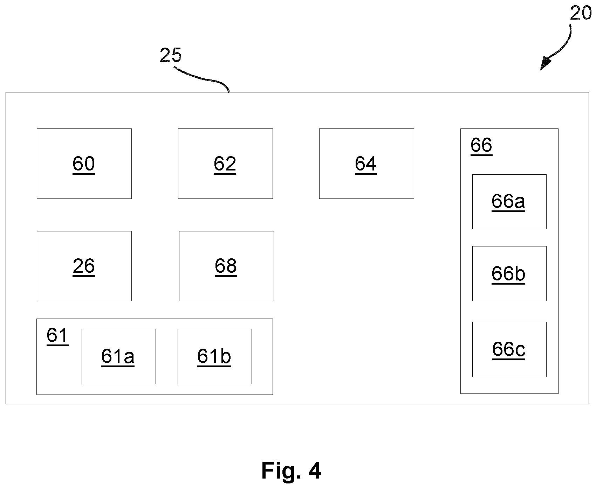

Fig. 4 is a block diagram of an exemplary video processing apparatus 20, such as the video processing apparatus 20 of the previous figures. The video processing apparatus 20 comprises a processing unit 60 and electronic memory 62. The electronic memory 62 may comprise both volatile and non-volatile memory. The video processing apparatus 20 may also comprise an orientation sensor 64 for determining the orientation of a housing, such as the first housing 25, relative to gravity. The orientation sensor 64 may comprise one or more accelerometers and/or a gyroscope. The video processing apparatus 20 may comprise an input/output module 66, such as for receiving image data from the image sensor 12 via connectors of the endoscope 4. The input/output module 66 may also comprise one or more network interfaces 66a, e.g. including ethernet connector, Wi-Fi transceiver and/or Bluetooth transceiver. The input/output module 66 may also comprise video connectors 66b (e.g. HDMI or DVI), USB ports 66c etc. The input/output module 66 may also comprise respective controllers. The video processing apparatus 20 also may comprise the touch sensitive display 26 as described earlier. The video processing apparatus 20 may display information, graphical user interface objects, images, buttons etc., with the touch sensitive display 26. The video processing apparatus 20 also may comprise a microphone 68. The video processing apparatus 20 may comprise a power unit 61 for powering the video processing apparatus 20. The power unit 61 may comprise a rechargeable battery 61a. The power unit 61 may comprise a power connection 61b for connecting the power unit 61 to an external power supply, such as a conventional AC power socket. The power connection 61b may provide for powering the video processing apparatus and/or for charging the battery 61a. The components of the video processing apparatus 20 may be interconnected by buses or signal lines. Some or all of the components of the video processing apparatus may be accommodated in the first housing 25 as illustrated. However, alternatively some of the components, e.g. the processing unit 60, the electronic memory 62, input/output module 66, the optical scanner 69, and/or the power unit 61 may be accommodated in a second housing (not shown) of the video processing apparatus 20.

The processing unit 60 may be a device or devices configured to execute machine-readable (coded) instructions, e.g. logic, to perform various functions. A processing unit typically converts coded instructions into timing and control signals that direct the operation of the other components of the device or system, such as memory, arithmetic logic unit, input and output devices, etc. Examples of integrated circuit controllers include complex programmable logic devices (CPLD), central processing units (CPU), graphic processing units (GPU), field programmable gate arrays (FPGAs), a master control unit (MCU) etc. The processing unit 60 may be a single integrated circuit part or may comprise more than one integrated circuit part. For example, the processing unit 60 may comprise a combination of a CPU and an FPGA, or a combination of a CPU, a GPU, and an FPGA. The FPGA and GPU may perform graphics processing functions while the MCU performs, as is known, timing and control functions. In an example where the processing unit 60 comprises more than one integrated circuit part, the integrated circuit parts may be linked in a supervised or a distributed manner. For example, a primary integrated circuit part may instruct other integrated circuit parts to execute tasks programmed for the other integrated circuit parts. Alternatively, the other integrated circuit parts may execute their functions independently.

The processing unit 60 may comprise a field programmable gate array (FPGA). FPGAs process data very fast compared to other non-volatile memory/instruction combinations and are reprogrammable. Therefore, FPGAs facilitate presentation of the live view of the images captured by the image sensor in real-time with minimal latency so that the physician observing the live view can take immediate actions even in emergency situations. The FPGA may process the raw image data generated by the videoscope by performing known optimization functions such as white balance, denoising and the like. The processing unit 60 may have embedded therein, or have access to, processing instructions embedded in non-volatile memory that allow the processing unit 60 to cause other devices to perform desired functions, such as presenting and controlling operation of a graphical user interface.

The electronic memory 62 may be machine-readable media. The electronic memory 62 may comprise multiple interconnected circuits, including a memory circuit embedded in the processing unit 60, a memory integrated circuit connected to the processing unit 60, a hard-drive or solid-state drive connected to the processing unit 60, and any other devices operable to store data and communicate with the processing unit 60. The electronic memory 62 may employ different storage mechanisms.

For example, a magnetic hard-drive, a solid-state drive, and a flash card may be examples of the electronic memory 62.

The power unit 61 may comprise components for, e.g. indirectly, measuring capacity of the rechargeable battery 61a. For example, the power unit 61 may comprise a voltage gauge to measure the voltage of the rechargeable battery 61a. Based on the measured voltage, the remaining capacity of the rechargeable battery 61a may be estimated, e.g. by the processing unit 60. The power unit 61 may also comprise components for measuring power consumption of the video processing apparatus 20. For example, the power unit 61 may comprise a power meter to measure the rate at which the video processing apparatus 20 consumes power from the rechargeable battery 61a. The voltage gauge may be a low current consumption integrated circuit or a resistor coupled in parallel with the battery. A current sensor may be provided and the power may be computed as the product of the voltage and current. Additionally, an integrated circuit may be provided that includes a voltage gauge and a current sensor, and which outputs a power value in digital form.

The video processing apparatus 20 may display content with the touch sensitive display 26 and/or with an externally coupled display (e.g. external display 80 of Fig. 1), which in some examples may be an external touch sensitive display. For example, the video processing apparatus 20 may display content by the processing unit 60 transmitting instructions to the touch sensitive display 26 and/or the externally coupled display indicative of the content to be displayed. The processing unit 60 may be adapted to receive the image data from the endoscope, e.g. as the image data is being generated. The processing unit 60 may be adapted to cause a display, such as the touch sensitive display 26 and/or the externally coupled display, to display a live representation of the image data. The processing unit 60 may be adapted to cause the display, such as the touch sensitive display 26 and/or the externally coupled display, to display the graphical user interface 27, as described above (see Fig. 2-3).

The video processing apparatus 20 may receive user input with the touch sensitive display 26. Particularly, the video processing apparatus 20 may detect user inputs with the touch sensitive display 26. For example, a user providing a touch input on the touch sensitive display 26 causes a change in one or more electrical parameters of the touch sensitive display 26 indicative of at least the location of the touch input. Information of the touch input is transmitted from the touch sensitive display 26 to the processing unit 60, and the processing unit 60 may determine whether the touch input corresponds to an action to perform, e.g. whether the location of the touch input corresponds to the location of a soft-button displayed at the touch sensitive display 26. Alternatively or additionally, the video processing apparatus 20 may receive user inputs by other means, e.g. by a keyboard, a trackpad or a mouse, which may be coupled to the video processing apparatus 20. As will be described later, the video processing apparatus 20 may also or alternatively receive user inputs by means of buttons on a coupled endoscope.

The user may interact with the video processing apparatus 20 via the graphical user interface 27 by providing user inputs, e.g. by means of providing touch inputs on the touch sensitive display 26, and the video processing apparatus 20 may detect such user inputs with the touch sensitive display 26. A touch input, e.g. a single tap, long press, double tap, swipe or similar, and the location of the touch input on the touch sensitive display 26 may be registered by the touch sensitive display 26, which transmits information of the touch input (e.g. including type of touch (double tap, long press, single tap, swipe, etc.) and/or location of the touch) to the processing unit 60 of the video processing apparatus 20. The processing unit 60 may interpret the information received and determine whether the touch input corresponds to activation of an action, e.g. whether the touch input correspond to activation of a button displayed with the touch sensitive display 26 at the location of the touch input. In response to a determination that the touch input corresponds to activation of an action, the processing unit 60 performs the respective action.

For example, with reference to Figs. 3 and 4, to capture an image corresponding to the presently shown live representation 70, e.g. corresponding to the image data received from the image sensor, the user may tap the image capture button 36a. The tap and the location of the tap is registered by the touch sensitive display 26, which transmits the information of the tap to the processing unit 60 of the video processing apparatus 20. The processing unit 60 interprets the information received and determines that the user tapped the location corresponding to the image capture button 36a. In response thereto, the processing unit 60 stores, in the electronic memory 62 an image data file corresponding to the image data received.

In further reference to Figs. 3 and 4, to capture a video sequence corresponding to the shown live representation 70 over a period of time, e.g. corresponding to the image data received from the image sensor over a period of time, the user may tap the video capture button 36b. The tap and the location of the tap is registered by the touch sensitive display 26, which transmits the information of the tap to the processing unit 60 of the video processing apparatus 20. The processing unit 60 interprets the information received and determines that the user tapped the location corresponding to the video capture button 36b. In response thereto, the processing unit 60 starts collection of image data received from the image sensor 12 and temporarily stores the data in the electronic memory 62. To stop the recording, the user may tap the video capture button 36b again. The processing unit 60 determines, based on the signal received from the touch sensitive display 26, that that the user tapped the video capture button 36b and stops collecting image data received from the image sensor 12. The processing unit 60 reads the temporarily stored data from the electronic memory 62 and creates a complete video sequence (e.g. a video data file) based thereon, which the processing unit 60 stores in the electronic memory 62.

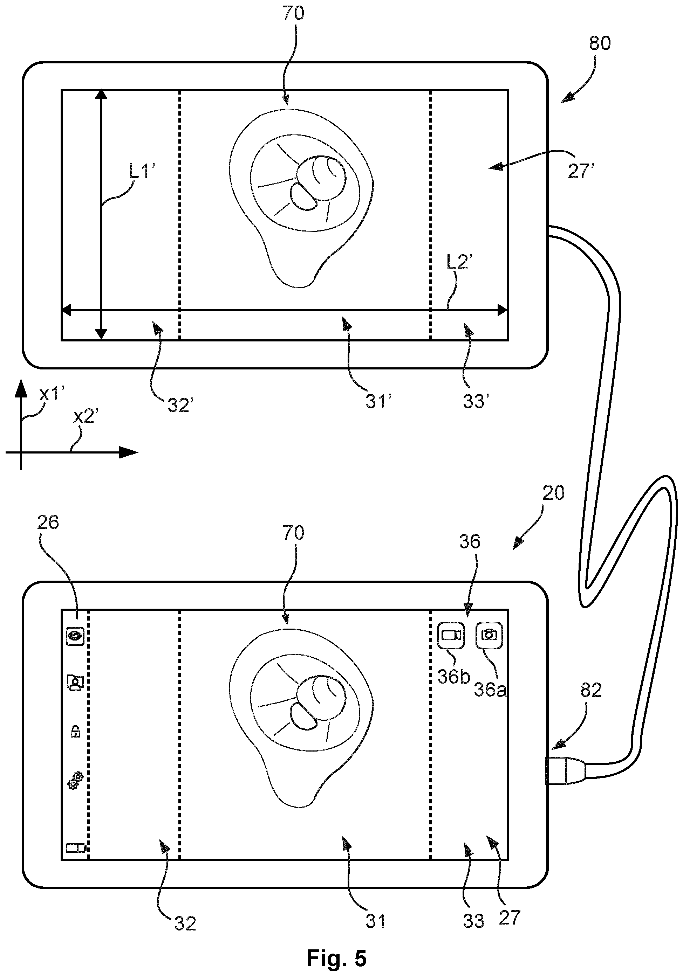

Fig. 5 schematically illustrates an exemplary video processing apparatus 20, such as the video processing apparatus 20 as illustrated in Figs. 1-4. Fig. 5 also schematically illustrate an exemplary external display 80, such as the external display 80 also illustrated in Fig. 1, being connected to the video processing apparatus 20.

The video processing apparatus 20 comprises a display interface 82 adapted to connect the video processing apparatus 20 to the external display 80. In the illustrated examples, connection between the video processing apparatus 20 and the external display 80 is provided by a wired connection. However, the connection may alternatively be provided by wireless technology. Accordingly, the display interface 82 may comprise DVI interface(s), HDMI interface(s), Wireless HDMI interface(s) and/or other suitable display interfaces.

The video processing apparatus 20 may be adapted to cause the external display 80 to display the secondary graphical user interface 27'. The secondary graphical user interface 27' may extend a first secondary length L1' along a first secondary direction x1' and a second secondary length L2' along a second secondary direction x2' perpendicular to the first secondary direction x1'. The secondary graphical user interface 27' comprises a plurality of non-overlapping secondary portions 31', 32', 33' including a first secondary portion 31' and a second secondary portion 32', wherein the first secondary portion 31' is arranged next to the second secondary portion 32' along the second secondary direction x2. The plurality of non-overlapping secondary portions 31', 32', 33' may further include a third secondary portion 33'. As illustrated the first secondary portion 31' may be arranged between the second secondary portion 32' and the third secondary portion 33'.

The video processing apparatus 20 may be adapted to display the live representation 70 of the image data within the first secondary portion 31' of the secondary graphical user interface 27'. Preferably, the live representation 70 is displayed at a full size covering the first secondary length L1' along the first secondary direction x1', as illustrated.

Preferably, the content, e.g. the live representation 70, being displayed in the first portion 31 of the graphical user interface 27 of the video processing apparatus 20, is displayed in the first secondary portion 31' of the secondary graphical user interface 27'. In effect, the first secondary portion 31' of the secondary graphical user interface 27' may be a mirror of the first portion 31 of the graphical user interface 27.

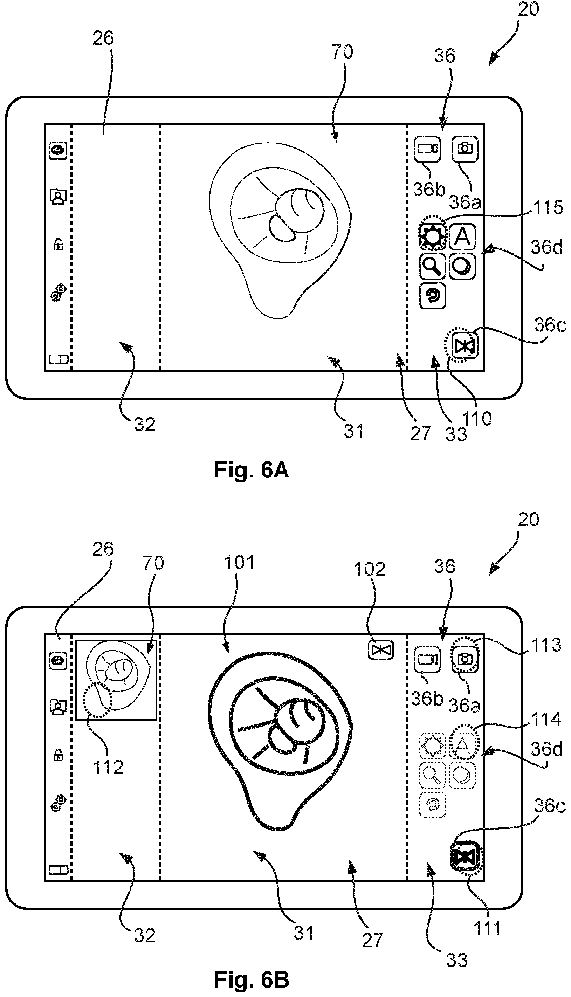

Figs. 6A-6H schematically illustrate parts of an exemplary graphical user interface 27, such as the graphical user interface 27 as described in relation to the previous figures. More specifically, Figs. 6A-6H are related to the graphical user interface 27 in relation to a freeze function of the video processing apparatus 20.

Fig. 6A illustrates the graphical user interface 27 comprising display of a live representation 70 of the image data within the first portion 31.

The video processing apparatus 20 is adapted to detect a first user input signal indicative of a request to activate a freeze function. For example, the video processing apparatus may be adapted to detect a first endoscope signal from the endoscope 4 (cf. Fig. 1), and the first user input signal may be instigated by detection of the first endoscope signal. For example, the first endoscope signal may be indicative of a user activating a first button (e.g. button 11a of Fig. 1) on the endoscope 4. In effect, the user may, by activating a first button on the endoscope, instigate the first user input signal, and accordingly request activation of the freeze function. Alternatively or additionally, The video processing apparatus 20 displays a freeze button 36c, e.g. as part of the one or more actionable items 36, e.g. within the third portion 33 of the graphical user interface 27. The video processing apparatus 20 may be adapted to detect a user input 110 corresponding to selection of the freeze button 36c, and the first user input signal may be instigated by detection of the user input 110 corresponding to selection of the freeze button 36c. In effect, the user may, by selecting the freeze button 36c (e.g. by the touch input 110), instigate the first user input signal, and accordingly request activation of the freeze function.

In response to detecting the first user input signal the video processing apparatus activates the freeze function, as schematically illustrated in Fig. 6B. Activating the freeze function includes ceasing display of the live representation 70 within the first portion 31 and displaying within the first portion 31 a first representation 101 of a still image corresponding to the image data received when the first user input signal was detected, e.g. as instigated by the user selecting 110 the freeze button 36c or activating the first button on the endoscope. Thus, it should be understood, that the first representation 101 in Fig. 6B is a static image, while the live representation 70 in Fig. 6A is a dynamic image. The first representation 101 is displayed at the full size, i.e. covering the entire first length L1 (i.e. the height, see Fig. 2) of the graphical user interface 27. The still image may be based on the image data received and/or the frame of the live representation being displayed, when the first user input signal was detected. GUI logic may be configured to capture the still image from a buffer containing, albeit temporarily, the image data as it is received from the endoscope or from another buffer. As is known in the art, a buffer is memory designated to contain something being worked on. For that reason, memory used as a buffer is generally faster to read and write to than other memory. An image buffer may contain images. The GUI logic may be configured temporarily store the captured still image in case the user wishes to take actions intended to store a non-volatile copy. Meanwhile, the processing unit may continue to buffer image data for displaying the live representation 70 and/or performing one or more image modifying functions.

Furthermore, as illustrated in Fig. 6B, activating the freeze function also includes displaying the live representation 70 within the second portion 32. The live representation 70 is displayed at a reduced size, smaller than the full size. In other words when activating the freeze function the live representation 70 is displayed at a size smaller than the size of the first representation 101 (the still image).

Thus, in effect the live representation 70 is made smaller and moved to the second portion 32 of the graphical user interface 27, while a first representation 101 of a still image is displayed in full size in the first portion 31 of the graphical user interface 27. In this way, the operator is able to verify that indeed the finding that he or she wants to confirm was visible in the picture, when they activated the freeze function. This may be used to inspect a certain finding without having to continuously maintain a sufficient view of the finding. Furthermore, the present disclosure provides that while allowing the operator to inspect a representation 101 of a still image, he/she is still provided the live representation 70, to enable the operator to see and possibly react to a present position of the endoscope. It is a further advantage of the present disclosure that neither the live representation 70 nor the first representation 101 is wholly or partly obscured, to enable the operator to fully inspect any visible information in the pictures.

As illustrated in Fig. 6B, while the freeze function is activated, and the first representation 101 of the still image being displayed in the first portion 31, a freeze indicator element 102 may also be displayed, e.g. within the first portion 31. The freeze indicator element 102 may provide a visible indication to the operator that what is being displayed is the still image and not the live representation 70. The freeze indicator element 102 may overlay a portion of the first representation 101. The freeze indicator 102 may be partly transparent to reduce impairing any information in the still image.

While the freeze function is active, as illustrated in Fig. 6B, the video processing apparatus 20 may be adapted to detect a second user input signal indicative of a request to deactivate the freeze function. For example, the video processing apparatus may be adapted to detect a second endoscope signal from the endoscope 4 (cf. Fig. 1), and the second user input signal may be instigated by detection of the second endoscope signal. For example, the second endoscope signal may be indicative of a user activating a second button and/or the first button (e.g. button 11a or 11b of Fig. 1) on the endoscope 4. Thus, the second endoscope signal may be triggered by the same button triggering the first endoscope signal, as described previously (e.g. button 11a of Fig. 1). In effect, the user may, by activating the first button on the endoscope again, instigate the second user input signal, and accordingly request deactivation of the freeze function. Alternatively, or additionally, the video processing apparatus 20 may be adapted to detect a further user input 111 corresponding to selection of the freeze button 36c, and the second user input signal may be instigated by detection of the further user input 111 corresponding to selection of the freeze button 36c. In effect, the user may, by (e.g. again) selecting the freeze button 36c (e.g. by the touch input 111), instigate the second user input signal, and accordingly request deactivation of the freeze function.

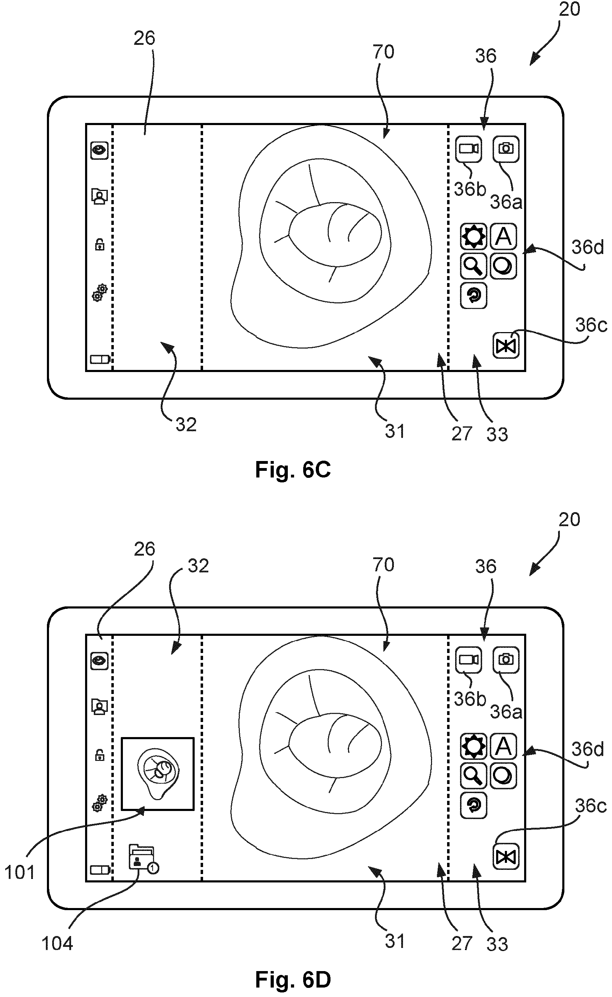

In response to detecting the second user input signal the video processing apparatus deactivates the freeze function, as schematically illustrated in Fig. 6C. Deactivating the freeze function includes ceasing display of the first representation 101 of the still image within the first portion 31 of the graphical user interface 27. Furthermore, the live representation 70 of the image data is again displayed, in full size, within the first portion 31 of the graphical user interface 27. The live representation 70 is, when the freeze function is deactivated, displayed at the full size, i.e. covering the entire first length L1 (i.e. the height, see Fig. 2) of the graphical user interface 27.

As alternatives or as supplements to the above-mentioned ways of deactivating the freeze function, the video processing apparatus 20 may be adapted to deactivate the freeze function based on other user inputs or state changes. For example, while the freeze function is active, as illustrated in Fig. 6B, the video processing apparatus may be adapted to detect a user input 112 directed to the live representation 70 of the image data being displayed within the second portion 32, and the second user input signal, causing deactivation of the freeze function, may be instigated by detection of the user input 112 directed to the live representation 70 of the image data. Alternatively, or additionally, the video processing apparatus 20 may be adapted to detect disconnection of the endoscope 4 (e.g. unplugging the device connector 16 from the connection port 40, see Fig. 1), and the second user input signal, causing deactivation of the freeze function, may be instigated in response to detecting the disconnection of the endoscope from the video processing apparatus 20. Alternatively, or additionally, the video processing apparatus 20 may be adapted to detect connection of an endoscope (e.g. inserting a device connector 16 into a connection port 40, see Fig. 1), and the second user input signal, causing deactivation of the freeze function, may be instigated in response to detecting the connection of an endoscope to the video processing apparatus. In some examples, the video processing apparatus 20 may be connected to a plurality of endoscopes simultaneously, e.g. a first endoscope and a second endoscope. This may for example be enabled by the video processing apparatus comprising a plurality of connection ports 40, as seen in Fig. 1. In such a situation, the second user input signal, causing deactivation of the freeze function, may be instigated in response to detecting the connection of a second endoscope to the video processing apparatus.

The second endoscope may be different from a first endoscope already being connected to the video processing apparatus 20.

While the freeze function is active, as illustrated in Fig. 6B, the video processing apparatus 20 may be adapted to detect a third user input signal indicative of a request to store the still image corresponding to the first representation 101. For example, the video processing apparatus may be adapted to detect a third endoscope signal from the endoscope 4 (cf. Fig. 1), and the third user input signal may be instigated by detection of the third endoscope signal. For example, the third endoscope signal may be indicative of a user activating a third button on the endoscope 4 (e.g. button 11b in Fig. 1). In effect, the user may, by activating a third button on the endoscope, instigate the third user input signal, and accordingly request storing of the still image corresponding to the first representation 101. Alternatively or additionally, the video processing apparatus 20 displays one or more actionable items 36, e.g. within the third portion 33 of the graphical user interface 27, wherein the one or more actionable items 36 comprise an image capture button 36a. The video processing apparatus 20 may be adapted to detect a user input 113 corresponding to selection of the image capture button 36a, and the third user input signal may be instigated by detection of the user input 113 corresponding to selection of the image capture button 36a. In effect, the user may, by selecting the image capture button 36a (e.g. by the touch input 113), instigate the third user input signal, and accordingly request storing of the still image corresponding to the first representation 101.

In response to detecting the third user input signal the video processing apparatus 20 stores in electronic memory of the video processing apparatus 20 an image data file corresponding to the image data received when the first user input signal was detected, i.e. when the freeze function was activated. Accordingly, the image data file stored by the video processing apparatus 20 corresponds to the first representation 101 currently being displayed in the first portion 31 of the graphical user interface 27.

When storing the image data file, while the freeze function is active, the stored image data file may comprise information indicative of the image data file being captured while the freeze function was active. This may provide information if reviewing the stored images at a later stage that a particular image was captured while the freeze function was activated. Such information may prove valuable during, e.g., quality inspections, reviews of procedures or similar. The information comprised in the stored image data file indicative of the image data file being captured while the freeze function was active may include the freeze indicator element 102, which may be included in the stored image, e.g. overlaid on the image.

Storing the still image corresponding to the first representation 101 may further cause deactivation of the freeze function. Thus, when the user stores the still image, it may be interpreted as an acknowledgement of accepting the still image, and an indication to continue with the procedure, and accordingly a request to deactivate the freeze function. Thus, the video processing apparatus 20 may, in response to detecting the third user input signal, further deactivate the freeze function, similarly as explained above.

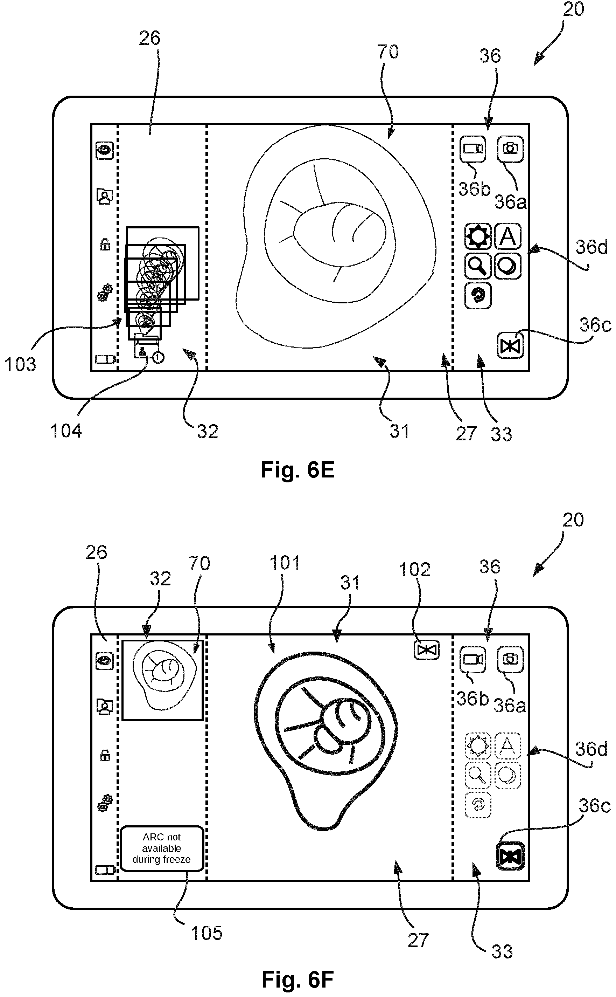

Fig. 6D schematically illustrates an exemplary result after the user has requested storing of the still image corresponding to the first representation 101, while the freeze function has been active, as illustrated in Fig. 6B, e.g. by the touch input 113 in Fig. 6B.

The video processing apparatus 20 may, in response to detecting the third user input signal, being indicative of the request to store the still image, display the first representation 101 of the still image within the second portion 32 of the graphical user interface 27. Thereby, the graphical user interface 27 signals to the user that the still image is being stored, and that it corresponds to the first representation 101. After a predetermined delay after detection of the third user input signal, i.e. after a predetermined delay after displaying the first representation 101 within the second portion 32, the video processing apparatus 20 may cease display of the first representation 101 of the still image within the second portion 32 of the graphical user interface 27. Before ceasing to display the first representation 101 within the second portion 32, the video processing apparatus 20 may display an animation 103, e.g. as illustrated in Fig. 6E of transitioning the first representation 101 to a folder icon 104.

The video processing apparatus 20 may comprise one or more image modifying functions. The one or more image modifying functions may result in the live representation 70 of the image data being visibly altered. For example, the one or more image modifying functions may include ARC (Advanced Red Contrast), color adjustment, contrast adjustment, sharpness adjustment, image brightness adjustment, zoom function, rotation of live representation, endoscope light on/off. The one or more image modifying functions (or some of them) may be activatable by image function buttons 36d, which may form part of the one or more actionable items 36, e.g. displayed within the third portion 33 of the graphical user interface 27. When activating an image modifying function of the one or more image modifying functions, e.g. by a user selecting one of the image function buttons 36d, one or more parameters of the live representation 70 of the image data may be modified. For example, contrast, color, sharpness, brightness, size, orientation etc. may be modified.

While the freeze function is not active, the one or more image modifying functions may be activatable, i.e. the one or more image modifying functions may work normally. While the freeze function is active, some or all image modifying functions may be not activatable, e.g. may be disabled. For example, as seen in Fig. 6B, when the freeze function is active, the image function buttons 36d, may be greyed out or in another way indicate that the image modifying functions are not activatable. In some examples, some of the image modifying functions may be activatable even when the freeze function is active. For example, it may be possible, even when the freeze function is active, to adjust the backlight of the display 26.

While the freeze function is active, as illustrated in Fig. 6B, the video processing apparatus 20 may detect, a fourth user input signal, such as the touch user input 114, as illustrated in Fig. 6B, indicative of a request to activate an image modifying function of the one or more image modifying functions. The fourth user input signal may alternatively or additionally to the touch user input 114 as illustrated, be instigated by the user pressing a certain button on the endoscope. In response to detecting the fourth user input signal, e.g. when the fourth user input signal is indicative of a request to activate an image modifying function which is not activatable while the freeze function is active, the video processing apparatus 20 may, as illustrated in Fig. 6F, display a notification 105, e.g. within the second portion 32 of the graphical user interface 27, indicating that the image modifying function is disabled and/or not available while the freeze function is active.

The video processing apparatus 20 may comprise, e.g. as one of the image modifying functions, an endoscope light switch function which when being activated instigates deactivation of a light emitter of the endoscope, such as the light emitter 13 as described in relation to Fig. 1. For example, as shown in Fig. 6A, the image function buttons 36d may include a button for toggling the endoscope light switch function, i.e. for activating and/or deactivating the light emitter of the endoscope. Alternatively, or additionally, the endoscope may comprise a button for toggling the endoscope light switch function. Being able to turn off the light emitter of the endoscope is convenient to avoid blinding other people in the room by the endoscope light before introducing it into the patient. As explained above, while the freeze function is not active, the endoscope light switch function may be activatable, and while the freeze function is active, the endoscope light switch function may be not activatable. Attempting to activate the endoscope light switch function, while the freeze function is active may result in a notification, like the notification 105 in Fig. 6F, indicating that the endoscope light switch function is not activatable while the freeze function is active.

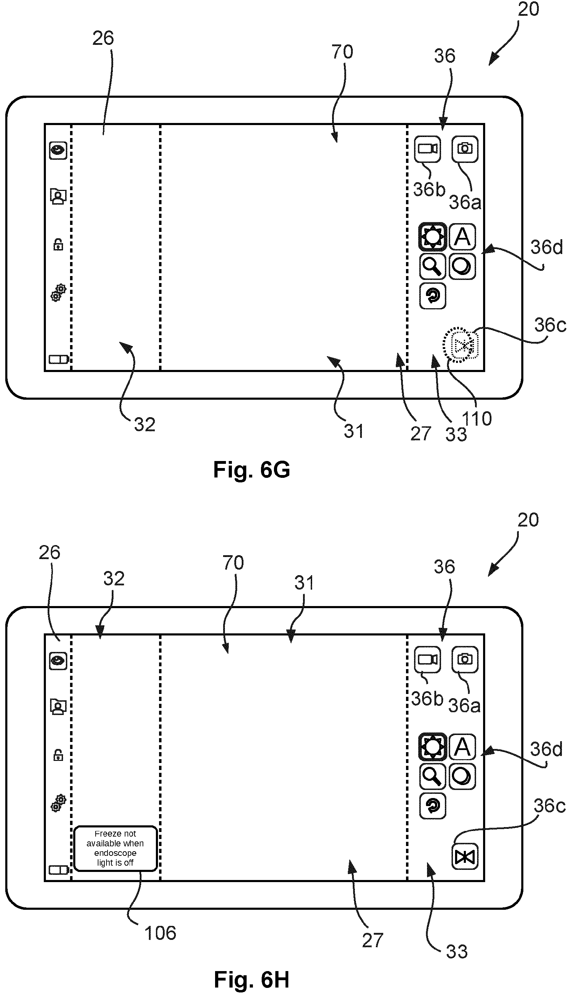

Activating the endoscope light switch function, e.g. by user input 115 illustrated in Fig. 6A, to deactivate the light emitter of the endoscope results, as illustrated in Fig. 6G in the live representation 70 not showing anything (or only very vaguely displaying something due to light from other light sources).

While the light emitter of the endoscope is deactivated the freeze function may be not activatable. In other words, the video processing apparatus 20 may be adapted such that it is not possible to activate the freeze function unless the light emitter of the endoscope is turned on. Accordingly, the freeze button 36c may be greyed out. In case the user attempts to activate the freeze function, while the light emitter of the endoscope is not active, e.g. by the user input 110 corresponding to selection of the freeze button 36c, or by pressing the first button (e.g. button 11a of Fig. 1) on the endoscope, the video processing apparatus 20 may forego activation of the freeze function. Optionally, the video processing apparatus 20 may display a notification 106, e.g. within the second portion 32 of the graphical user interface 27, as illustrated in Fig. 6H, indicating that the freeze function is not activatable while the light emitter of the endoscope is deactivated.

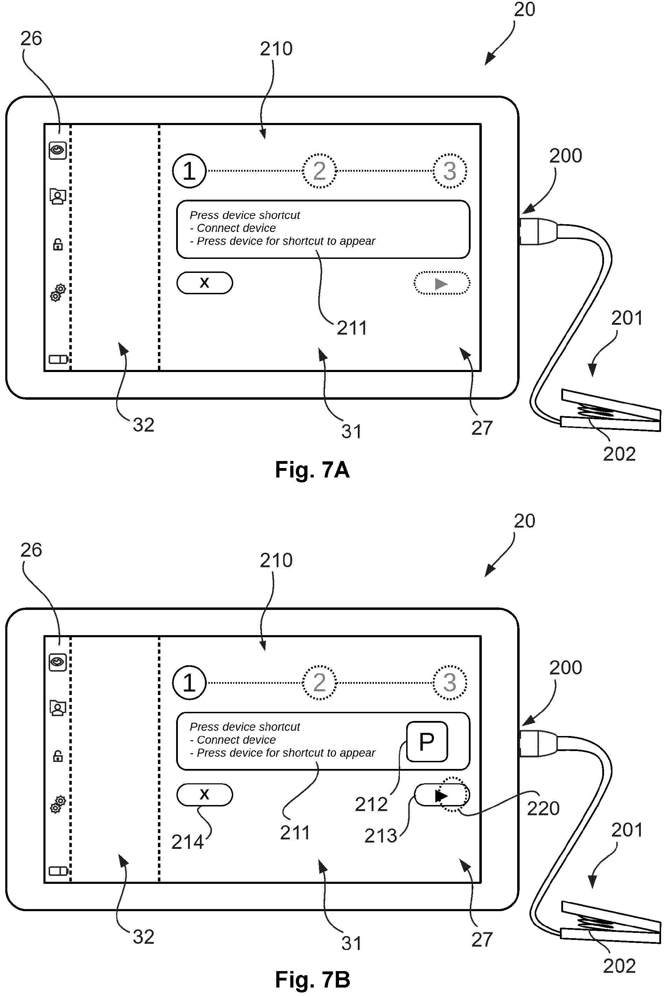

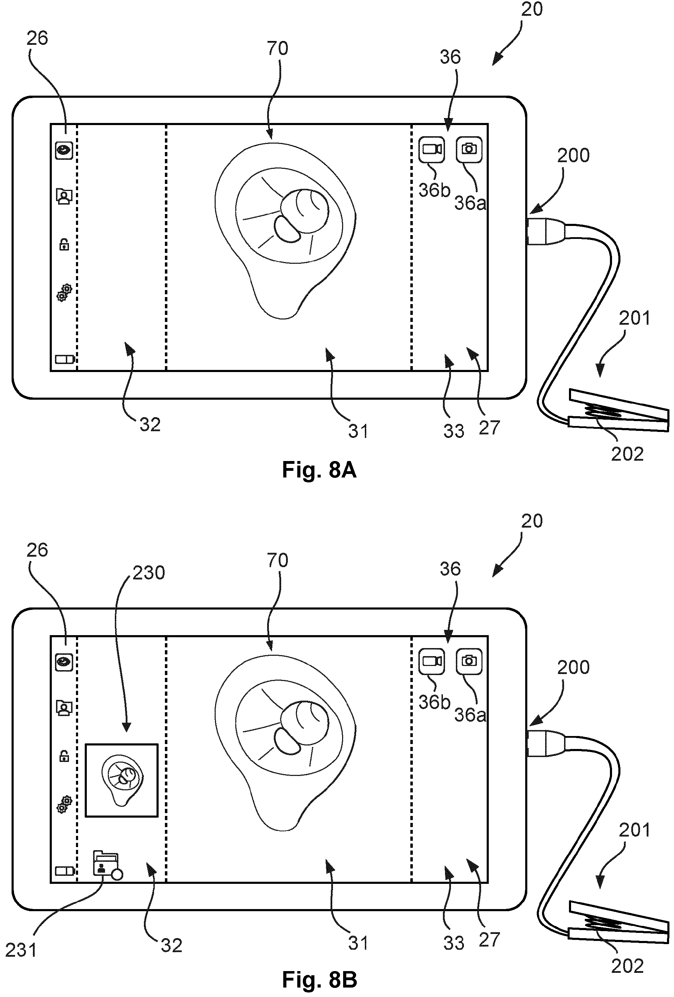

Figs. 7A-8D schematically illustrate parts of an exemplary graphical user interface 27, such as the graphical user interface 27 as described in relation to the previous figures. More specifically, Figs. 7A-8D are related to the graphical user interface 27 in relation to a shortcut functionality of the video processing apparatus 20. More specifically, Figs. 7A-7H are related to the graphical user interface 27 in relation to procedure for setting up a shortcut functionality, and Figs. 8A-8D are related to the graphical user interface 27 in relation to subsequent use of the shortcut functionality.

Fig. 7A illustrates that the video processing apparatus 20 may comprise a peripheral input device interface 200, e.g. comprising a USB interface, Bluetooth transceiver, and/or another interface type known in the art. The peripheral input device interface 200 may form part of the input output module 66 as described in relation to the block diagram in Fig. 4. In the example of Fig. 7A, the peripheral input device interface 200 comprises a wired type of interface, such as a USB interface, or similar. However, it might alternatively or additionally comprise a wireless interface, such as Bluetooth, or similar.

The peripheral input device interface 200 is adapted to couple the video processing apparatus 20 to one or more peripheral input devices 201, such as a foot pedal, a keyboard, a mouse, a trackpad, a game controller, or similar. In the illustrated example, a foot pedal is illustrated as the peripheral input device 201. In the illustrated example, the peripheral input device 201 is illustrated as being connected by a wired connection. However, alternatively the peripheral input device 201 could have been connected by a wireless connection. The peripheral input device 201 comprises an input component 202. In the illustrated example, the input component 202 is the pedal of the foot pedal 201 (or more specifically, the switch being activated by pressing the pedal). In some examples, one peripheral input device may have a plurality of input components. For example, a traditional PC keyboard may have 104 or 105 keys, and accordingly such keyboard may have up to 104 or 105 input components.

The peripheral input device interface 200 may be adapted to receive a peripheral input signal from the peripheral input device 201. In an example, where more than one peripheral input device is coupled to the peripheral input device interface 200, the peripheral input device interface 200 may be adapted to receive a plurality of peripheral input signals, e.g. one or more from each coupled peripheral input devices.

The peripheral input device interface 200 may further be adapted to transmit one or more secondary peripheral input signals to the processing unit of the video processing apparatus 20 indicative of the peripheral input signal(s) received.

The video processing apparatus 20 displays a shortcut setup user interface 210 within a first portion 31 of the graphical user interface 27. In the present example, and as illustrated, the first portion 31 may include also the third portion 33 of the graphical user interface 27 of Fig. 3. The shortcut setup user interface 210 may, as illustrated, comprise instructions 211 to connect a peripheral input device and/or to activate the desired input component of the peripheral input device. In some examples, the video processing apparatus 20 stores one or more user profiles each including a username and a password for activating an authorized mode of the video processing apparatus 20, and the shortcut setup user interface is displayed in accordance with the authorized mode being active. In other words, the shortcut setup user interface 210 may be restricted to only be available for authorized users of the video processing apparatus 20.

While displaying the shortcut setup user interface 210, the video processing apparatus 20 is adapted to detect a first peripheral input signal from the coupled peripheral input device 201. In response to detecting a first peripheral input signal, the video processing apparatus, displays, as illustrated in Fig. 7B, a shortcut indicator 212 indicative of the input component 202 of the peripheral input device 201 associated with the detected first peripheral input signal. Furthermore, a proceed button 213 may be displayed and enabled. Also, a cancel button 214 may be displayed, selectable to exit the procedure for setting up a shortcut and the shortcut setup user interface 210 without storing new shortcut information.

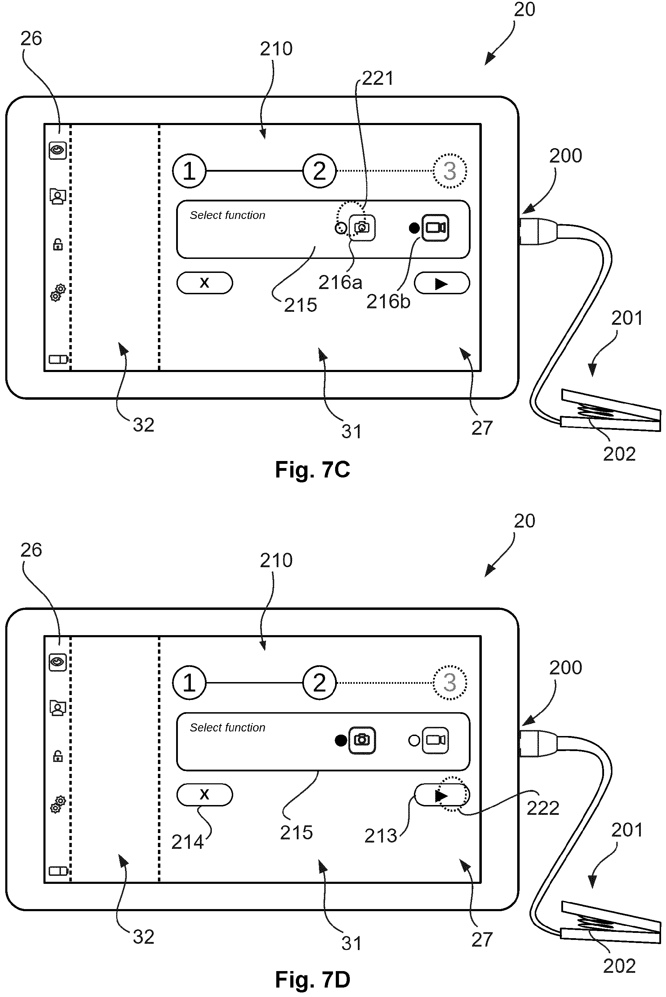

Further, in response to detecting the first peripheral input signal, e.g. after a user selects the proceed button 213, e.g. by touch user input 220, the video processing apparatus displays, as illustrated in Fig. 7C, a function selection interface 215. In the illustrated example, the function selection interface 215 forms part of the shortcut setup user interface 210. The function selection interface 215 comprises at least two representations 216a, 216b of two or more functions assignable to the input component 202 of the peripheral input device 201. In the present example, the two representations 216a, 216b, are of the capture image function and video capture function, respectively. In some examples, one of the functions may be preselected. In the illustrated example, the video capture function has been preselected as indicated by the radio button for the video capture representation 216b being filled.

While displaying the function selection interface 215, the video processing apparatus is adapted to detect a first user input 221 (e.g. a touch input) indicative of selection of a first function, e.g. the capture image function 216a, of the two or more functions. Selection of the first function (e.g. the capture image function) may be indicated, as illustrated in Fig. 7D, by the radio button for the capture image function representation 216a being filled.

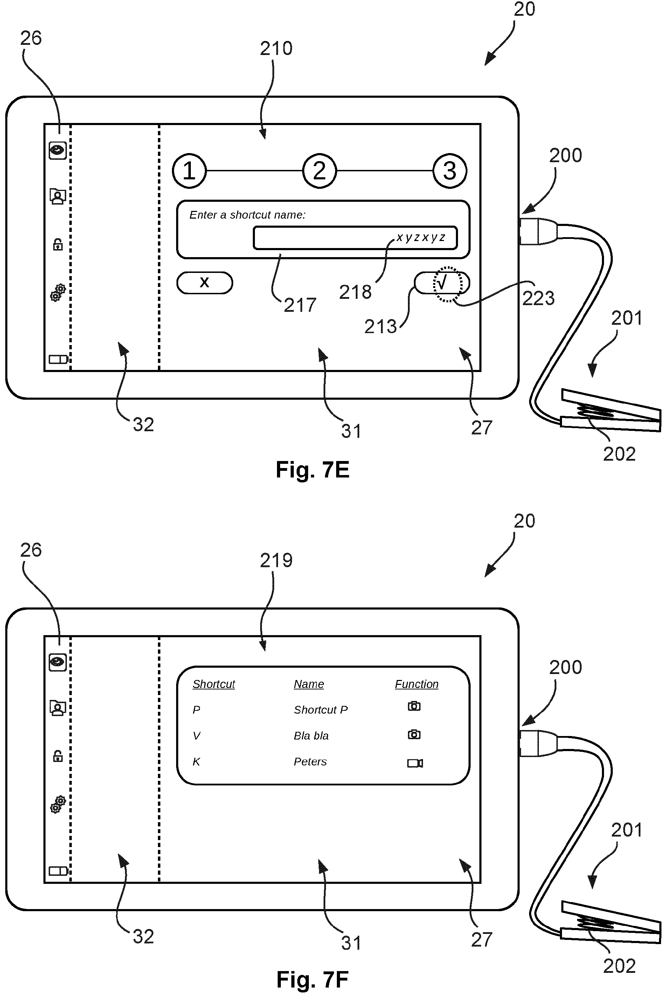

In some examples, as illustrated in Fig. 7E, further, in response to detecting the first peripheral input signal, e.g. after a user (again) selects the proceed button 213, e.g. by touch user input 222, the video processing apparatus displays a naming dialogue 217. The naming dialogue 217 is adapted to receive a user input name 218 for identifying the assignment of the first peripheral input signal to the selected function. In some examples, a default name is given to the shortcut if the user does not input a desired user input name 218.

In response to detecting the first user input 221, and optionally, further in response to the user selecting the proceed button 213 (again) , e.g. by touch user input 223, while displaying the naming dialogue 217, the video processing apparatus 20 stores first shortcut information including assignment of the first peripheral input signal corresponding to the input component 202 to the first function (e.g. the capture image function) corresponding to the function selected in the function selection interface 215 of Fig. 7C. The user input name 218, as provided in the naming dialogue 217 of Fig. 7E may be included in the stored first shortcut information. The proceed button 213 displayed together with the naming dialogue 217 may, as illustrated, have a difference appearance to indicate that selecting the proceed button 213 at this stage finalizes the shortcut setup procedure.

In response to the user selecting the proceed button 213, e.g. by touch user input 223, thereby concluding the procedure to setup a shortcut, the video processing apparatus, as illustrated in Fig. 7F may display a shortcut overview 219. The shortcut overview 219 may comprise options to delete one or more shortcuts from the list. The shortcut overview 219 may comprise options to add one or more further shortcuts.

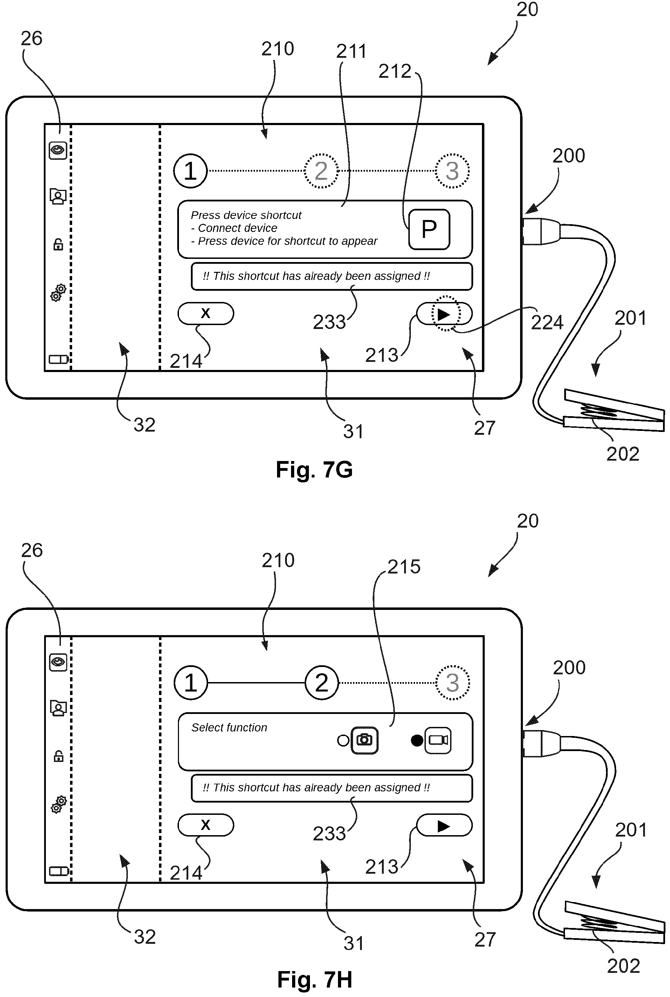

While displaying the shortcut setup user interface, as in Fig. 7A, and in response to detecting the first peripheral input signal, if the video processing apparatus already comprises shortcut information including assignment of the first peripheral input signal to a previous function (in other words, if the input component 202 of the peripheral device 201 is already assigned to a function), the video processing apparatus may provide a warning message 233 together with the shortcut indicator 212 indicative of the input component 202 of the peripheral input device 201 associated with the detected first peripheral input signal, as illustrated in Fig. 7G. In case the user wants to abort the potential reassignment of the input component 202, he/she may press the cancel button 214 to exit the shortcut setup user interface without storing new shortcut information. Alternatively, if the user wants to proceed he/she may press the proceed button 213.

In case the user proceeds by selecting the proceed button 213, e.g. by touch user input 224, the video processing apparatus may, as illustrated in Fig. 7H, display the function selection interface 215, like in Fig. 7C, with the addition of also displaying the warning message 233. The warning message 233 (both in Fig. 7G and 7H) may include information of the current assignment of the input component 202. For example, the warning message 233 may indicate that the input component 202 is assigned to the video capture function.

Fig. 8A schematically illustrates the graphical user interface 27, after the first shortcut information has been stored in accordance with the examples described above in relation to Fig. 7A-7H. For example, a shortcut may have been stored assigning the first peripheral input signal corresponding to the input component 202 to the capture image function of the video processing apparatus 20. The illustrated graphical user interface 27 comprises display of a live representation 70 of the image data within the first portion 31.

While displaying the live representation 70 and after the first shortcut information has been stored, the video processing apparatus is adapted to detect the first peripheral input signal from the first peripheral input device 201. In response to detecting the first peripheral input signal, e.g. corresponding to the pedal 202 of the foot pedal 201 being pressed down, the video processing apparatus, activates the assigned function, i.e. the capture image function. Fig. 8B schematically illustrates the capture of the image, in response to the user activating the foot pedal. As illustrated, the video processing apparatus 20 may, in response to detecting the first peripheral input signal, which in the present example is indicative of a request to store the still image, display a representation 230 of the still image within the second portion 32 of the graphical user interface 27. Thereby, signaling to the user that the still image is being stored, and that it corresponds to the live representation 70 when pressing the foot pedal 201. After a predetermined delay, the video processing apparatus 20 may display an animation of transitioning the representation 230 to the folder icon 231.

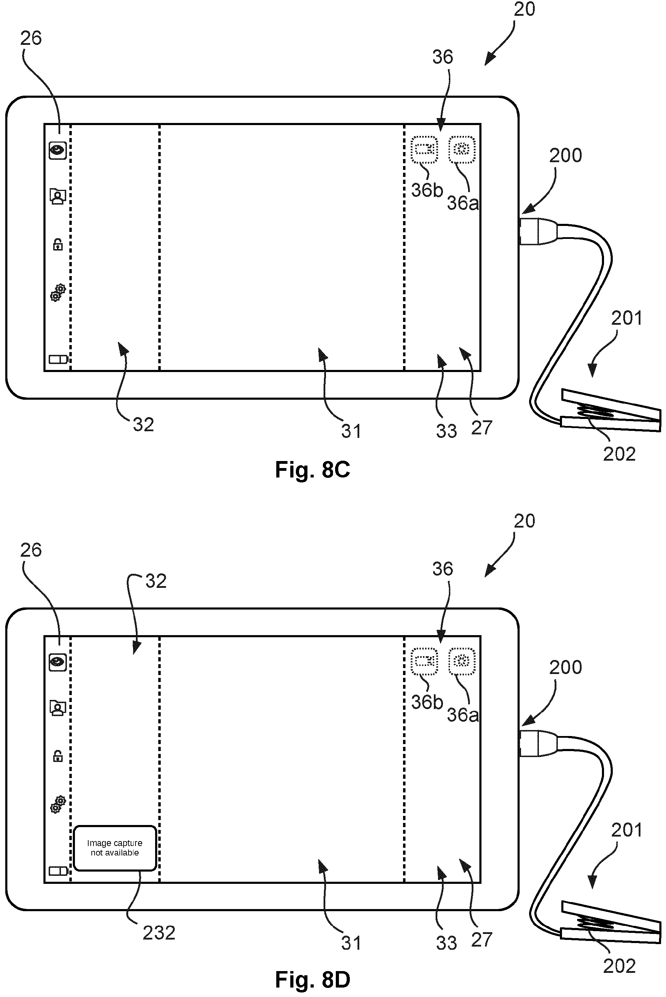

Fig. 8C, like Fig. 8A, schematically illustrates the graphical user interface 27 after the first shortcut information has been stored in accordance with the examples described above in relation to Fig. 7A-7H. However, as opposed to Fig. 8A, Fig. 8C illustrates an example where no live representation 70 is displayed by the video processing apparatus 20. This may be because no endoscope is connected to the video processing apparatus or it may be for other reasons. Thus, in this case, the function assigned to the peripheral input device 201, such as the capture image function, may be unavailable. Accordingly, the example of Fig. 8A may corresponds to a situation where the video processing apparatus 20 operates in a first mode where the function assigned to the peripheral input device 201 is available, and the example of Fig. 8C may correspond to a situation where the video processing apparatus 20 operates in a second mode where the function assigned to the peripheral input device 201 is not available. If in the situation where the video processing apparatus 20 operates in the second mode (Fig. 8C), and the video processing apparatus 20 detects the first peripheral input signal from the peripheral input device 201, the video processing apparatus may display an error notification 232, as illustrated in Fig. 8D. The video processing apparatus may further forego activating the assigned function, e.g. the video processing apparatus 20 may forego capturing a (blank) still image.

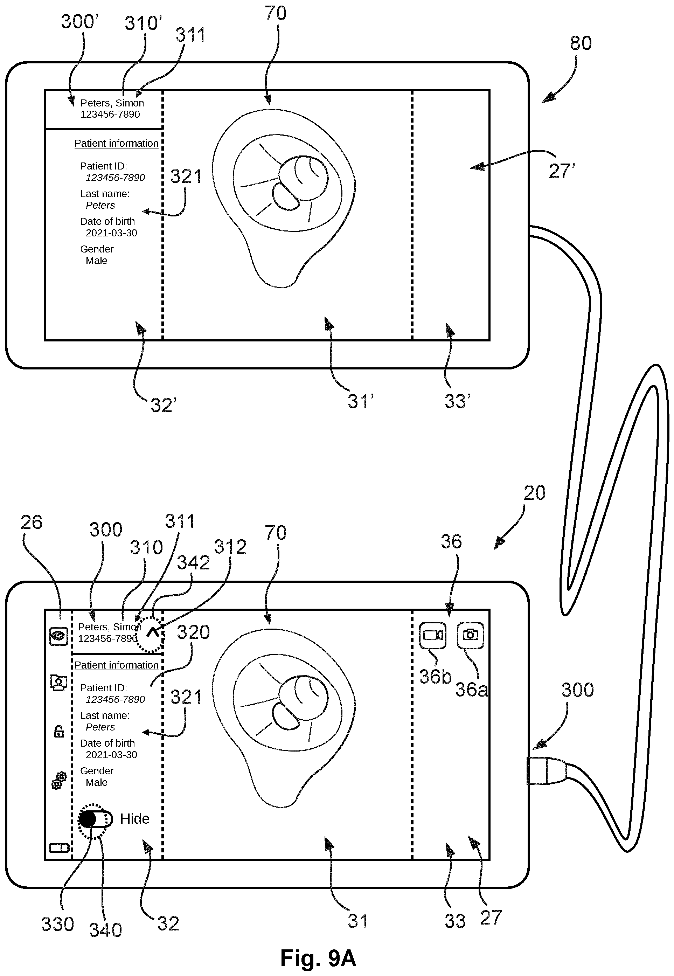

Figs. 9A-9D schematically illustrate parts of an exemplary graphical user interface 27 of the video processing apparatus 20, such as the graphical user interface 27 as described in relation to the previous figures. Figs. 9A-9D also schematically illustrate parts of an exemplary secondary graphical user interface 27' of an external display 80, such as the external display 80 also illustrated in Fig. 5, being connected to the video processing apparatus 20. While the below description is provided with respect to an example where an external display 80 is connected to the video processing apparatus 20, it will become clear in the following that some of the below explained features also provides certain advantages even when used in situations without the external display 80 being connected.

More specifically, Figs. 9A-9D are related to the graphical user interfaces 27, 27' in relation to a function giving an operator an ability to hide certain information, such as patient information. This may be advantageous in many situations, but specifically it may be mentioned that when using the video processing apparatus 20 and/or the external display 80 for presentation, demonstration and/or teaching purposes, where spectators are following the procedure, it is advantageous to be able to hide such information e.g. for privacy reasons.

Further to displaying a live representation 70, as illustrated, the video processing apparatus 20 displays a patient information panel 300 within the second portion 32 of the graphical user interface 27. The patient information panel 300 comprises a heading section 310, a detailed section 320 and a hide button 330. The heading section 310 may comprise first patient information 311, e.g. the name of the patient and/or personal identification number of the patient. The detailed section 320 may comprise detailed patient information 321 and optionally, the detailed section 320 may comprise the hide button 330. The hide button 330, in Fig. 9A, has a first appearance indicating that a hide data function is currently deactivated. The detailed patient information 321 may include the first patient information 311, i.e. the name and/or personal identification number of the patient, and/or the detailed patient information 321 may include additional patient information of the patient, e.g. date of birth and/or gender of the patient, as also illustrated in Fig. 9A.

In the illustrated example, where the external display 80 is connected to the video processing apparatus, the video processing apparatus 20 also displays the live representation 70 of the image data within the first secondary portion 31' of the secondary graphical user interface 27' on the external display 80. Furthermore, a secondary patient information panel 300' is displayed within the second secondary portion 32' of the secondary graphical user interface 27'. The secondary patient information panel 300' may comprise the detailed patient information 321. The secondary patient information panel 300' may comprise a secondary heading section 310' including the first patient information 311. As illustrated, the secondary patient information panel 300' may be different than the patient information panel 300 of the graphical user interface 27. However, the same, or some of the same, information may be displayed in the secondary patient information panel 300' as in the patient information panel 300 of the graphical user interface 27.

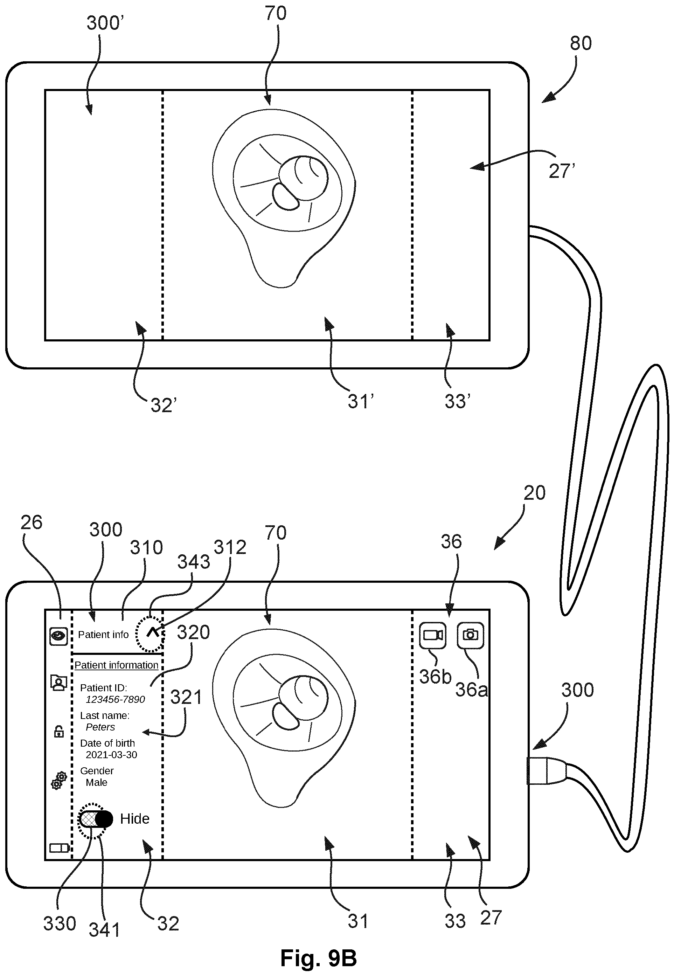

The video processing apparatus 20 is adapted to detect a hide user input 340 (e.g. touch user input) corresponding to selection of the hide button 330. In response to detecting the hide user input 340, the video processing apparatus, as illustrated in Fig. 9B activates the hide data function. Activating the hide data function may include changing the visual appearance of the hide button 330 to a second appearance, as illustrated in Fig. 9B.

Activating the hide data function, e.g. by the touch user input 340, includes ceasing display of the first patient information in the heading section 310. Furthermore, activating the hide data function may include ceasing display of the secondary patient information panel 300' on the external display 80. In some examples, activating the hide data function may include ceasing only part of the secondary patient information panel 300', while maintaining display of other elements of the secondary patient information panel 300'.

While the hide data function is active (as illustrated in Fig. 9B), the video processing apparatus 20 is adapted to (again) detect the hide user input 341 corresponding to selection of the hide button 330. In response to detecting the hide user input 341 while the hide data function is active, the video processing apparatus deactivates the hide data function.

Deactivating the hide data function includes displaying the first patient information in the heading section 310. Furthermore, deactivating the hide data function may include displaying the secondary patient information panel 300' on the external display 80 and/or displaying all parts of the secondary patient information panel 300' previously hidden. In essence, deactivating the hide data function results again in the state illustrated in Fig. 9A. Deactivating the hide data function may include changing the visual appearance of the hide button 330 to the first appearance, as illustrated in Fig. 9A.

Thus, in response to detecting the hide user input 340, when the hide data function is not active when detecting the hide user input 340 (Fig. 9A), the video processing apparatus activates the hide data function (resulting in the state illustrated in Fig. 9B). In response to detecting the hide user input 341, when the hide data function is active when detecting the hide user input 341 (Fig. 9B), the video processing apparatus deactivates the hide data function (resulting in the state illustrated in Fig. 9A).

The video processing apparatus may be further adapted to detect a detail user input 342-345 indicative of a request to toggle the detailed section 320 of the patient information panel 300. The detail user input 342-345 may correspond to selection of a collapse icon 312 of the heading section 310.

In response to detecting the detail user input 342-345, the video processing apparatus either ceases display of the detailed section 320 of the patient information panel 300 or displays the detailed section 320 of the patient information panel 300.

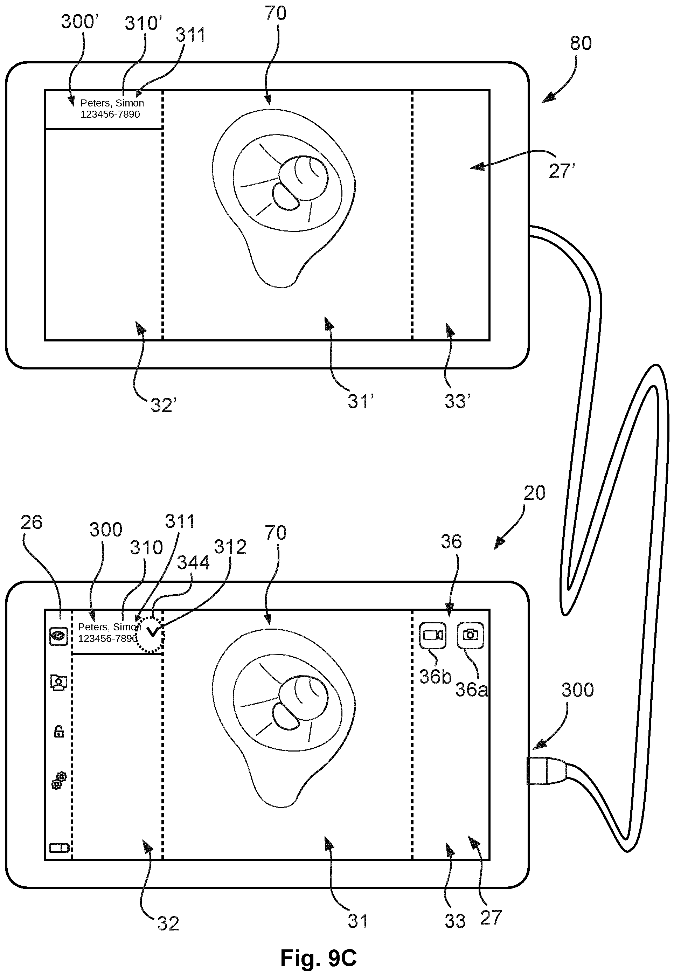

Accordingly, in accordance with the detailed section 320 being displayed when detecting the detail user input 342 (as in Fig. 9A), the video processing apparatus ceases display of the detailed section 320 of the patient information panel 300. For example, resulting in the state illustrated in Fig. 9C. In accordance with the detailed section 320 not being displayed when detecting the detail user input 344 (as in Fig. 9C), the video processing apparatus displays the detailed section of the patient information panel. For example, resulting in the state illustrated in Fig. 9A.

Similar behavior may be caused in the secondary graphical user interface 27' at the external display 80. Thus, in accordance with the detailed section 320 being displayed when detecting the detail user input 342 (as in Fig. 9A), the video processing apparatus ceases display of detailed patient information 321 of the secondary patient information panel 300'. As also illustrated in Fig. 9C. In some examples, the display of the entire secondary patient information panel 300' may be ceased. In accordance with the detailed section 320 not being displayed when detecting the detail user input 344 (as in Fig. 9C), the video processing apparatus displays the secondary patient information panel 300' including the detailed patient information 321, as illustrated in Fig. 9A.

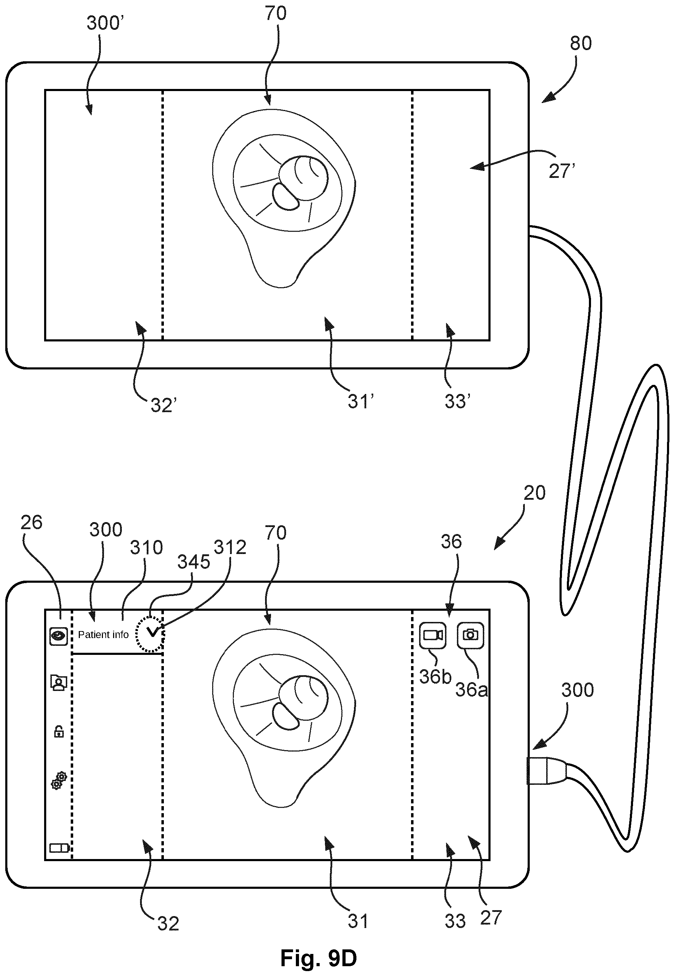

Figs. 9A and 9C illustrate the situation where the detail user input 342, 344 is detected while the hide data function is not active. The detail user input 343, 345 may also or alternatively be detected while the hide data function is active. This is illustrated in Figs. 9B and 9D.

Accordingly, in accordance with the hide data function being active and the detailed section 320 being displayed when detecting the detail user input 343 (as in Fig. 9B), the video processing apparatus ceases display of the detailed section 320 of the patient information panel 300. For example, resulting in the state illustrated in Fig. 9D. As opposed to Fig. 9C, where the hide data function is not active, the patient information panel 300 of Fig. 9D only shows the heading section 310, which also does not show the patient's name, but merely a generic "patient info" placeholder.

In accordance with the hide data function being active and the detailed section 320 not being displayed when detecting the detail user input 345 (as in Fig. 9D), the video processing apparatus displays the detailed section 320 of the patient information panel. For example, resulting in the state illustrated in Fig. 9B.

Furthermore, in accordance with the hide data function being active (e.g. Figs. 9B and 9D) when detecting the detail user input 343, 345, the secondary patient information panel 300', otherwise displayed within the second secondary portion 32' of the secondary graphical user interface 27', is not displayed.

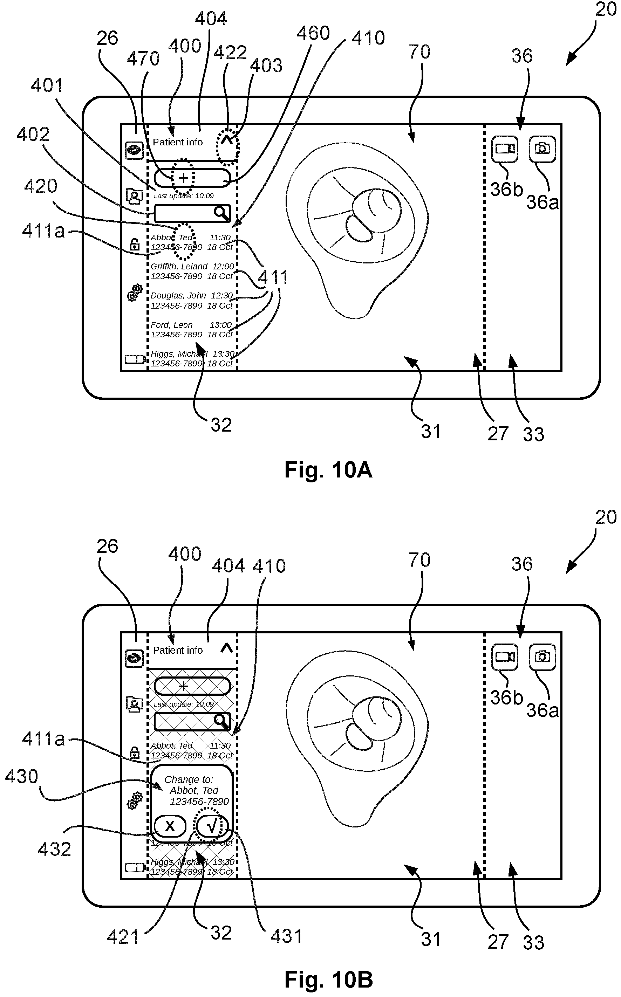

Figs. 10A-10J schematically illustrate parts of an exemplary graphical user interface 27, such as the graphical user interface 27 as described in relation to the previous figures. More specifically, Figs. 10A-10J are related to the graphical user interface 27 in relation to a procedure for entering patient data.

The video processing apparatus 20 may be adapted to open a procedure session. For example, the video processing apparatus 20 may be adapted to detect connection of an endoscope and open the procedure session in response to detecting connection of the endoscope. In response to detecting connection of the endoscope the video processing apparatus 20 may obtain device identifier information from a device identifier of the endoscope. For example, the endoscope may be fitted with an EPROM (alternatively a QR code, RFID tag, NFC etc. may be used), which the video processing apparatus 20 is able to read. For example, the processing unit of the video processing apparatus may execute a process for interrogating the device identifier, via the device connector and connection port. The EPROM may store information of the endoscope, e.g. a serial number of the endoscope, which may uniquely identify the endoscope. Also, the device identifier information may be indicative of the type of endoscope, e.g. whether it is a bronchoscope, a duodenoscope, a laryngoscope, etc., brand of the endoscope, production version, batch number etc.

After obtaining the device identifier information, the video processing apparatus 20 may open (create or reopen, depending on whether the endoscope has previously been connected) a procedure session corresponding to the device identifier information. For example, a first procedure session may be opened corresponding to first device identifier information obtained from a first endoscope, and a second procedure session may be opened corresponding to a second device identifier information obtained from a second endoscope. The procedure sessions may be unique, and therefore reconnecting a previously connected endoscope may cause the video processing apparatus 20 to reopen a previously created session. A procedure session may be implemented by creating a folder in the file system of the video processing apparatus 20, e.g. within the electronic memory of the video processing apparatus 20. Image data files and video sequences (e.g. video data files) obtained with a particular endoscope may be stored in the folder corresponding to the particular endoscope.

Fig. 10A illustrates the graphical user interface 27 comprising display of a live representation 70 of the image data within the first portion 31. Furthermore, a patient information panel 400 is displayed within the second portion 32 of the graphical user interface 27.

Either while displaying the live representation 70 and/or prior to displaying the live representation 70, the video processing apparatus 20 is adapted to retrieves worklist data from a remote server, such as the remote server 90 as described in relation to Fig. 1. The worklist data may comprise patient information data for a plurality of scheduled procedures, such as for scheduled procedures for the next 24 hours, or the next week, or another appropriate time interval. As also exemplary explained in relation to Fig. 1, the video processing apparatus and the remote server may be part of a local area network, and accordingly, the video processing apparatus may retrieve the worklist data via the local area network.

In some examples, the video processing apparatus may retrieve worklist data corresponding to procedures between a first time (e.g. 12 hours) prior to a current system time of the video processing apparatus and a second time (e.g. 24 hours) after the current system time. Thus, the worklist data retrieved may be limited to a timespan surrounding the current system time. Accordingly, the patient information data of the worklist data may consist of (e.g. be limited to) patient information data having a scheduled time for procedure between the first time prior to the current system time of the video processing apparatus and the second time after the current system time. Such implementation would limit the amount of data needed to be retrieved, while still allowing the user flexibility to select procedures, which were scheduled for the past, but which e.g. in view of delays was not performed when scheduled, as well as selection of procedures scheduled for the future in case it became possible to perform the procedure earlier.

In some examples, the video processing apparatus 20 may be adapted to detect connection of the endoscope. Connection of the endoscope may trigger receipt of the worklist data. Thus, the video processing apparatus 20 may in response to detecting connection of the endoscope retrieve the worklist data.

The patient information panel 400, as illustrated in Fig. 10A, comprises a worklist section 410 comprising a plurality of selectable patient information entries 411. The selectable patient information entries 411 corresponds to the patient information data of the worklist data retrieved from the remote server. Each of the plurality of selectable patient information entries 411 may comprise patient name, patient ID and/or scheduled time for procedure. The plurality of selectable patient information entries 411 of the worklist section 410 may be sorted in chronological order based on scheduled time for procedure.

In some examples, the video processing apparatus 20 stores one or more user profiles each including a username and a password for activating an authorized mode of the video processing apparatus 20, and the worklist section 410 is displayed in accordance with the authorized mode being active. In other words, the worklist section 410 may be restricted to only be available for authorized users of the video processing apparatus 20.

The patient information panel 400 may, as illustrated in Fig. 10A, comprise update information 401, which indicates when the worklist data was last retrieved/updated from the remote server. The update information 401 may be selectable by the user and/or a selectable icon may be provided, which when selected by the user results in the video processing apparatus may be adapted to retrieve the worklist data (such as updated worklist data) from the remote server.

The patient information panel 400 may, as illustrated in Fig. 10A, comprise a search field 402 of a search function. The search function may allow the user to input one or more characters in the search field 402. Accordingly, the video processing apparatus may be adapted to receive a sequence of inputs corresponding to inputs of a sequence of characters in the search field 402. The video processing apparatus, e.g. in response detecting the input of characters in the search field 402, may update the worklist section 410, such that the displayed selectable patient information entries 411 correspond to the one or more characters entered in the search field 402. The video processing apparatus 20 may in response to receiving the sequence of inputs filter the displayed selectable patient information entries 411 of the plurality of selectable patient information entries 411 based on a comparison between the sequence of characters in the search field 402 and the patient information data corresponding to the plurality of selectable patient information entries 411.

The video processing apparatus 20 is adapted to detect a first user input 420 (e.g. a touch user input), corresponding to selection of a first patient information entry 411a of the plurality of selectable patient information entries 411. In accordance with detecting the first user input 420, the video processing apparatus 20 assigns first patient information data corresponding to the first patient information entry 411a to the procedure session. Thus, in the illustrated example, the first user input 420 corresponds to selection of the patient information related to the patient "Abbot, Ted", and accordingly, Ted Abbot's information (e.g. name, identification number, scheduled time of procedure etc.) is assigned to the procedure session presently open and active.

In some examples, as illustrated in Fig. 10B, a confirmation dialogue 430 may be displayed by the video processing apparatus 20, e.g. in the second portion 32 of the graphical user interface 27, in response to detecting the first user input 420. The confirmation dialogue 430 may include a representation of the first patient information data, such as patient information related to the selected patient information entry 411a. The confirmation dialogue 430 may include a confirmation button 431 and optionally a cancel button 432. The confirmation button 431 may be selectable to proceed to assign the first patient information data corresponding to the first patient information entry 411a to the procedure session. The cancel button 432 may be selectable to forego assigning the first patient information data to the procedure session. Accordingly, the video processing apparatus 20 may be adapted to detect a confirmation user input 421 (e.g. a touch user input) corresponding to selection of the confirmation button 431. In response to detecting the confirmation user input 421, the video processing apparatus 20 may assign the first patient information data corresponding to the first patient information entry 411a to the procedure session.

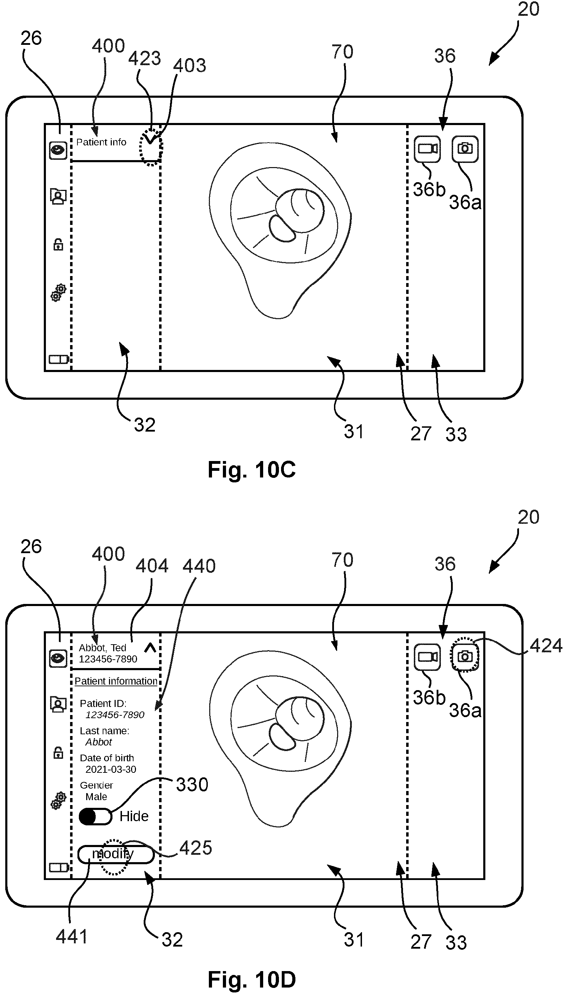

Returning to Fig. 10A, the video processing apparatus is adapted to detect a second user input 422 (e.g. a touch user input), e.g. directed to a collapse icon 403 of the patient information panel 400. The second user input 422 is indicative of a request to toggle display of the worklist section 410 of the patient information panel 400. In response to detecting the second user input 422 the video processing apparatus ceases display of the worklist section 410 of the patient information panel 400, as illustrated in Fig. 10C. If the worklist section 410 is not displayed, as in Fig. 10C, the second user input 423 directed to the collapse icon 403 (which may have changed appearance relative to Fig. 10A), the video processing apparatus 20 may, in response to detecting the second user input 423 (re)display the worklist section 410 of the patient information panel 400. Essentially resulting in the state illustrated in Fig. 10A. Thus, in other words, in accordance with the worklist section 410 being displayed (as in Fig. 10A) when detecting the second user input 422, the video processing apparatus may cease display of the worklist section 410 of the patient information panel 400 (as in Fig. 10C). And in accordance with the worklist section 410 not being displayed (as in Fig. 10C) when detecting the second user input 423, the video processing apparatus may display the worklist section of the patient information panel (as in Fig. 10A).

In some examples, the video processing apparatus 20 may, in response to detecting the second user input 423 while the worklist section 410 is not being displayed (as in Fig. 10C), retrieve the worklist data from the remote server. Thus, toggling display of the worklist section 410, particularly when toggling to display the worklist section 410, may cause updating and/or re-retrieving the worklist data from the remote server.

The patient information panel 400 may comprise a heading section 405. After assigning the first patient information data to the procedure session (see Fig. 10D), the video processing apparatus 20 may display first patient information (e.g. the name of the patient and/or personal identification number of the patient) of the first patient information data in the heading section 405.

After assigning the first patient information data to the procedure session, as illustrated in Fig. 10D, the video processing apparatus may cease display of the worklist section 410 of the patient information panel 400. Instead, the video processing apparatus 20 may display a detailed patient section 440, which may be similar to the detailed section 320 as explained in relation to Figs. 9A-9D. For example, the detailed section 440 may comprise a hide button 330 for toggling the hide data function, as described in relation to Figs. 9A-9D.

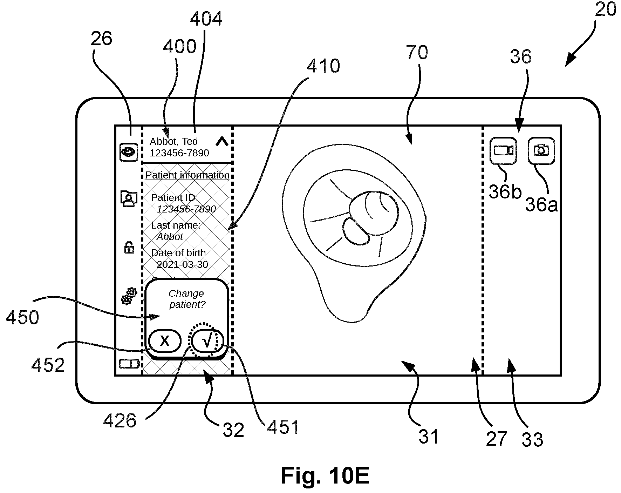

The patient information panel 400, such as the detailed patient section 440, may comprise a change patient button 442. The video processing apparatus 20 may be adapted to detect a change request user input 425 corresponding to selection of the change patient button 442. In response to detecting the change request user input 425, as illustrated in Fig. 10E, the video processing apparatus displays a confirmation dialogue 450 including a confirm button 451 and a cancel button 452. The confirmation dialogue 450 may be displayed in the second portion 32 of the graphical user interface 27, as illustrated. Alternatively, the confirmation dialogue 450 may be displayed in the first portion 31 of the graphical user interface 27. The video processing apparatus 20 is adapted to detect a confirm user input 426 corresponding to selection of the confirm button 451. The video processing apparatus 20 is also adapted to detect a cancel user input (not illustrated) corresponding to selection of the cancel button 452. In accordance with detecting the confirm user input 426, as illustrated, the video processing apparatus 20 removes the assignment of the first patient information data to the procedure session, ceases display of the confirmation dialogue 450, and redisplays the worklist section 410 in the patient information panel 400. In effect returning to the state as illustrated in Fig. 10A. In accordance with detecting the cancel user input, the video processing apparatus 20 maintains the assignment of the first patient information data to the procedure session and ceases display of the confirmation dialogue. In effect returning to the state as illustrated in Fig. 10D.

The video processing apparatus 20 may, as previously explained, display one or more actionable items 36, e.g. comprising an image capture button 36a and/or a video capture button 36b. The video processing apparatus 20 may be adapted to detect a capture user input 424 (e.g. a touch user input) corresponding to selection of the image capture button 36a. In response to detecting the capture user input 424 the video processing apparatus 20 may store an image data file corresponding to the image data received when the capture user input 424 was detected. Furthermore, the video processing apparatus may associate the stored image data file with the procedure session. Accordingly, having previously assigned the first patient information data to the procedure session as explained in relation to Figs. 10A-10C, the first patient information data is, in effect, coupled to the stored image data file. A similar effect may be achieved with respect to video capture if the user selects the video capture button 36b.

In some examples, the topmost displayed patient information entry of the patient information entries 411 is the next scheduled procedure, according to a system time of the video processing apparatus 20.

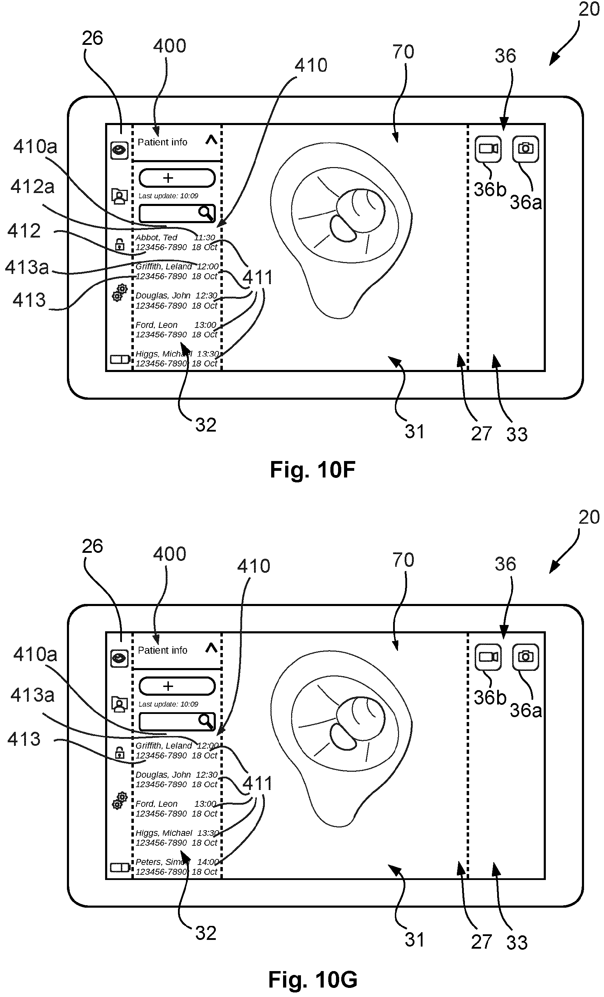

For example, as illustrated in Figs. 10F and 10G, the selectable patient information entries 411 comprise a primary selectable patient information entry 412 and a secondary selectable patient information entry 413. The primary selectable patient information entry 412 has a primary scheduled time for procedure 412a and the secondary selectable patient information entry 413 has a secondary scheduled time for procedure 413a. The secondary scheduled time for procedure 413a is later than the primary scheduled time for procedure 412a. Furthermore, the selectable patient information entries 411 do not comprise an intermediate selectable patient information entry having an intermediate scheduled time for procedure between the primary scheduled time for procedure 412a and the secondary scheduled time for procedure 413b. In other words, the secondary selectable patient information entry 413 is scheduled immediately following the primary selectable patient information entry 412.

In Fig. 10F the current system time of the video processing apparatus 20 is before 11:30, such as 11:15. In Fig. 10G the current system time of the video processing apparatus 20 is after 11:30, but before 12:00, such as 11:45.

The video processing apparatus 20, in displaying the patient information panel 400, in accordance with the current system time of the video processing apparatus 20 being before the primary scheduled time for procedure 412a (such as in Fig. 10F), the primary selectable patient information 412 is displayed in the worklist section 410 adjacent to a top 410a of the worklist section 410.

The video processing apparatus 20, in displaying the patient information panel 400, in accordance with the current system time of the video processing apparatus 20 being between the primary scheduled time for procedure 412a and the secondary scheduled time for procedure 413a (such as in Fig. 10G), does not display the primary selectable patient information 412 in the worklist section 410. Accordingly, the secondary selectable patient information entry 413a is displayed in the worklist section 410 adjacent to a top 410a of the worklist section 410.

The worklist section 410 may be scrollable. For example, the worklist section 410 may be scrollable in a first scroll direction (e.g. downwards in the exemplary figures) to show earlier selectable patient information entries of the plurality of selectable patient information entries 411 and in a second scroll direction (e.g. opposite the first scroll direction, such as upwards in the exemplary figures) to show later selectable patient information entries of the plurality of selectable patient information entries 411. The worklist section 410 may be scrollable in response to a touch and drag input (either upwards or downwards) on the touch sensitive display 26.

As illustrated in Fig. 10A, the patient information panel 400 may comprise a manual entry button 460. In some examples, if the worklist section 410 is restricted to be only available for authorized users, the manual entry button 460 may still be available for non-authorized users.

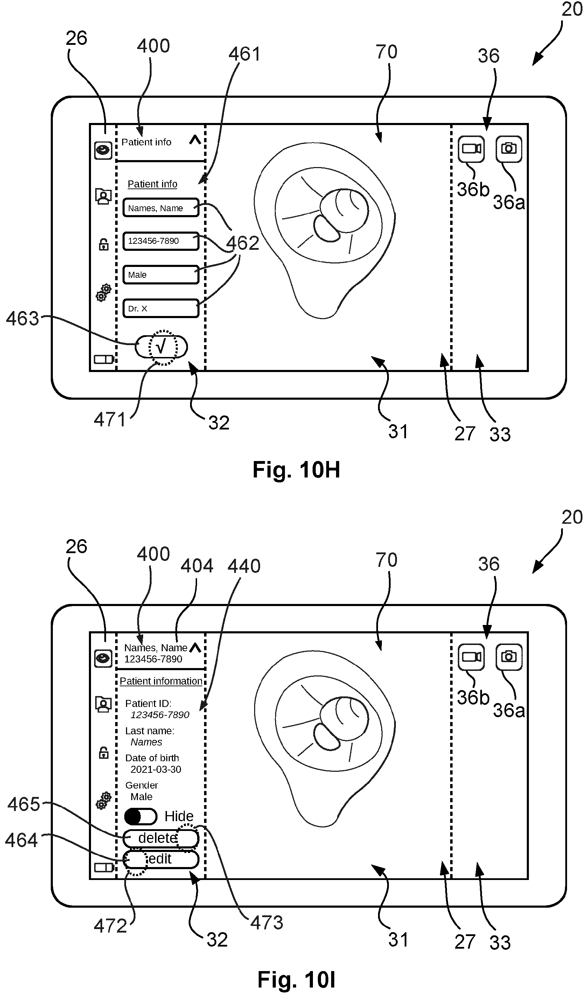

The video processing apparatus 20 may be adapted to detect a manual entry button user input 470 corresponding to selection of the manual entry button 460. In response to detecting the manual entry button user input 470, the video processing apparatus may display a patient entry panel 461, e.g. within the second portion 32 of the graphical user interface 27, as illustrated in Fig. 10H. The patient entry panel 461 may comprise one or more, such as a plurality of, patient info entry fields 462 and a confirm button 463.

The video processing apparatus 20 is adapted to receive a sequence of inputs corresponding to input of a sequence of characters in the patient entry fields 462. For example, a soft-keyboard may be displayed when detecting a user input directed to a patient entry field 462. Alternatively, an external keyboard may be used to provide the sequence of characters. Subsequent to receiving the sequence of inputs, the video processing apparatus 20 is adapted to detect a confirm user input 471 corresponding to selection of the confirm button 463.

In response to receiving the confirm user input 471 the video processing apparatus assigns manual patient information data corresponding to the sequence of characters in the one or more patient entry fields 462 to the procedure session. Some of the patient entry fields 462 may be left blank.

After assigning the manual patient information data, according to the information entered in the patient entry fields 462, to the procedure session, the video processing apparatus 20 may, as illustrated in Fig. 10I cease display of the patient entry panel 461 and redisplay the patient information panel 400 with the heading section 404 comprising second patient information of the manual patient information data according to the information entered in the patient entry fields 462. The patient information panel 400 may further comprise the detailed patient section 440, as explained, e.g., in relation to Fig. 10D, showing detailed patient information of the manual patient information data according to the information entered in the patient entry fields 462.

The patient information panel 400 may, as illustrated in Fig. 10I, comprise an edit button 464. The video processing apparatus 20 may be adapted to detect an edit button user input 472 corresponding to selection of the edit button 464. In response to detecting the edit button user input 472 the video processing apparatus may (re)display the patient entry panel 461, i.e. returning to the state as illustrated in Fig. 10H.

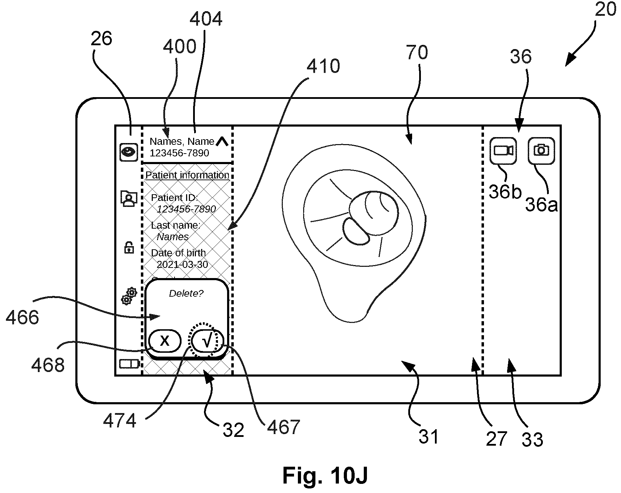

The patient information panel 400 may, as illustrated in Fig. 10I, comprise a delete button 465. The video processing apparatus 20 may be adapted to detect a delete button user input 473 corresponding to selection of the delete button 465. In response to detecting the delete button user input 473 the video processing apparatus displays a confirmation dialogue 466, as illustrated in Fig. 10J, including a confirm button 467 and a cancel button 468. The video processing apparatus is adapted to detect a confirm user input 474 corresponding to selection of the confirm button 467. The video processing apparatus is also adapted to detect a cancel user input (not illustrated) corresponding to selection of the cancel button 468.

In accordance with detecting the confirm user input 474, the video processing apparatus 20, removes the assignment of the manual patient information data to the procedure session, ceases display of the confirmation dialogue 466, and redisplays the patient information panel 400 comprising the manual entry button 460. In effect returning to the state of Fig. 10A.

In accordance with detecting the cancel user input corresponding to selection of the cancel button 468, the video processing apparatus 20 maintains the assignment of the manual patient information data to the procedure session, ceases display of the confirmation dialogue, and redisplays the patient information panel 400 comprising the delete button. In effect returning to the state of Fig. 10I.

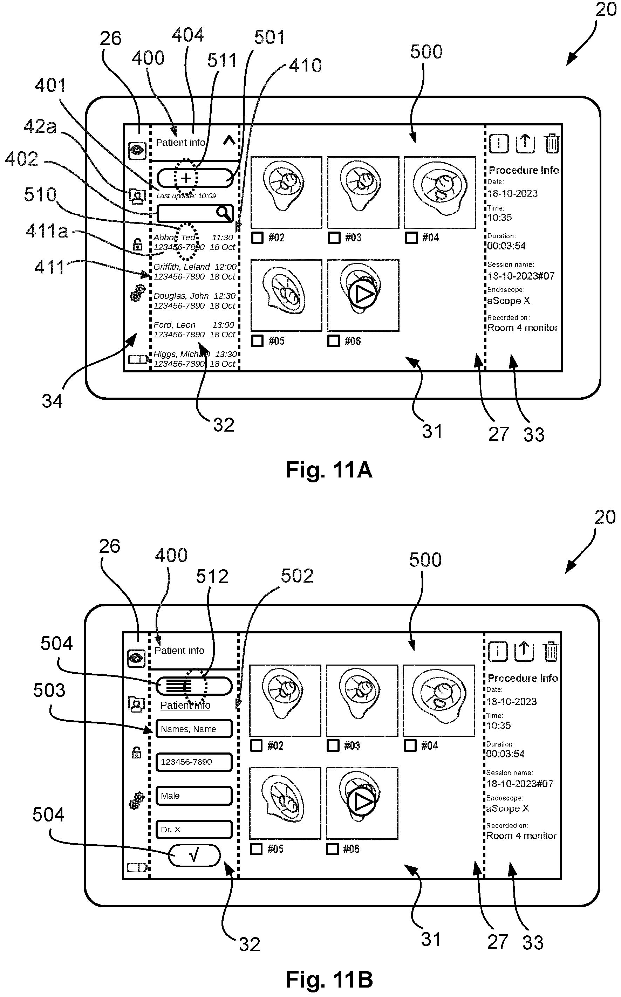

Figs. 11A-11B schematically illustrate parts of an exemplary graphical user interface 27, such as the graphical user interface 27 as described in relation to the previous figures. More specifically, Figs. 11A-11B are related to the graphical user interface 27 in relation to an alternative procedure for entering patient data. The procedure for entering patient data as explained in relation to Figs. 11A-11B may be provided together with the procedure as explained in relation to Figs. 10A-10J, i.e. within the same graphical user interface 27. More specifically

In Fig 11A the video processing apparatus 20 displays a plurality of representations 500 within the first portion 31 of the graphical user interface 27. The plurality of representations 500 corresponds to a plurality of stored data files (e.g. image data files and/or video data files) stored during a first procedure session, e.g. a previous procedure session or an ongoing procedure session. The plurality of representations 500 may be displayed in response to a user entering an archive mode of the video processing apparatus 20. This may, for example, be done by the user having selected an archive menu item 42a of the one or more actionable menu items 42 displayed in the fourth portion 34 of the graphical user interface 27.

As explained previously, the video processing apparatus 20 may be adapted to retrieve worklist data from a remote server, such as the remote server 90 as described in relation to Fig. 1. As also previously explained, the worklist data may comprise patient information data for a plurality of scheduled procedures, such as for scheduled procedures within an appropriate time interval, e.g. between 12 hours prior to a current system time of the video processing apparatus 20 and 24 hours after the current system time of the video processing apparatus. Retrieval of the worklist data may be performed similarly as explained in relation to Figs. 10A-10J.

As also illustrated in Fig. 11A, the video processing apparatus displays a patient information panel 400 within the second portion 32 of the graphical user interface 27. The patient information panel 400, similarly to the patient information panel 400 as illustrated in Fig. 10A, comprises a worklist section 410 comprising a plurality of selectable patient information entries 411 corresponding to the patient information data of the worklist data retrieved from the remote server.

The video processing apparatus 20 is adapted to detect a first user input 510 corresponding to selection of a first patient information entry 411a of the plurality of selectable patient information entries 411. In accordance with detecting the first user input 510 the video processing apparatus assigns first patient information data corresponding to the first patient information entry 411a to the first procedure session, i.e. the procedure session during which the plurality of stored data files corresponding to the plurality of representations 500 were stored.

Like in Fig. 10A, the patient information panel 400, as illustrated in Fig. 11A, may comprise a manual entry button 501. The video processing apparatus 20 may be adapted to detect a manual entry button user input 511 corresponding to selection of the manual entry button 501. In response to detecting the manual entry button user input 511, the video processing apparatus may display a patient entry panel 502, e.g. within the second portion 32 of the graphical user interface 27, as illustrated in Fig. 11B. The patient entry panel 502 may comprise one or more, such as a plurality of, patient info entry fields 503 and a confirm button 504. The patient entry panel 502 may act similarly to the patient entry panel 461 as described in relation to Fig. 10H.

Particularly, the video processing apparatus 20 may be adapted to receive a sequence of inputs corresponding to input of a sequence of characters in the patient entry fields 503. Subsequent to receiving the sequence of inputs, the video processing apparatus 20 is adapted to detect a confirm user input corresponding to selection of the confirm button 504, and in response to receiving the confirm user input the video processing apparatus assigns manual patient information data corresponding to the sequence of characters in the one or more patient entry fields 503 to the first procedure session. As described in relation to Fig. 10H, some of the patient entry fields 503 may be left blank.

As illustrated in Fig. 11B, the video processing apparatus may, e.g. as part of the patient information panel 400, display a worklist button 504, in the second portion 32 of the graphical user interface 27. The video processing apparatus 20 may be adapted to detect a worklist button user input 512 corresponding to selection of the worklist button 504. In response to detecting the worklist button user input 512 the video processing apparatus 20 displays the patient information panel 400 comprising the worklist section 410 within the second portion 32 of the graphical user interface 27. In effect transitioning to the state illustrated in Fig. 11A.

Thus, as explained, it may be possible to change between the manual entry mode, as illustrated in Fig. 11B, and the worklist entry mode, as illustrated in Fig. 11A. In some examples, the manual entry mode (Fig. 11B) is provided as the initial mode, for assigning patient data to information, such as data files, stored during a first procedure session, e.g. a previous procedure session or an ongoing procedure session. Thus, for the user to select patient information from a retrieved worklist, he/she has to first enter the worklist entry mode by selecting the worklist button 504 in Fig. 11B.

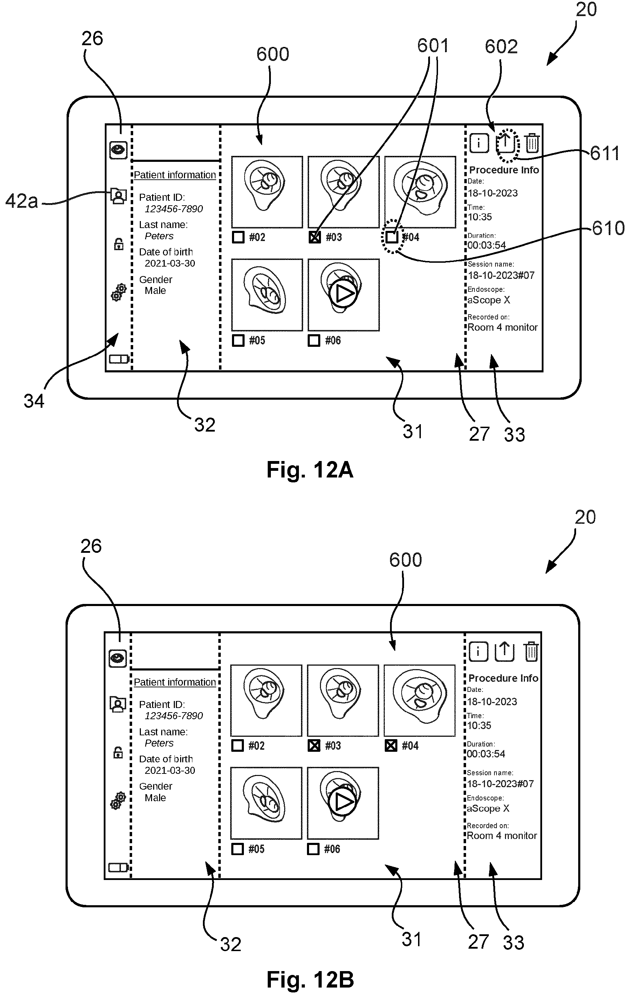

Figs. 12A-12D schematically illustrate parts of an exemplary graphical user interface 27, such as the graphical user interface 27 as described in relation to the previous figures. More specifically, Figs. 12A-12D are related to the graphical user interface 27 in relation to procedures for exporting stored data files, such as image data files and/or video data files.

In the illustrated examples, the video processing apparatus 20 may, as also described in relation to Fig. 4, comprise a network interface, such as a network interface to enable the video processing apparatus to be in communication with a network server, such as an SMB server or a PACS server. Alternatively or additionally, the video processing apparatus 20 may comprise an external communication interface, such as a USB interface, e.g. to enable transfer of data to an external data carrier, such as a USB storage device.

In Fig 12A the video processing apparatus 20 displays a plurality of representations 600 within the first portion 31 of the graphical user interface 27. The plurality of representations 600 corresponds to a plurality of stored data files (e.g. image data files and/or video data files) stored during a first procedure session, e.g. a previous procedure session or an ongoing procedure session. The plurality of representations 600 may be displayed in response to a user entering an archive mode of the video processing apparatus 20. This may, for example, be done by the user having selected an archive menu item 42a of the one or more actionable menu items 42 displayed in the fourth portion 34 of the graphical user interface 27. The plurality of representations 600 as illustrated in Fig. 12A, may correspond to the plurality of representations 500 as illustrated in Fig. 11A. Thus, the parts of the graphical user interface 27 illustrated in Figs. 11A and 12A, respectively, may be for the same archive mode of the video processing apparatus 20.

The video processing apparatus 20 may provide possibilities for exporting the stored data files. For example, a user may select one or more of the stored data files for export. Each of the plurality of representations 600 may comprise a respective selection indicator 601, for the user to select or deselect to indicate which of the stored data files to export. In the example of Fig. 12A the selection indicator of one representation is indicated as being active (showing a crossed box), while the remaining selection indicators 601 are indicated as not being active (showing an empty box). The user may select or deselect each of the selection indicators 601 toggling between active (crossed box) and not active (empty box). For example, the video processing apparatus 20 is adapted to detect a selection user input 610 corresponding to selection of a selection indicator 601 of a representation of the plurality of representations 600. In response to detecting the selection user input 610 and because the exemplary selected selection indicator is not indicating current activation, the video processing apparatus changes display of the exemplary selected selection indicator to indicate activation, as illustrated in Fig. 12B (i.e. a cross has been added to the box). Had the exemplary selected selection indicator indicated current activation, the video processing apparatus would have changed display of the exemplary selected selection indicator to not indicate activation (i.e. the cross had been removed from the box).

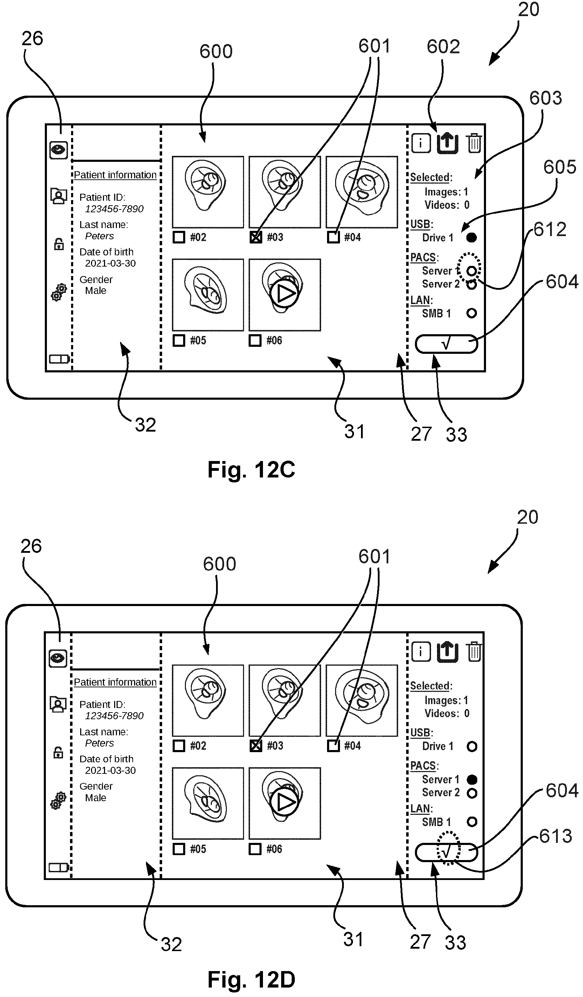

As also illustrated in Fig. 12A, an export button 602 may be displayed, e.g. in the third portion 33 of the graphical user interface 27. The video processing apparatus 20 may be adapted to detect a user input 611 (e.g. a touch input) corresponding to selection of the export button 602. In response to detection of the user input 611, the video processing apparatus may display, as illustrated in Fig. 12C, an export menu 603, e.g. in the third portion 33 of the graphical user interface 27. As an indicator of the export menu 603 being the content of the third portion 33, the export button 602 may change appearance to a second appearance (e.g. a second color, e.g. green), e.g. different from a first appearance (e.g. a first color, e.g. white).

In the export menu 603, the user may choose how to export the selected data files (according to selection state as indicated by the selection indicators 601). In other words which of a plurality of selectable export modalities the user wants to use for the export of the selected data files. For example, the user may export to an external storage device, such as a USB drive, the user may export to a PACS (picture archiving and communication system) server, or the user may export to a network drive, such as an SMB storage entity. The export menu 603, as illustrated, comprises a plurality of selectable export modalities 605, such as for exporting to USB, PACS and/or a network drive.

In some examples, the video processing apparatus 20 stores one or more user profiles each including a username and a password for activating an authorized mode of the video processing apparatus 20, and the possibility to export to PACS may be displayed in accordance with the authorized mode being active. In other words, the PACS server(s) may be restricted to only be available for authorized users of the video processing apparatus 20.

In the illustrated example of Fig. 12C the USB drive with the name "Drive 1" has been selected (as indicated by the filled indicator) as the current export location. Thus, the export modality is currently selected to be USB device storage. The video processing apparatus 20 may be adapted to detect a user input (e.g. user input 612) corresponding to selection of another export location, for the user to export the selected data files to one of the other locations, e.g. one of the PACS servers or the network drive. For example, the video processing apparatus 20 may detect the user input 612 corresponding to selection of the PACS server "Server 1", and in response to detecting the user input 612, the video processing apparatus 20, as indicated in Fig. 12D, changes the selected export location to "Server 1", as also indicated by the filled indicator next to "Server 1" in Fig. 12D and the now open indicator next to the USB drive "Drive 1". Thus, the export modality has been selected to be PACS.

As illustrated in Figs. 12C and 12D, an export confirm button 604 is displayed, e.g. as part of the export menu 603. The video processing apparatus 20 is adapted to detect an export confirm user input 613 (as illustrated in Fig. 12D) corresponding to selection of the export confirm button 604. In response to detection of the export confirm user input 613, the video processing apparatus transmits stored image data corresponding to the selected of the stored data files (those having a selection indicator 601 indicating current activation) to the selected export location by the selected export modality, in the illustrated example of Fig. 12D to the PACS server "Server 1".

Thus, in accordance with the selected export modality corresponding to export to the PACS server (as in the state of Fig. 12D), the video processing apparatus transmits 20 stored image data corresponding to the selected of the stored data files to the PACS server "Server 1". In accordance with the selected export modality corresponding to export to the USB drive (as if detecting the user input 613 while in the state of Fig. 12C), the video processing apparatus 20 transmits stored image data corresponding to the selected of the stored data files to the USB drive "Drive 1". In accordance with the selected export modality corresponding to export to a network drive (as if the network drive "SMB 1" had been selected), the video processing apparatus 20 transmits stored image data corresponding to the selected of the stored data files to the network drive "SMB 1".

In particular when exporting to USB storage and/or network drive storage, the video processing apparatus 20 may transmit the stored image data corresponding to the selected of the stored data files to a location on the USB drive or network drive, where the location is based on details of the procedure session. For example, a folder may be created at the location, which may be named based on a date (and optionally time) of the procedure and/or a patient identifier, such as the patient's name and/or the patient's identification number. Such information may be assigned to the procedure, e.g., as described in relation to Figs. 10A-11B. However, in some examples, it may be advantageous to not include patient specific details when exporting to USB drive and/or network drive (e.g. a setting may be set in a configuration menu of the video processing apparatus to include or exclude patient specific details in export). Thus, the folder may be named purely based on the date (and optionally time) of the procedure.

When exporting to USB storage and/or network drive storage, the stored image data transmitted to the USB drive or network drive may comprise the selected of the stored data files in a common picture file format, e.g. PNG, JPEG, or TIFF. When exporting to PACS, the stored image data transmitted to the PACS server may comprise the selected of the stored data files in DICOM format (Digital Imaging and Communications in Medicine). Thus, the selection of export modality influences the format of which the selected data files are exported. When exporting to PACS procedure information may be exported. For example, time and date of the procedure, patient details (e.g. assigned to the procedure as described in relation to Figs. 10A-11B) and/or operator details may be exported together with the image data.

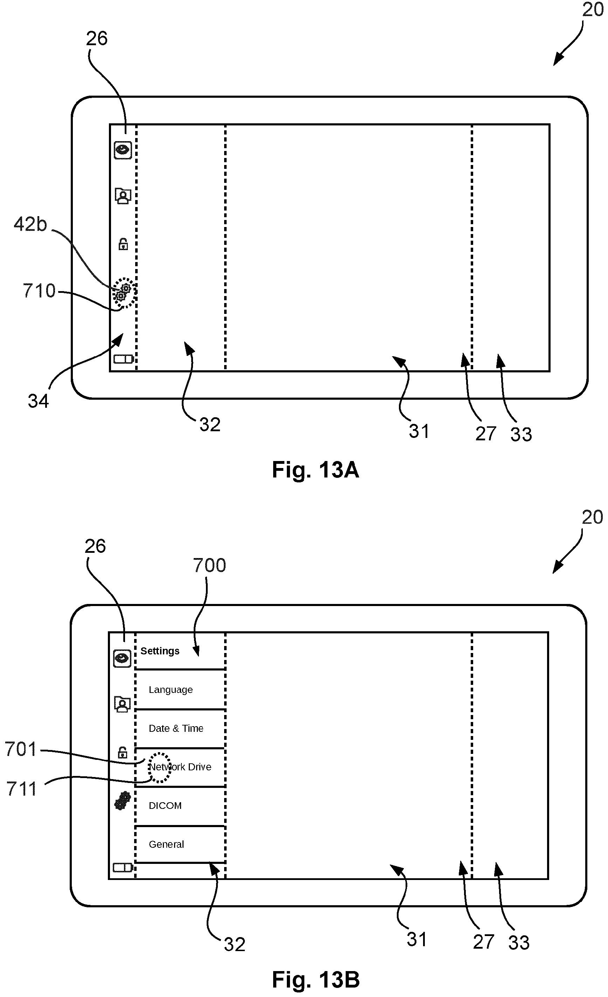

Figs. 13A-13E schematically illustrate parts of an exemplary graphical user interface 27, such as the graphical user interface 27 as described in relation to the previous figures. More specifically, Figs. 13A-13E are related to the graphical user interface 27 in relation to procedures for configuring export of stored data files, such as image data files and/or video data files. The procedures as described in relation to Figs. 13A-13E influence the export procedures, as described in relation to Figs. 12A-12D.

Fig. 13A illustrates that the video processing apparatus 20 detects a user input 710 corresponding to selection of a settings menu item 42b of the one or more actionable menu items 42 displayed in the fourth portion 34 of the graphical user interface 27. In response to detection of the user input 710 a settings menu 700 is displayed, e.g. in the second portion 32 of the graphical user interface 27, as illustrated in Fig. 13B. The settings menu 700 may comprise a plurality of settings buttons for configuration of respective settings, such as changing language of the graphical user interface 27, changing date and time of the system time of the video processing apparatus 20, etc. The settings menu 700 may comprise a network drive settings button 701 to allow the user to configure network drive settings for enabling export of data files to a network drive, e.g. as explained in relation to Figs. 12A-12D.

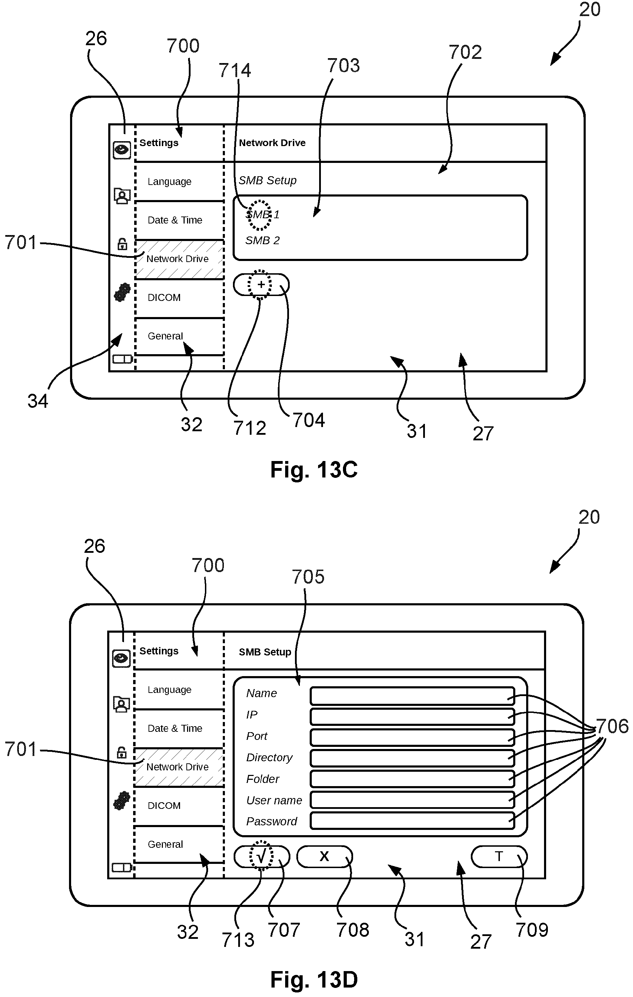

The video processing apparatus 20 may be adapted to detect a further user input 711 corresponding to selection of the network drive settings button 701 of the settings menu 700 settings menu item 42b of the one or more actionable menu items 42 displayed in the fourth portion 34 of the graphical user interface 27. In response to detection of the further user input 711, the video processing apparatus displays a network drive overview 702, as illustrated in Fig. 13C. The network drive overview 702 may be displayed in the first portion 31 of the graphical user interface 27. In some examples, the network drive overview 702 may also extend into the second portion 32, as illustrated. Furthermore, as also illustrated in Fig. 13C, the network drive settings button 701 may have changed appearance (e.g. from a first background color to a second (different) background color) to indicate that the currently displayed page corresponds to the network drive settings button 701.

The network drive overview 702 may comprise a list of currently setup network drives 703. In the illustrated example the list 703 comprises two network drives. However, the list 703 may be empty if no network drives are configured. The network drive overview 702 may comprise an add new button 704.

The video processing apparatus 20 may be adapted to detect a user input 712 corresponding to selection of the add new button 704. In response to detecting the user input 712 corresponding to selection of the add new button 704, the video processing apparatus 20 displays, a network drive setup 705, as illustrated in Fig. 13D. The network drive setup 705 may be displayed in the first portion 31 of the graphical user interface 27. In some examples, the network drive setup 705 may also extend into the second portion 32, as illustrated.

The network drive setup 705 comprises a plurality of network drive input fields 706. The network drive setup 705 comprises a confirm button 707. The network drive setup 705 comprises a cancel button 708, which if selected cause return to the network drive overview 702. The network drive setup 705 comprises a test button 709.

While displaying the network drive setup 705, the video processing apparatus 20 is adapted to receive a sequence of one or more user inputs corresponding to input of a sequence of characters in the network drive input fields 706. For example, a soft-keyboard may be displayed when detecting a user input directed to a network drive input fields 706. Alternatively, an external keyboard may be used to provide the sequence of characters. The network drive input fields may include server name (e.g. for providing a label indicative of the network drive), server IP address, server port number, directory name, and user credentials (e.g. if the network drive requires authorization to accept storage of data). The test button 709 may be selected by the user to test the network drive according to the information provided in the network drive input fields 706, and accordingly provide a warning if one or more of the content of the network drive input fields 706 fails validation tests or the server according to the information provided does not respond.

The video processing apparatus 20 is adapted to, e.g. subsequent to receiving the sequence of inputs, detect a confirm user input 713 corresponding to selection of the confirm button 707. In response to receiving the confirm user input 713 the video processing apparatus 20 stores network drive information for a first network drive corresponding to the sequence of characters in the one or more network drive input fields. Accordingly, the information that the user has input into the network drive input fields 706 are stored in the electronic memory of the video processing apparatus 20.

When afterwards, entering the export procedure as explained in relation to Fig. 12A-12D, the export menu 603 includes a possibility to choose the network drive in accordance with the network drive information provided in the network drive input fields, and in accordance with the selected export modality (in Fig. 12D) corresponds to export to the network drive according to the information input into the network drive input fields 706 in Fig. 13D, the video processing apparatus 20 transmits stored image data corresponding to the selected of the stored data files to the network drive according to the information input into the network drive input fields 706.

Returning to Fig. 13C, the list of currently setup network drives 703 comprises names of currently setup network drives, and if displayed after adding the network drive as explained in relation to Fig. 13D, the list 703 will also contain a reference to this newly added network drive.

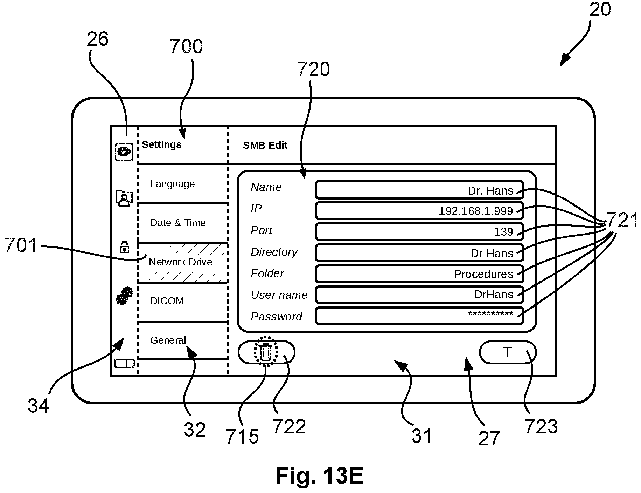

The user may be provided a possibility to amend the configuration for the currently setup network drives. Thus, the video processing apparatus 20 may be adapted to detect an edit user input 714 corresponding to selection of a first network drive of the list of currently setup network drives 703. In some examples, the list 703 may comprise an edit button next to the names of the network drives of the list 703. Accordingly, the edit user input 714 may additionally or alternatively correspond to selection of an edit button of the first network drive. In response to receiving the edit user input 714 the video processing apparatus 20 may display, as illustrated in Fig. 13E, a network drive edit page 720. The network drive edit page 720 comprises a plurality of network drive edit fields 721, similar to the plurality of network drive input fields 706 in Fig. 13D, but with current information being provided in the fields. The user may change some or all of the information in the network drive edit fields 721 to change the configuration. For example, a soft-keyboard may be displayed when detecting a user input directed to a network drive edit field 721. Alternatively, an external keyboard may be used to provide input for the network drive edit fields 721. The test button 723 may be selected by the user to test the network drive according to the information provided in the network drive edit fields 721, and accordingly provide a warning if one or more of the content of the network drive edit fields 721 fails validation tests or the server according to the information provided does not respond.

Changes of the network drive configuration by changing the content of one or more of the network drive edit fields 721 may be autosaved. Alternatively, an edit confirm button, like the confirm button 707 of Fig 13D may be provided to save the amendments. Similarly a cancel button may be provided to undue any entered amendments.

The network drive edit page 720 may comprise a delete button 722. The video processing apparatus 20 may be adapted to detect a delete user input 714 corresponding to selection of the delete button 722. In response to receiving the delete user input 714 the video processing apparatus 20 may delete the stored information related to the network drive currently being edited. Subsequently, the video processing apparatus 20 may display the network drive overview 702. Thus, essentially returning to Fig. 13C, where the network drive now deleted will be omitted from the list 703.

The disclosure has been described with reference to a preferred embodiment. However, the scope of the invention is not limited to the illustrated embodiment, and alterations and modifications can be carried out without deviating from the scope of the invention.

Throughout the description, the use of the terms "first", "second", "third", "fourth", "primary", "secondary", "tertiary" etc. does not imply any particular order or importance but are included to identify individual elements. Furthermore, the labelling of a first element does not imply the presence of a second element and vice versa.

### EXEMPLARY EMBODIMENTS

Exemplary embodiments of the present disclosure are set out in the following items:
1. A video processing apparatus operable to receive image data from an endoscope having an image sensor configured to generate image data indicative of a view from the endoscope, the video processing apparatus comprising:
   - a housing,
   - a processing unit adapted to receive the image data from the endoscope as the image data is being generated and cause a display to display a graphical user interface extending a first length along a first direction and a second length along a second direction perpendicular to the first direction, the graphical user interface comprising a plurality of non-overlapping portions including a first portion and a second portion, wherein the first portion is arranged next to the second portion along the second direction,

   and wherein the video processing apparatus displays a live representation of the image data within the first portion of the graphical user interface, wherein the live representation is displayed at a full size covering the first length along the first direction,
   the video processing apparatus being adapted to detect a first user input signal indicative of a request to activate a freeze function, and in response to detecting the first user input signal the video processing apparatus activates the freeze function including:
      - ceasing display of the live representation of the image data within the first portion of the graphical user interface,
      - displaying within the first portion of the graphical user interface a first representation of a still image corresponding to the image data received when the first user input signal was detected, wherein the first representation of the still image is displayed at the full size, and
      - displaying the live representation of the image data within the second portion of the graphical user interface, wherein the live representation is displayed at a reduced size smaller than the full size.
2. Video processing apparatus according to any one of the preceding items, wherein while the freeze function is active, the video processing apparatus is adapted to detect a second user input signal indicative of a request to deactivate the freeze function, and in response to detecting the second user input signal, the video processing apparatus deactivates the freeze function including:
   - ceasing display of the first representation of the still image within the first portion of the graphical user interface,
   - displaying the live representation of the image data within the first portion of the graphical user interface, wherein the live representation is displayed at the full size, and
   - ceasing display of the live representation of the image data within the second portion of the graphical user interface.
3. Video processing apparatus according to item 2, wherein the video processing apparatus is adapted to detect a user input directed to the live representation of the image data being displayed within the second portion of the graphical user interface, and wherein the second user input signal is instigated by detection of the user input directed to the live representation of the image data.
4. Video processing apparatus according to any one of items 2-3, wherein the video processing apparatus is adapted to detect disconnection of the endoscope, and wherein the second user input signal is instigated in response to detecting disconnection of the endoscope from the video processing apparatus.
5. Video processing apparatus according to any one of items 2-4, wherein the video processing apparatus is adapted to detect connection of the endoscope, and wherein the second user input signal is instigated in response to detecting connection of a second endoscope to the video processing apparatus.
6. Video processing apparatus according to any one of the preceding items, wherein displaying within the first portion of the graphical user interface the first representation of the still image includes displaying a freeze indicator element overlaying a portion of the first representation of the still image.
7. Video processing apparatus according to any one of the preceding items, wherein the video processing apparatus displays a freeze button, e.g. within a third portion of the graphical user interface, wherein the video processing apparatus is adapted to detect a user input corresponding to selection of the freeze button, and wherein the first user input signal is instigated by detection of the user input corresponding to selection of the freeze button.
8. Video processing apparatus according to any one of the preceding items, wherein the video processing apparatus is adapted to detect a first endoscope signal from the endoscope indicative of a user activating a first button on the endoscope, and wherein the first user input signal is instigated by detection of the first endoscope signal.
9. Video processing apparatus according to any one of the preceding items, wherein while the freeze function is active, the video processing apparatus is adapted to detect a third user input signal indicative of a request to store the still image, and in response to detecting the third user input signal the video processing apparatus stores in electronic memory of the video processing apparatus an image data file corresponding to the image data received when the first user input signal was detected.
10. Video processing apparatus according to item 9, wherein the image data file comprises information indicative of the image data file being captured while the freeze function was active.
11. Video processing apparatus according to any one of items 9-10, wherein the video processing apparatus displays one or more actionable items, e.g. within the third portion of the graphical user interface, wherein the one or more actionable items comprise an image capture button, wherein the video processing apparatus is adapted to detect a capture user input corresponding to selection of the image capture button, and wherein the third user input signal is instigated by the user input corresponding to selection of the image capture button.
12. Video processing apparatus according to any one of items 9-11, wherein the video processing apparatus is adapted to detect a second endoscope signal from the endoscope indicative of a user activating a second button on the endoscope, wherein the third user input signal is instigated by detection of the second endoscope signal.
13. Video processing apparatus according to any one of items 9-12, wherein the video processing apparatus, in response to detecting the third user input signal, further deactivates the freeze function, including:
   - ceasing display of the first representation of the still image within the first portion of the graphical user interface,
   - displaying the live representation of the image data within the first portion of the graphical user interface, wherein the live representation is displayed at the full size, and
   - ceasing display of the live representation of the image data within the second portion of the graphical user interface.
14. Video processing apparatus according to any one of items 9-13, wherein the video processing apparatus, in response to detecting the third user input signal, further displays the first representation of the still image within the second portion of the graphical user interface.
15. Video processing apparatus according to item 14, wherein, after a predetermined delay after detection of the third user input signal, the video processing apparatus cease display of the first representation of the still image within the second portion of the graphical user interface.
16. Video processing apparatus according to any one of the preceding items, wherein the video processing apparatus comprises one or more image modifying functions each of which when being activated modifies one or more parameters of the live representation of the image data, and wherein, while the freeze function is not active, the one or more image modifying functions are activatable, and wherein while the freeze function is active, the one or more image modifying functions are not activatable.
17. Video processing apparatus according to item 16, wherein while the freeze function is active, the video processing apparatus in response to detecting a fourth user input signal indicative of a request to activate an image modifying function of the one or more image modifying functions, displays a notification indicating that the image modifying function is disabled while the freeze function is active.
18. Video processing apparatus according to any one of the preceding items, wherein the video processing apparatus comprises an endoscope light switch function which when being activated instigate deactivation of a light emitter of the endoscope, and wherein while the freeze function is not active, the endoscope light switch function is activatable, and wherein while the freeze function is active, the endoscope light switch function is not activatable.
19. Video processing apparatus according to item 18, wherein the video processing apparatus, in response to detecting the first user input signal, in accordance with the light emitter of the endoscope being active, activates the freeze function, and wherein the video processing apparatus, in response to detecting the first user input signal, in accordance with the light emitter of the endoscope not being active, foregoes activation of the freeze function.
20. A video processing apparatus operable to receive image data from an endoscope having an image sensor configured to generate image data indicative of a view from the endoscope, the video processing apparatus comprising:
   - a housing,
   - a peripheral input device interface adapted to couple the video processing apparatus to one or more peripheral input devices, and
   - a processing unit adapted to receive the image data from the endoscope as the image data is being generated and cause a display to display a graphical user interface extending a first length along a first direction and a second length along a second direction perpendicular to the first direction, the graphical user interface comprising a plurality of non-overlapping portions including a first portion, a second portion, wherein the first portion is arranged next to the second portion along the second direction, the processing unit being further adapted to detect one or more peripheral input signals from the one or more peripheral input devices,

   wherein the video processing apparatus displays a shortcut setup user interface within the first portion of the graphical user interface,
   while displaying the shortcut setup user interface, the video processing apparatus is adapted to detect a first peripheral input signal from a first peripheral input device coupled to the video processing apparatus, wherein the first peripheral input signal is indicative of activation of an input component of the first peripheral input device, and in response to detecting the first peripheral input signal the video processing apparatus:
      - displays a shortcut indicator indicative of the input component of the first peripheral input device associated with the first peripheral input signal, and
      - displays a function selection interface comprising two or more representation of two or more functions assignable to the input component of the first peripheral input device,
   while displaying the function selection interface, the video processing apparatus is adapted to detect a first user input indicative of selection of a first function of the two or more functions, and in response to detecting the first user input, the video processing apparatus stores first shortcut information including assignment of the first peripheral input signal to the first function,
   and wherein after storing the first shortcut information, the video processing apparatus is adapted to detect the first peripheral input signal from the first peripheral input device, and in response to detecting the first peripheral input signal the video processing apparatus, in accordance with the video processing apparatus operating in a first mode where the first function is available, activates the first function.
21. Video processing apparatus according to item 20, wherein when operating in the first mode, the video processing apparatus displays a live representation of the image data within the first portion of the graphical user interface.
22. Video processing apparatus according to any one of items 20-21, wherein after storing the first shortcut information and in response to detecting the first peripheral input signal, the video processing apparatus, in accordance with the video processing apparatus operating in a second mode where the first function is not available, displays an error notification in the graphical user interface and forgoes activating the first function.
23. Video processing apparatus according to any one of items 20-22, wherein the peripheral input device interface includes a USB interface and/or a Bluetooth interface.
24. Video processing apparatus according to any one of items 20-23, wherein the one or more peripheral input devices includes one or more of a keyboard, a mouse, a trackpad, a game controller and a foot pedal, and/or wherein the first peripheral input device is a foot pedal.
25. Video processing apparatus according to any one of items 20-24, wherein the shortcut setup user interface comprises instructions to connect a first peripheral input device and/or to activate the input component of the first peripheral input device.
26. Video processing apparatus according to any one of items 20-25, wherein, in response to detecting the first peripheral input signal while displaying the shortcut setup user interface, the video processing apparatus, in accordance with the video processing apparatus comprising shortcut information including assignment of the first peripheral input signal to a previous function, displays a warning message.
27. Video processing apparatus according to any one of items 20-26, wherein, in response to detecting the first peripheral input signal while displaying the shortcut setup user interface, the video processing apparatus displays a cancel button selectable to exit the shortcut setup user interface without storing the first shortcut information.
28. Video processing apparatus according to any one of items 20-27, wherein, in response to detecting the first user input the video processing apparatus displays a naming dialogue adapted to receive a first user input name for identifying the assignment of the first peripheral input signal to the first function, and wherein the stored first shortcut information includes the first user input name.
29. Video processing apparatus according to any one of items 20-28, wherein the two or more functions assignable to the input component of the first peripheral input device includes a capture image function causing the video processing apparatus to store an image data file corresponding to the image data received when the capture image function was activated.
30. Video processing apparatus according to any one of items 20-29, wherein the two or more functions assignable to the input component of the first peripheral input device includes a video capture function causing the video processing apparatus to store a video sequence of image data corresponding to the image data received when the video capture function was activated.
31. Video processing apparatus according to any one of items 20-30, wherein the video processing apparatus stores one or more user profiles each including a username and a password for activating an authorized mode of the video processing apparatus, and wherein the shortcut setup user interface is displayed in accordance with the authorized mode being active.
32. A video processing apparatus operable to receive image data from an endoscope having an image sensor configured to generate image data indicative of a view from the endoscope, the video processing apparatus comprising:
   - a housing,
   - a processing unit adapted to receive the image data from the endoscope as the image data is being generated and cause a primary display to display a graphical user interface extending a first length along a first direction and a second length along a second direction perpendicular to the first direction, the graphical user interface comprising a plurality of non-overlapping portions including a first portion and a second portion, wherein the first portion is arranged next to the second portion along the second direction,

   and wherein the video processing apparatus:
      - displays a live representation of the image data within the first portion of the graphical user interface, and
      - displays a patient information panel within the second portion of the graphical user interface, the patient information panel comprising a heading section and a detailed section further comprising detailed patient information and a hide button,
   the video processing apparatus being adapted to detect a hide user input corresponding to selection of the hide button, and in response to detecting the hide user input the video processing apparatus:
      - in accordance with a hide data function being active when detecting the hide user input, deactivates the hide data function including displaying first patient information in the heading section, and
      - in accordance with the hide data function not being active when detecting the hide user input, activates the hide data function including ceasing display of the first patient information in the heading section,
   and wherein the video processing apparatus is further adapted to detect a detail user input indicative of a request to toggle the detailed section of the patient information panel, and in response to detecting the detail user input the video processing apparatus:
      - in accordance with the detailed section being displayed when detecting the detail user input, ceases display of the detailed section of the patient information panel, and
      - in accordance with the detailed section not being displayed when detecting the detail user input, displays the detailed section of the patient information panel.
33. Video processing apparatus according to item 32, wherein the video processing apparatus comprises a display interface adapted to connect the video processing apparatus to an external display, and wherein the video processing apparatus is adapted to cause the external display to display a secondary graphical user interface extending a first secondary length along a first secondary direction and a second secondary length along a second secondary direction perpendicular to the first secondary direction, the secondary graphical user interface comprising a plurality of non-overlapping secondary portions including a first secondary portion and a second secondary portion, wherein the first secondary portion is arranged next to the second secondary portion along the second secondary direction,
   wherein the video processing apparatus:
   - displays the live representation of the image data within the first secondary portion of the secondary graphical user interface,
   - in accordance with the hide data function not being active displays a secondary patient information panel within the second secondary portion of the secondary graphical user interface, the secondary patient information panel comprising the detailed patient information, and
   - in accordance with the hide data function being active foregoes display of the secondary patient information panel within the second secondary portion of the secondary graphical user interface.
34. Video processing apparatus according to item 33, wherein the video processing apparatus further in response to detecting the detail user input:
   - in accordance with the hide data function not being active and the detailed section not being displayed when detecting the detail user input, displays the secondary patient information panel comprising the detailed patient information,
   - in accordance with the hide data function not being active and the detailed section being displayed when detecting the detail user input, ceases display of the secondary patient information panel, and
   - in accordance with the hide data function being active when detecting the detail user input foregoes display of the secondary patient information panel within the second secondary portion of the secondary graphical user interface.
35. Video processing apparatus according to any one of items 32-34, wherein the video processing apparatus in response to detecting the hide user input:
   - in accordance with the hide data function being active when detecting the hide user input, changes a visual appearance of the hide button to a first appearance, and
   - in accordance with the hide data function not being active when detecting the hide user input, changes the visual appearance of the hide button to a second appearance different from the first appearance.
36. Video processing apparatus according to any one of items 32-35, wherein the first patient information includes a name of the patient and/or a personal identification number of the patient.
37. Video processing apparatus according to any one of items 32-36, wherein the detailed patient information includes the first patient information and additional patient information of the patient e.g. including date of birth and/or gender of the patient.
38. A video processing apparatus operable to receive image data from an endoscope having an image sensor configured to generate image data indicative of a view from the endoscope, the video processing apparatus comprising:
   - a housing,
   - a processing unit adapted to receive the image data from the endoscope as the image data is being generated and cause a primary display to display a graphical user interface extending a first length along a first direction and a second length along a second direction perpendicular to the first direction, the graphical user interface comprising a plurality of non-overlapping portions including a first portion and a second portion, wherein the first portion is arranged next to the second portion along the second direction,

   and wherein the video processing apparatus:
      - retrieves worklist data from a remote server, wherein the worklist data comprises patient information data for a plurality of scheduled procedures,
      - opens a procedure session,
      - displays a live representation of the image data within the first portion of the graphical user interface, and
      - displays a patient information panel within the second portion of the graphical user interface, the patient information panel comprising a worklist section comprising a plurality of selectable patient information entries corresponding to the patient information data of the worklist data,
   the video processing apparatus being adapted to detect a first user input corresponding to selection of a first patient information entry of the plurality of selectable patient information entries, and in accordance with detecting the first user input the video processing apparatus assigns first patient information data corresponding to the first patient information entry to the procedure session.
39. Video processing apparatus according to item 38, wherein the video processing apparatus and the remote server is part of a local area network, and wherein the video processing apparatus retrieves the worklist data via the local area network.
40. Video processing apparatus according to any one of items 38-39, wherein the video processing apparatus is adapted to detect a second user input indicative of a request to toggle display of the worklist section of the patient information panel, and in response to detecting the second user input the video processing apparatus:
   - in accordance with the worklist section being displayed when detecting the second user input, ceases display of the worklist section of the patient information panel, and
   - in accordance with the worklist section not being displayed when detecting the second user input, displays the worklist section of the patient information panel.
41. Video processing apparatus according to item 40, wherein in response to detecting the second user input and in accordance with the worklist section not being displayed when detecting the second user input, the video processing apparatus retrieves the worklist data from the remote server.
42. Video processing apparatus according to any one of items 38-41, wherein the video processing apparatus in response to detecting the first user input displays a representation of the first patient information data and a confirmation button, the video processing apparatus being adapted to detect a confirmation user input corresponding to selection of the confirmation button, and in response to detecting the confirmation user input the video processing apparatus assigns the first patient information data to the procedure session.
43. Video processing apparatus according to any one of items 38-42, wherein each of the plurality of selectable patient information entries comprises one or more of patient name, patient identification number, scheduled time for procedure.
44. Video processing apparatus according to any one of items 38-43, wherein the plurality of selectable patient information entries of the worklist section is sorted in chronological order based on scheduled time for procedure.
45. Video processing apparatus according to any one of items 38-44, wherein the patient information data of the worklist consists of patient information data having a scheduled time for procedure between a first time prior to a current system time of the video processing apparatus and a second time after the current system time.
46. Video processing apparatus according to any one of items 38-45, wherein the selectable patient information entries comprise a primary selectable patient information entry and a secondary selectable patient information entry, wherein the primary selectable patient information entry has a primary scheduled time for procedure and the secondary selectable patient information entry has a secondary scheduled time for procedure later than the primary scheduled time for procedure,
   wherein the selectable patient information entries do not comprise an intermediate selectable patient information entry having an intermediate scheduled time for procedure between the primary scheduled time for procedure and the secondary scheduled time for procedure, and
   wherein the video processing apparatus, in displaying the patient information panel, in accordance with the current system time of the video processing apparatus being between the primary scheduled time for procedure and the secondary scheduled time for procedure, does not display the primary selectable patient information entry in the worklist section and displays the secondary selectable patient information entry in the worklist section adjacent to a top of the worklist section.
47. Video processing apparatus according to any one of items 38-46, wherein the worklist section is scrollable in a first scroll direction to show earlier selectable patient information entries of the plurality of selectable patient information entries and in a second scroll direction to show later selectable patient information entries of the plurality of selectable patient information entries.
48. Video processing apparatus according to any one of items 38-47, wherein the video processing apparatus is adapted to detect connection of the endoscope, and wherein the video processing apparatus in response to detecting connection of the endoscope retrieves the worklist data and opens the procedure session.
49. Video processing apparatus according to item 48, wherein the video processing apparatus in response to detecting connection of the endoscope obtains device identifier information from a device identifier of the endoscope, and wherein the procedure session is associated with the device identifier information.
50. Video processing apparatus according to any one of items 38-48, wherein the video processing apparatus displays one or more actionable items within the graphical user interface, wherein the one or more actionable items comprise an image capture button, and wherein the video processing apparatus is adapted to detect a capture user input corresponding to selection of the image capture button, and in response to detecting the capture user input the video processing apparatus stores an image data file corresponding to the image data received when the capture user input was detected and associates the image data file with the procedure session.
51. Video processing apparatus according to any one of items 38-50, wherein the patient information panel comprises a search field, and wherein the video processing apparatus is adapted to receive a sequence of inputs corresponding to inputs of a sequence of characters in the search field, and in response to receiving the sequence of inputs the video processing apparatus filters the displayed selectable patient information entries of the plurality of selectable patient information entries based on a comparison between the sequence of characters in the search field and the patient information data corresponding to the plurality of selectable patient information entries.
52. Video processing apparatus according to any one of items 38-51, wherein the patient information panel comprises a heading section, and wherein after assigning the first patient information data to the procedure session, the video processing apparatus displays first patient information of the first patient information data in the heading section.
53. Video processing apparatus according to any one of items 38-52, wherein after assigning the first patient information data to the procedure session the video processing apparatus ceases display of the worklist section of the patient information panel.
54. Video processing apparatus according to any one of items 38-53, wherein the patient information panel comprises a change patient button, and wherein the video processing apparatus is adapted to detect a change request user input corresponding to selection of the change patient button, and in response to detecting the change request user input the video processing apparatus displays a confirmation dialogue including a confirm button and a cancel button, and is adapted to detect a confirm user input corresponding to selection of the confirm button and a cancel user input corresponding to selection of the cancel button, the video processing apparatus:
   - in accordance with detecting the confirm user input removes the assignment of the first patient information data to the procedure session, ceases display of the confirmation dialogue, and redisplays the worklist section in the patient information panel,
   - in accordance with detecting the cancel user input maintains the assignment of the first patient information data to the procedure session, ceases display of the confirmation dialogue, and foregoes redisplay of the worklist section in the patient information panel.
55. Video processing apparatus according to any one of items 38-54, wherein the patient information panel comprises a manual entry button, and wherein the video processing apparatus is adapted to detect a manual entry button user input corresponding to selection of the manual entry button, and
   in accordance with detecting the manual entry button user input the video processing apparatus displays a patient entry panel within the second portion of the graphical user interface comprising one or more patient info entry fields and a confirm button, wherein the video processing apparatus is adapted to receive a sequence of inputs corresponding to input of a sequence of characters in the one or more patient entry fields and a subsequent confirm user input corresponding to selection of the confirm button, and
   in response to receiving the confirm user input the video processing apparatus assigns manual patient information data corresponding to the sequence of characters in the one or more patient entry fields to the procedure session.
56. Video processing apparatus according to any one of items 38-55, wherein the video processing apparatus stores one or more user profiles each including a username and a password for activating an authorized mode of the video processing apparatus, and wherein the worklist section is displayed in accordance with the authorized mode being active.
57. A video processing apparatus operable to receive image data from an endoscope having an image sensor configured to generate image data indicative of a view from the endoscope, the video processing apparatus comprising:
   - a housing,
   - a processing unit adapted to receive the image data from the endoscope as the image data is being generated and cause a primary display to display a graphical user interface extending a first length along a first direction and a second length along a second direction perpendicular to the first direction, the graphical user interface comprising a plurality of non-overlapping portions including a first portion and a second portion, wherein the first portion is arranged next to the second portion along the second direction,

   and wherein the video processing apparatus:
      - retrieves worklist data from a remote server, wherein the worklist data comprises patient information data for a plurality of scheduled procedures,
      - displays a plurality of representations corresponding to a plurality of stored data files stored during a procedure session within the first portion of the graphical user interface,
      - displays a patient information panel within the second portion of the graphical user interface, the patient information panel comprising a worklist section comprising a plurality of selectable patient information entries corresponding to the patient information data of the worklist data,
   the video processing apparatus being adapted to detect a first user input corresponding to selection of a first patient information entry of the plurality of selectable patient information entries, and in accordance with detecting the first user input the video processing apparatus assigns first patient information data corresponding to the first patient information entry to the procedure session.
58. Video processing apparatus according to item 57, wherein the video processing apparatus before displaying the patient information panel comprising the worklist section displays a worklist button in the second portion of the graphical user interface, the video processing apparatus being adapted to detect a worklist button user input corresponding to selection of the worklist button, and in response to detecting the worklist button user input the video processing apparatus displays the patient information panel comprising the worklist section within the second portion of the graphical user interface.
59. A video processing apparatus operable to receive image data from an endoscope having an image sensor configured to generate image data indicative of a view from the endoscope, the video processing apparatus comprising:
   - a housing,
   - a processing unit adapted to receive the image data from the endoscope as the image data is being generated and cause a primary display to display a graphical user interface extending a first length along a first direction and a second length along a second direction perpendicular to the first direction, the graphical user interface comprising a plurality of non-overlapping portions including a first portion and a second portion, wherein the first portion is arranged next to the second portion along the second direction,

   and wherein the video processing apparatus:
      - opens a procedure session,
      - displays a live representation of the image data within the first portion of the graphical user interface,
      - displays a patient entry panel within the second portion of the graphical user interface comprising one or more patient info entry fields and a confirm button,
   the video processing apparatus being adapted to receive a sequence of inputs corresponding to input of a sequence of characters in the one or more patient entry fields and a subsequent confirm user input corresponding to selection of the confirm button, and
   in response to receiving the confirm user input the video processing apparatus assigns manual patient information data corresponding to the sequence of characters in the one or more patient entry fields to the procedure session.
60. Video processing apparatus according to item 59, wherein the video processing apparatus before displaying the patient entry panel displays a manual entry button within the second portion of the graphical user interface, the video processing apparatus being adapted to detect a manual entry button user input corresponding to selection of the manual entry button, and in response to detecting the manual entry button user input the video processing apparatus displays the patient entry panel within the second portion of the graphical user interface.
61. Video processing apparatus according to any one of items 59-60, wherein after assigning the manual patient information data to the procedure session the video processing apparatus ceases display of the patient entry panel and displays a patient information panel within the second portion of the graphical user interface.
62. Video processing apparatus according to item 61, wherein the patient information panel comprises a heading section, and wherein the video processing apparatus displays first patient information of the manual patient information data in the heading section.
63. Video processing apparatus according to any one of items 61-62, wherein the patient information panel comprises an edit button, and wherein the video processing apparatus is adapted to detect an edit button user input corresponding to selection of the edit button, and in response to detecting the edit button user input the video processing apparatus displays the patient entry panel within the second portion of the graphical user interface comprising the one or more patient info entry fields.
64. Video processing apparatus according to any one of items 61-63, wherein the patient information panel comprises a delete button, and wherein the video processing apparatus is adapted to detect a delete button user input corresponding to selection of the delete button, and in response to detecting the delete button user input the video processing apparatus displays a confirmation dialogue including a confirm button and a cancel button, and is adapted to detect a confirm user input corresponding to selection of the confirm button and a cancel user input corresponding to selection of the cancel button, the video processing apparatus:
   - in accordance with detecting the confirm user input removes the assignment of the manual patient information data to the procedure session, ceases display of the confirmation dialogue, and displays the manual entry button within the second portion of the graphical user interface, and
   - in accordance with detecting the cancel user input maintains the assignment of the manual patient information data to the procedure session, ceases display of the confirmation dialogue, and redisplays the patient information panel within the second portion of the graphical user interface.
65. A video processing apparatus operable to receive image data from an endoscope having an image sensor configured to generate image data indicative of a view from the endoscope, the video processing apparatus comprising:
   - a housing,
   - a processing unit adapted to receive the image data from the endoscope as the image data is being generated and cause a primary display to display a graphical user interface extending a first length along a first direction and a second length along a second direction perpendicular to the first direction, the graphical user interface comprising a plurality of non-overlapping portions including a first portion and a second portion, wherein the first portion is arranged next to the second portion along the second direction,

   and wherein the video processing apparatus:
      - displays a plurality of representations corresponding to a plurality of stored data files stored during a procedure session within the first portion of the graphical user interface,
      - displays a patient entry panel within the second portion of the graphical user interface comprising one or more patient info entry fields and a confirm button,
   the video processing apparatus being adapted to receives a sequence of inputs corresponding to input of a sequence of characters in the one or more patient entry fields and a subsequent confirm user input corresponding to selection of the confirm button, and
   in response to receiving the confirm user input the video processing apparatus assigns manual patient information data corresponding to the sequence of characters in the one or more patient entry fields to the procedure session.
66. Video processing apparatus according to item 65, wherein the video processing apparatus before displaying the patient entry panel displays a manual entry button in the second portion of the graphical user interface, the video processing apparatus being adapted to detect a manual entry button user input corresponding to selection of the manual entry button, and in response to detecting the manual entry button user input the video processing apparatus displays the patient entry panel within the second portion of the graphical user interface.
67. A video processing apparatus operable to receive image data from an endoscope having an image sensor configured to generate image data indicative of a view from the endoscope, the video processing apparatus comprising:
   - a housing,
   - a network interface,
   - a processing unit adapted to receive the image data from the endoscope as the image data is being generated and cause a primary display to display a graphical user interface extending a first length along a first direction and a second length along a second direction perpendicular to the first direction, the graphical user interface comprising a plurality of non-overlapping portions including a first portion and a second portion, wherein the first portion is arranged next to the second portion along the second direction,

   wherein the video processing apparatus displays a network drive setup within the first portion of the graphical user interface, the network drive setup comprising a plurality of network drive input fields and a confirm button,
   while displaying the network drive setup, the video processing apparatus is adapted to receive a sequence of one or more user inputs corresponding to input of a sequence of characters in the one or more network drive input fields and a confirm user input corresponding to selection of the confirm button,
   in response to receiving the confirm user input the video processing apparatus stores network drive information for a first network drive corresponding to the sequence of characters in the one or more network drive input fields.
68. Video processing apparatus according to item 67, wherein the plurality of network drive input fields includes one or more of server name, server IP address, server port number, directory name, and user credentials.
69. Video processing apparatus according to any one of items 67-68, wherein after storing the network drive information for the first network drive, the video processing apparatus:
   - displays, within the first portion of the graphical user interface, a plurality of representations corresponding to a plurality of stored data files stored during a procedure session, and
   - displays an export button,

   wherein the video processing apparatus is adapted to detect an export user input corresponding to selection of the export button, and in response to detection of the export user input the video processing apparatus displays an export menu comprising an export confirm button, wherein the video processing apparatus is adapted to receive an export confirm user input corresponding to selection of the export confirm button, the export menu further comprising a plurality of selectable export modalities including export to a first PACS server and export to the first network drive, and wherein the video processing apparatus is adapted to receive a modality selection user input corresponding to selection of a selected export modality of the plurality selectable export modalities,
   wherein the video processing apparatus, in response to detecting export confirm user input, and in accordance with the selected export modality corresponding to the export to the first network drive, transmits stored image data corresponding to one or more of the plurality of stored data files to the first network drive.
70. A video processing apparatus operable to receive image data from an endoscope having an image sensor configured to generate image data indicative of a view from the endoscope, the video processing apparatus comprising:
   - a housing,
   - a network interface,
   - a processing unit adapted to receive the image data from the endoscope as the image data is being generated and cause a primary display to display a graphical user interface extending a first length along a first direction and a second length along a second direction perpendicular to the first direction, the graphical user interface comprising a plurality of non-overlapping portions including a first portion and a second portion, wherein the first portion is arranged next to the second portion along the second direction,

   and wherein the video processing apparatus:
      - displays, within the first portion of the graphical user interface, a plurality of representations corresponding to a plurality of stored data files stored during a procedure session, and
      - displays an export button,
   wherein the video processing apparatus is adapted to detect an export user input corresponding to selection of the export button, and in response to detection of the export user input the video processing apparatus displays an export menu comprising an export confirm button, wherein the video processing apparatus is adapted to receive an export confirm user input corresponding to selection of the export confirm button, the export menu further comprising a plurality of selectable export modalities including export to a first PACS server and export to a first network drive, and wherein the video processing apparatus is adapted to receive a modality selection user input corresponding to selection of a selected export modality of the plurality selectable export modalities,
   wherein the video processing apparatus, in response to detecting the export confirm user input, and in accordance with the selected export modality corresponding to the export to the first network drive, transmits stored image data corresponding to one or more of the plurality of stored data files to the first network drive.
71. Video processing apparatus according to any one of items 69-70, wherein each of the plurality of representations corresponding to the plurality of stored data files comprises a selection indicator, and wherein the video processing apparatus is adapted to detect a first selection user input corresponding to selection of a first selection indicator of a first representation of the plurality of representations, and wherein in response to detecting the first selection user input the video processing apparatus:
   - in accordance with the first selection indicator not indicating current activation, displays the first selection indicator indicating activation,
   - in accordance with the first selection indicator indicating current activation, displays the first selection indicator not indicating activation.
72. Video processing apparatus according to item 71, wherein the stored image data transmitted to the first network drive corresponds to one or more of the plurality of stored data files each having a selection indicator indicating current activation.
73. Video processing apparatus according to any one of items 69-72, wherein the video processing apparatus transmits the stored image data corresponding to the one or more of the plurality of stored data files to a location on the first network drive based on stored network drive information for the first network drive at the video processing apparatus.
74. Video processing apparatus according to item 73, wherein the location is based on details of the procedure session, e.g. a folder of the location is named based on a date of the procedure and/or a patient identifier.
75. Video processing apparatus according to any one of items 67-74, wherein the first network drive is an SMB network drive.
76. Video processing apparatus according to any one of items 67-75, wherein the stored image data transmitted to the first network drive comprises the one or more of the plurality of stored images files in a common picture file format, e.g. PNG, JPEG, or TIFF.
77. A video processing apparatus operable to receive image data from an endoscope having an image sensor configured to generate image data indicative of a view from the endoscope, the video processing apparatus comprising:
   - a housing,
   - a USB interface,
   - a processing unit adapted to receive the image data from the endoscope as the image data is being generated and cause a primary display to display a graphical user interface extending a first length along a first direction and a second length along a second direction perpendicular to the first direction, the graphical user interface comprising a plurality of non-overlapping portions including a first portion and a second portion, wherein the first portion is arranged next to the second portion along the second direction,

   and wherein the video processing apparatus:
      - displays, within the first portion of the graphical user interface, a plurality of representations corresponding to a plurality of stored data files stored during a procedure session, and
      - displays an export button,
   wherein the video processing apparatus is adapted to detect an export user input corresponding to selection of the export button, and in response to detection of the export user input the video processing apparatus displays an export menu comprising an export confirm button, wherein the video processing apparatus is adapted to receive an export confirm user input corresponding to selection of the export confirm button, the export menu further comprising a plurality of selectable export modalities including export to a first PACS server and export to a first USB drive, and wherein the video processing apparatus is adapted to receive a modality selection user input corresponding to selection of a selected export modality of the plurality selectable export modalities,
   wherein the video processing apparatus, in response to detecting the export confirm user input, and in accordance with the selected export modality corresponding to the export to the first USB drive, transmits stored image data corresponding to one or more of the plurality of stored data files to the first USB drive.
78. Video processing apparatus according to item 77, wherein each of the plurality of representations corresponding to the plurality of stored data files comprises a selection indicator, and wherein the video processing apparatus is adapted to detect a first selection user input corresponding to selection of a first selection indicator of a first representation of the plurality of representations, and wherein in response to detecting the first selection user input the video processing apparatus:
   - in accordance with the first selection indicator not indicating current activation, displays the first selection indicator indicating activation,
   - in accordance with the first selection indicator indicating current activation, displays the first selection indicator not indicating activation.
79. Video processing apparatus according to item 78, wherein the stored image data transmitted to the first USB drive corresponds to one or more of the plurality of stored data files each having a selection indicator indicating current activation.
80. Video processing apparatus according to any one of items 77-79, wherein the video processing apparatus transmits the stored image data corresponding to the one or more of the plurality of stored data files to a location on the first USB drive based on details of the procedure session, e.g. a folder of the location is named based on a date of the procedure and/or a patient identifier.
81. Video processing apparatus according to any one of items 77-80, wherein the stored image data transmitted to the first USB drive comprises the one or more of the plurality of stored images files in a common picture file format, e.g. PNG, JPEG, or TIFF.
82. A medical visualization system comprising a video processing apparatus according to any one of the preceding items and the endoscope.
83. A method for operating a video processing apparatus to receive image data from an endoscope, the method being performed contemporaneously with an unclaimed surgical procedure, the endoscope having an image sensor configured to generate image data indicative of a view from the endoscope, the method comprising the steps of:
   - receive the image data from the endoscope as the image data is being generated and cause a display to display a graphical user interface extending a first length along a first direction and a second length along a second direction perpendicular to the first direction, the graphical user interface comprising a plurality of non-overlapping portions including a first portion and a second portion, wherein the first portion is arranged next to the second portion along the second direction;
   - display a live representation of the image data within the first portion of the graphical user interface at a full size covering the first length along the first direction;
   - detect a first user input signal indicative of a request to activate a freeze function;
   - cease display of the live representation of the image data within the first portion of the graphical user interface;
   - display within the first portion of the graphical user interface a first representation of a still image at full size, corresponding to the image data received when the first user input signal was detected; and
   - display the live representation of the image data within the second portion of the graphical user interface, wherein the live representation is displayed at a reduced size smaller than the full size.
84. The method according to item 83, wherein the method further comprises the steps of:
   - detect a third user input signal indicative of a request to store the still image; and
   store an image data file corresponding to the image data received when the first user input signal was detected.

## Claims

1. A video processing apparatus operable to receive image data from an endoscope having an image sensor configured to generate image data indicative of a view from the endoscope, the video processing apparatus comprising:
- a housing,
- a processing unit adapted to receive the image data from the endoscope as the image data is being generated and cause a display to display a graphical user interface extending a first length along a first direction and a second length along a second direction perpendicular to the first direction, the graphical user interface comprising a plurality of non-overlapping portions including a first portion and a second portion, wherein the first portion is arranged next to the second portion along the second direction,
and wherein the video processing apparatus displays a live representation of the image data within the first portion of the graphical user interface, wherein the live representation is displayed at a full size covering the first length along the first direction,
the video processing apparatus being adapted to detect a first user input signal indicative of a request to activate a freeze function, and in response to detecting the first user input signal the video processing apparatus activates the freeze function including:
- ceasing display of the live representation of the image data within the first portion of the graphical user interface,
- displaying within the first portion of the graphical user interface a first representation of a still image corresponding to the image data received when the first user input signal was detected, wherein the first representation of the still image is displayed at the full size, and
- displaying the live representation of the image data within the second portion of the graphical user interface, wherein the live representation is displayed at a reduced size smaller than the full size,
wherein while the freeze function is active, the video processing apparatus is adapted to detect a third user input signal indicative of a request to store the still image, and in response to detecting the third user input signal the video processing apparatus stores in electronic memory of the video processing apparatus an image data file corresponding to the image data received when the first user input signal was detected.

2. Video processing apparatus according to any one of the preceding claims, wherein while the freeze function is active, the video processing apparatus is adapted to detect a second user input signal indicative of a request to deactivate the freeze function, and in response to detecting the second user input signal, the video processing apparatus deactivates the freeze function including:
- ceasing display of the first representation of the still image within the first portion of the graphical user interface,
- displaying the live representation of the image data within the first portion of the graphical user interface, wherein the live representation is displayed at the full size, and
- ceasing display of the live representation of the image data within the second portion of the graphical user interface.

3. Video processing apparatus according to claim 2, wherein the video processing apparatus is adapted to detect a user input directed to the live representation of the image data being displayed within the second portion of the graphical user interface, and wherein the second user input signal is instigated by detection of the user input directed to the live representation of the image data, and/or
wherein the video processing apparatus is adapted to detect disconnection of the endoscope, and wherein the second user input signal is instigated in response to detecting disconnection of the endoscope from the video processing apparatus, and/or
wherein the video processing apparatus is adapted to detect connection of the endoscope, and wherein the second user input signal is instigated in response to detecting connection of a second endoscope to the video processing apparatus.

4. Video processing apparatus according to any one of the preceding claims, wherein displaying within the first portion of the graphical user interface the first representation of the still image includes displaying a freeze indicator element overlaying a portion of the first representation of the still image.

5. Video processing apparatus according to any one of the preceding claims, wherein the video processing apparatus displays a freeze button, e.g. within a third portion of the graphical user interface, wherein the video processing apparatus is adapted to detect a user input corresponding to selection of the freeze button, and wherein the first user input signal is instigated by detection of the user input corresponding to selection of the freeze button.

6. Video processing apparatus according to any one of the preceding claims, wherein the video processing apparatus is adapted to detect a first endoscope signal from the endoscope indicative of a user activating a first button on the endoscope, and wherein the first user input signal is instigated by detection of the first endoscope signal.

7. Video processing apparatus according to any one of the preceding claims, wherein the image data file comprises information indicative of the image data file being captured while the freeze function was active.

8. Video processing apparatus according to any one of the preceding claims, wherein the video processing apparatus is adapted to detect a second endoscope signal from the endoscope indicative of a user activating a second button on the endoscope, wherein the third user input signal is instigated by detection of the second endoscope signal.

9. Video processing apparatus according to any one of the preceding claims, wherein the video processing apparatus, in response to detecting the third user input signal, further deactivates the freeze function, including:
- ceasing display of the first representation of the still image within the first portion of the graphical user interface,
- displaying the live representation of the image data within the first portion of the graphical user interface, wherein the live representation is displayed at the full size, and
- ceasing display of the live representation of the image data within the second portion of the graphical user interface.

10. Video processing apparatus according to any one of the preceding claims, wherein the video processing apparatus, in response to detecting the third user input signal, further displays the first representation of the still image within the second portion of the graphical user interface, and
optionally wherein, after a predetermined delay after detection of the third user input signal, the video processing apparatus ceases display of the first representation of the still image within the second portion of the graphical user interface.

11. Video processing apparatus according to any one of the preceding claims, wherein the video processing apparatus comprises one or more image modifying functions each of which when being activated modifies one or more parameters of the live representation of the image data, and wherein, while the freeze function is not active, the one or more image modifying functions are activatable, and wherein while the freeze function is active, the one or more image modifying functions are not activatable, and
optionally wherein while the freeze function is active, the video processing apparatus in response to detecting a fourth user input signal indicative of a request to activate an image modifying function of the one or more image modifying functions, displays a notification indicating that the image modifying function is disabled while the freeze function is active.

12. Video processing apparatus according to any one of the preceding claims, wherein the video processing apparatus comprises an endoscope light switch function which when being activated instigate deactivation of a light emitter of the endoscope, and wherein while the freeze function is not active, the endoscope light switch function is activatable, and wherein while the freeze function is active, the endoscope light switch function is not activatable, and
optionally wherein the video processing apparatus, in response to detecting the first user input signal, in accordance with the light emitter of the endoscope being active, activates the freeze function, and wherein the video processing apparatus, in response to detecting the first user input signal, in accordance with the light emitter of the endoscope not being active, foregoes activation of the freeze function.

13. A medical visualization system comprising a video processing apparatus according to any one of the preceding claims and the endoscope.

14. A method for operating a video processing apparatus to receive image data from an endoscope, the claimed method being performed contemporaneously with an unclaimed surgical procedure, the endoscope having an image sensor configured to generate image data indicative of a view from the endoscope, the method comprising the steps of:
- receive the image data from the endoscope as the image data is being generated and cause a display to display a graphical user interface extending a first length along a first direction and a second length along a second direction perpendicular to the first direction, the graphical user interface comprising a plurality of non-overlapping portions including a first portion and a second portion, wherein the first portion is arranged next to the second portion along the second direction;
- display a live representation of the image data within the first portion of the graphical user interface at a full size covering the first length along the first direction;
- detect a first user input signal indicative of a request to activate a freeze function;
- cease display of the live representation of the image data within the first portion of the graphical user interface;
- display within the first portion of the graphical user interface a first representation of a still image at full size, corresponding to the image data received when the first user input signal was detected; and
- display the live representation of the image data within the second portion of the graphical user interface, wherein the live representation is displayed at a reduced size smaller than the full size.

15. The method according to claim 14, wherein the method further comprises the steps of:
- detect a third user input signal indicative of a request to store the still image; and
- store an image data file corresponding to the image data received when the first user input signal was detected.
